# EUROPEAN PATENT SPECIFICATION

(11) **EP 3 058 068 B1**
(45) Date of publication and mention of the grant of the patent: **24.04.2019**
(21) Application number: 14854291.3
(22) Date of filing: 14.10.2014
(51) Int. Cl.: C12N 15/113, A61K 31/712

(54) **COMPOSITIONS FOR MODULATING EXPRESSION OF C9ORF72 ANTISENSE TRANSCRIPT**
ZUSAMMENSETZUNGEN ZUR MODULATION DER EXPRESSION DES C9ORF72-ANTISENSE-TRANSKRIPTS
COMPOSITIONS DESTINÉES À MODULER L'EXPRESSION DU TRANSCRIT ANTISENS C9ORF72

(30) Priority: 14.10.2013 US 201361890849 P
(43) Date of publication of application: 24.08.2016
(73) Proprietor: Ionis Pharmaceuticals, Inc., Carlsbad, CA 92010 (US)
(72) Inventor: RIGO, Frank, Carlsbad, CA 92010 (US)
(74) Representative: Chapman, Desmond Mark
(86) International application number: PCT/US2014/060512
(87) International publication number: WO 2015/057727

(56) References cited:
- WO-A1-2012/114111
- WO-A2-2007/056113
- US-A1- 2006 286 575
- SORANA CIURA ET AL: "Loss of function of C9orf72 causes motor deficits in a zebrafish model of Amyotrophic Lateral Sclerosis", ANNALS OF NEUROLOGY., 1 April 2013 (2013-04-01), pages n/a-n/a, XP55228421, BOSTON, US ISSN: 0364-5134, DOI: 10.1002/ana.23946
- Z. XU ET AL: "Expanded GGGGCC repeat RNA associated with amyotrophic lateral sclerosis and frontotemporal dementia causes neurodegeneration", PROCEEDINGS NATIONAL ACADEMY OF SCIENCES PNAS, vol. 110, no. 19, 3 April 2013 (2013-04-03), pages 7778-7783, XP055359239, US ISSN: 0027-8424, DOI: 10.1073/pnas.1219643110
- ASH ET AL.: 'Unconventional translation of C90RF72 GGGGCC expansion generates insoluble polypeptides specific to c9FTD/ALS.' NEURON vol. 77, no. 4, 20 February 2014, pages 639 - 646, XP055105697
- GENDRON ET AL.: 'Antisense transcripts of the expanded C90RF72 hexanucleotide repeat form nuclear RNA foci and undergo repeat-associated non-ATG translation in c9FTD/ALS.' ACTA NEUROPATHOL vol. 126, no. 6, 16 October 2013, pages 829 - 844, XP055334252
- RENTON ET AL.: 'A hexanucleotide repeat expansion in C90RF72 is the cause of chromosome: 9p21-linked ALS-FTD.' NEURON vol. 72, no. 2, 20 October 2011, pages 257 - 268, XP028322561

### SEQUENCE LISTING

<110> Isis Pharmaceuticals, Inc.
<120> COMPOSITIONS FOR MODULATING EXPRESSION OF C9ORF72 ANTISENSE TRANSCRIPT
<130> BIOL0236WO
<160> 94
<170> PatentIn version 3.5
<210> 1
   <211> 3339
   <212> DNA
   <213> Homo sapiens
<220>
   <221> CDS
   <222> (203)..(1648)
<400> 1
   acgtaaccta cggtgtcccg ctaggaaaga gaggtgcgtc aaacagcgac aagttccgcc        60
   cacgtaaaag atgacgcttg gtgtgtcagc cgtccctgct gcccggttgc ttctcttttg       120
   ggggcggggt ctagcaagag caggtgtggg tttaggagat atctccggag catttggata       180
   atgtgacagt tggaatgcag tg atg tcg act ctt tgc cca ccg cca tct cca        232
                            Met Ser Thr Leu Cys Pro Pro Pro Ser Pro
                            1               5                   10
   gct gtt gcc aag aca gag att gct tta agt ggc aaa tca cct tta tta         280
   Ala Val Ala Lys Thr Glu Ile Ala Leu Ser Gly Lys Ser Pro Leu Leu
                   15                  20                  25
   gca gct act ttt gct tac tgg gac aat att ctt ggt cct aga gta agg         328
   Ala Ala Thr Phe Ala Tyr Trp Asp Asn Ile Leu Gly Pro Arg Val Arg
               30                  35                  40
   cac att tgg gct cca aag aca gaa cag gta ctt ctc agt gat gga gaa         376
   His Ile Trp Ala Pro Lys Thr Glu Gln Val Leu Leu Ser Asp Gly Glu
           45                  50                  55
   ata act ttt ctt gcc aac cac act cta aat gga gaa atc ctt cga aat         424
   Ile Thr Phe Leu Ala Asn His Thr Leu Asn Gly Glu Ile Leu Arg Asn
       60                  65                  70
   gca gag agt ggt gct ata gat gta aag ttt ttt gtc ttg tct gaa aag         472
   Ala Glu Ser Gly Ala Ile Asp Val Lys Phe Phe Val Leu Ser Glu Lys
   75                  80                  85                  90
   gga gtg att att gtt tca tta atc ttt gat gga aac tgg aat ggg gat         520
   Gly Val Ile Ile Val Ser Leu Ile Phe Asp Gly Asn Trp Asn Gly Asp
                   95                  100                 105
   cgc agc aca tat gga cta tca att ata ctt cca cag aca gaa ctt agt         568
   Arg Ser Thr Tyr Gly Leu Ser Ile Ile Leu Pro Gln Thr Glu Leu Ser
               110                 115                 120
   ttc tac ctc cca ctt cat aga gtg tgt gtt gat aga tta aca cat ata         616
   Phe Tyr Leu Pro Leu His Arg Val Cys Val Asp Arg Leu Thr His Ile
           125                 130                 135
   atc cgg aaa gga aga ata tgg atg cat aag gaa aga caa gaa aat gtc        664
   Ile Arg Lys Gly Arg Ile Trp Met His Lys Glu Arg Gln Glu Asn Val
       140                 145                 150
   cag aag att atc tta gaa ggc aca gag aga atg gaa gat cag ggt cag        712
   Gln Lys Ile Ile Leu Glu Gly Thr Glu Arg Met Glu Asp Gln Gly Gln
   155                 160                 165                 170
   agt att att cca atg ctt act gga gaa gtg att cct gta atg gaa ctg        760
   Ser Ile Ile Pro Met Leu Thr Gly Glu Val Ile Pro Val Met Glu Leu
                   175                 180                 185
   ctt tca tct atg aaa tca cac agt gtt cct gaa gaa ata gat ata gct        808
   Leu Ser Ser Met Lys Ser His Ser Val Pro Glu Glu Ile Asp Ile Ala
               190                 195                 200
   gat aca gta ctc aat gat gat gat att ggt gac agc tgt cat gaa ggc        856
   Asp Thr Val Leu Asn Asp Asp Asp Ile Gly Asp Ser Cys His Glu Gly
           205                 210                 215
   ttt ctt ctc aat gcc atc agc tca cac ttg caa acc tgt ggc tgt tcc        904
   Phe Leu Leu Asn Ala Ile Ser Ser His Leu Gln Thr Cys Gly Cys Ser
       220                 225                 230
   gtt gta gta ggt agc agt gca gag aaa gta aat aag ata gtc aga aca        952
   Val Val Val Gly Ser Ser Ala Glu Lys Val Asn Lys Ile Val Arg Thr
   235                 240                 245                 250
   tta tgc ctt ttt ctg act cca gca gag aga aaa tgc tcc agg tta tgt       1000
   Leu Cys Leu Phe Leu Thr Pro Ala Glu Arg Lys Cys Ser Arg Leu Cys
                   255                 260                 265
   gaa gca gaa tca tca ttt aaa tat gag tca ggg ctc ttt gta caa ggc       1048
   Glu Ala Glu Ser Ser Phe Lys Tyr Glu Ser Gly Leu Phe Val Gln Gly
               270                 275                 280
   ctg cta aag gat tca act gga agc ttt gtg ctg cct ttc cgg caa gtc       1096
   Leu Leu Lys Asp Ser Thr Gly Ser Phe Val Leu Pro Phe Arg Gln Val
           285                 290                 295
   atg tat gct cca tat ccc acc aca cac ata gat gtg gat gtc aat act       1144
   Met Tyr Ala Pro Tyr Pro Thr Thr His Ile Asp Val Asp Val Asn Thr
       300                 305                 310
   gtg aag cag atg cca ccc tgt cat gaa cat att tat aat cag cgt aga       1192
   Val Lys Gln Met Pro Pro Cys His Glu His Ile Tyr Asn Gln Arg Arg
   315                 320                 325                 330
   tac atg aga tcc gag ctg aca gcc ttc tgg aga gcc act tca gaa gaa       1240
   Tyr Met Arg Ser Glu Leu Thr Ala Phe Trp Arg Ala Thr Ser Glu Glu
                   335                 340                 345
   gac atg gct cag gat acg atc atc tac act gac gaa agc ttt act cct       1288
   Asp Met Ala Gln Asp Thr Ile Ile Tyr Thr Asp Glu Ser Phe Thr Pro
               350                 355                 360
   gat ttg aat att ttt caa gat gtc tta cac aga gac act cta gtg aaa       1336
   Asp Leu Asn Ile Phe Gln Asp Val Leu His Arg Asp Thr Leu Val Lys
           365                 370                 375
   gcc ttc ctg gat cag gtc ttt cag ctg aaa cct ggc tta tct ctc aga       1384
   Ala Phe Leu Asp Gln Val Phe Gln Leu Lys Pro Gly Leu Ser Leu Arg
       380                 385                 390
   agt act ttc ctt gca cag ttt cta ctt gtc ctt cac aga aaa gcc ttg       1432
   Ser Thr Phe Leu Ala Gln Phe Leu Leu Val Leu His Arg Lys Ala Leu
   395                 400                 405                 410
   aca cta ata aaa tat ata gaa gac gat acg cag aag gga aaa aag ccc       1480
   Thr Leu Ile Lys Tyr Ile Glu Asp Asp Thr Gln Lys Gly Lys Lys Pro
                   415                 420                 425
   ttt aaa tct ctt cgg aac ctg aag ata gac ctt gat tta aca gca gag       1528
   Phe Lys Ser Leu Arg Asn Leu Lys Ile Asp Leu Asp Leu Thr Ala Glu
               430                 435                 440
   ggc gat ctt aac ata ata atg gct ctg gct gag aaa att aaa cca ggc       1576
   Gly Asp Leu Asn Ile Ile Met Ala Leu Ala Glu Lys Ile Lys Pro Gly
           445                 450                 455
   cta cac tct ttt atc ttt gga aga cct ttc tac act agt gtg caa gaa       1624
   Leu His Ser Phe Ile Phe Gly Arg Pro Phe Tyr Thr Ser Val Gln Glu
       460                 465                 470
   cga gat gtt cta atg act ttt taa atgtgtaact taataagcct attccatcac      1678
   Arg Asp Val Leu Met Thr Phe
   475                 480
   aatcatgatc gctggtaaag tagctcagtg gtgtggggaa acgttcccct ggatcatact     1738
   ccagaattct gctctcagca attgcagtta agtaagttac actacagttc tcacaagagc     1798
   ctgtgagggg atgtcaggtg catcattaca ttgggtgtct cttttcctag atttatgctt     1858
   ttgggataca gacctatgtt tacaatataa taaatattat tgctatcttt taaagatata     1918
   ataataggat gtaaacttga ccacaactac tgtttttttg aaatacatga ttcatggttt     1978
   acatgtgtca aggtgaaatc tgagttggct tttacagata gttgactttc tatcttttgg     2038
   cattctttgg tgtgtagaat tactgtaata cttctgcaat caactgaaaa ctagagcctt     2098
   taaatgattt caattccaca gaaagaaagt gagcttgaac ataggatgag ctttagaaag     2158
   aaaattgatc aagcagatgt ttaattggaa ttgattatta gatcctactt tgtggattta     2218
   gtccctggga ttcagtctgt agaaatgtct aatagttctc tatagtcctt gttcctggtg     2278
   aaccacagtt agggtgtttt gtttatttta ttgttcttgc tattgttgat attctatgta     2338
   gttgagctct gtaaaaggaa attgtatttt atgttttagt aattgttgcc aactttttaa     2398
   attaattttc attatttttg agccaaattg aaatgtgcac ctcctgtgcc ttttttctcc    2458
   ttagaaaatc taattacttg gaacaagttc agatttcact ggtcagtcat tttcatcttg     2518
   ttttcttctt gctaagtctt accatgtacc tgctttggca atcattgcaa ctctgagatt     2578
   ataaaatgcc ttagagaata tactaactaa taagatcttt ttttcagaaa cagaaaatag     2638
   ttccttgagt acttccttct tgcatttctg cctatgtttt tgaagttgtt gctgtttgcc     2698
   tgcaataggc tataaggaat agcaggagaa attttactga agtgctgttt tcctaggtgc     2758
   tactttggca gagctaagtt atcttttgtt ttcttaatgc gtttggacca ttttgctggc      2818
   tataaaataa ctgattaata taattctaac acaatgttga cattgtagtt acacaaacac      2878
   aaataaatat tttatttaaa attctggaag taatataaaa gggaaaatat atttataaga      2938
   aagggataaa ggtaatagag cccttctgcc ccccacccac caaatttaca caacaaaatg      2998
   acatgttcga atgtgaaagg tcataatagc tttcccatca tgaatcagaa agatgtggac      3058
   agcttgatgt tttagacaac cactgaacta gatgactgtt gtactgtagc tcagtcattt      3118
   aaaaaatata taaatactac cttgtagtgt cccatactgt gttttttaca tggtagattc      3178
   ttatttaagt gctaactggt tattttcttt ggctggttta ttgtactgtt atacagaatg      3238
   taagttgtac agtgaaataa gttattaaag catgtgtaaa cattgttata tatcttttct      3298
   cctaaatgga gaattttgaa taaaatatat ttgaaatttt g                          3339
<210> 2
   <211> 30001
   <212> DNA
   <213> Homo sapiens
<400> 2
   caaagaaaag ggggaggttt tgttaaaaaa gagaaatgtt acatagtgct ctttgagaaa 60
   attcattggc actattaagg atctgaggag ctggtgagtt tcaactggtg agtgatggtg 120
   gtagataaaa ttagagctgc agcaggtcat tttagcaact attagataaa actggtctca 180
   ggtcacaacg ggcagttgca gcagctggac ttggagagaa ttacactgtg ggagcagtgt 240
   catttgtcct aagtgctttt ctacccccta cccccactat tttagttggg tataaaaaga 300
   atgacccaat ttgtatgatc aactttcaca aagcatagaa cagtaggaaa agggtctgtt 360
   tctgcagaag gtgtagacgt tgagagccat tttgtgtatt tattcctccc tttcttcctc 420
   ggtgaatgat taaaacgttc tgtgtgattt ttagtgatga aaaagattaa atgctactca 480
   ctgtagtaag tgccatctca cacttgcaga tcaaaaggca cacagtttaa aaaacctttg 540
   tttttttaca catctgagtg gtgtaaatgc tactcatctg tagtaagtgg aatctataca 600
   cctgcagacc aaaagacgca aggtttcaaa aatctttgtg ttttttacac atcaaacaga 660
   atggtacgtt tttcaaaagt taaaaaaaaa caactcatcc acatattgca actagcaaaa 720
   atgacattcc ccagtgtgaa aatcatgctt gagagaattc ttacatgtaa aggcaaaatt 780
   gcgatgactt tgcaggggac cgtgggattc ccgcccgcag tgccggagct gtcccctacc 840
   agggtttgca gtggagtttt gaatgcactt aacagtgtct tacggtaaaa acaaaatttc 900
   atccaccaat tatgtgttga gcgcccactg cctaccaagc acaaacaaaa ccattcaaaa 960
   ccacgaaatc gtcttcactt tctccagatc cagcagcctc ccctattaag gttcgcacac 1020
   gctattgcgc caacgctcct ccagagcggg tcttaagata aaagaacagg acaagttgcc 1080
   ccgccccatt tcgctagcct cgtgagaaaa cgtcatcgca catagaaaac agacagacgt 1140
   aacctacggt gtcccgctag gaaagagagg tgcgtcaaac agcgacaagt tccgcccacg 1200
   taaaagatga cgcttggtgt gtcagccgtc cctgctgccc ggttgcttct cttttggggg 1260
   cggggtctag caagagcagg tgtgggttta ggaggtgtgt gtttttgttt ttcccaccct 1320
   ctctccccac tacttgctct cacagtactc gctgagggtg aacaagaaaa gacctgataa 1380
   agattaacca gaagaaaaca aggagggaaa caaccgcagc ctgtagcaag ctctggaact 1440
   caggagtcgc gcgctagggg ccggggccgg ggccggggcg tggtcggggc gggcccgggg 1500
   gcgggcccgg ggcggggctg cggttgcggt gcctgcgccc gcggcggcgg aggcgcaggc 1560
   ggtggcgagt gggtgagtga ggaggcggca tcctggcggg tggctgtttg gggttcggct 1620
   gccgggaaga ggcgcgggta gaagcggggg ctctcctcag agctcgacgc atttttactt 1680
   tccctctcat ttctctgacc gaagctgggt gtcgggcttt cgcctctagc gactggtgga 1740
   attgcctgca tccgggcccc gggcttcccg gcggcggcgg cggcggcggc ggcgcaggga 1800
   caagggatgg ggatctggcc tcttccttgc tttcccgccc tcagtacccg agctgtctcc 1860
   ttcccgggga cccgctggga gcgctgccgc tgcgggctcg agaaaaggga gcctcgggta 1920
   ctgagaggcc tcgcctgggg gaaggccgga gggtgggcgg cgcgcggctt ctgcggacca 1980
   agtcggggtt cgctaggaac ccgagacggt ccctgccggc gaggagatca tgcgggatga 2040
   gatgggggtg tggagacgcc tgcacaattt cagcccaagc ttctagagag tggtgatgac 2100
   ttgcatatga gggcagcaat gcaagtcggt gtgctcccca ttctgtggga catgacctgg 2160
   ttgcttcaca gctccgagat gacacagact tgcttaaagg aagtgactat tgtgacttgg 2220
   gcatcacttg actgatggta atcagttgtc taaagaagtg cacagattac atgtccgtgt 2280
   gctcattggg tctatctggc cgcgttgaac accaccaggc tttgtattca gaaacaggag 2340
   ggaggtcctg cactttccca ggaggggtgg ccctttcaga tgcaatcgag attgttaggc 2400
   tctgggagag tagttgcctg gttgtggcag ttggtaaatt tctattcaaa cagttgccat 2460
   gcaccagttg ttcacaacaa gggtacgtaa tctgtctggc attacttcta cttttgtaca 2520
   aaggatcaaa aaaaaaaaag atactgttaa gatatgattt ttctcagact ttgggaaact 2580
   tttaacataa tctgtgaata tcacagaaac aagactatca tataggggat attaataacc 2640
   tggagtcaga atacttgaaa tacggtgtca tttgacacgg gcattgttgt caccacctct 2700
   gccaaggcct gccactttag gaaaaccctg aatcagttgg aaactgctac atgctgatag 2760
   tacatctgaa acaagaacga gagtaattac cacattccag attgttcact aagccagcat 2820
   ttacctgctc caggaaaaaa ttacaagcac cttatgaagt tgataaaata ttttgtttgg 2880
   ctatgttggc actccacaat ttgctttcag agaaacaaag taaaccaagg aggacttctg 2940
   tttttcaagt ctgccctcgg gttctattct acgttaatta gatagttccc aggaggacta 3000
   ggttagccta cctattgtct gagaaacttg gaactgtgag aaatggccag atagtgatat 3060
   gaacttcacc ttccagtctt ccctgatgtt gaagattgag aaagtgttgt gaactttctg 3120
   gtactgtaaa cagttcactg tccttgaagt ggtcctgggc agctcctgtt gtggaaagtg 3180
   gacggtttag gatcctgctt ctctttgggc tgggagaaaa taaacagcat ggttacaagt 3240
   attgagagcc aggttggaga aggtggctta cacctgtaat gccagagctt tgggaggcgg 3300
   aggcaagagg atcacttgaa gccaggagtt caagctcaac ctgggcaacg tagaccctgt 3360
   ctctacaaaa aattaaaaac ttagccgggc gtggtgatgt gcacctgtag tcctagctac 3420
   ttgggaggct gaggcaggag ggtcatttga gcccaagagt ttgaagttac cgagagctat 3480
   gatcctgcca gtgcattcca gcctggatga caaaacgaga ccctgtctct aaaaaacaag 3540
   aagtgagggc tttatgattg tagaattttc actacaatag cagtggacca accacctttc 3600
   taaataccaa tcagggaaga gatggttgat tttttaacag acgtttaaag aaaaagcaaa 3660
   acctcaaact tagcactcta ctaacagttt tagcagatgt taattaatgt aatcatgtct 3720
   gcatgtatgg gattatttcc agaaagtgta ttgggaaacc tctcatgaac cctgtgagca 3780
   agccaccgtc tcactcaatt tgaatcttgg cttccctcaa aagactggct aatgtttggt 3840
   aactctctgg agtagacagc actacatgta cgtaagatag gtacataaac aactattggt 3900
   tttgagctga tttttttcag ctgcatttgc atgtatggat ttttctcacc aaagacgatg 3960
   acttcaagta ttagtaaaat aattgtacag ctctcctgat tatacttctc tgtgacattt 4020
   catttcccag gctatttctt ttggtaggat ttaaaactaa gcaattcagt atgatctttg 4080
   tccttcattt tctttcttat tctttttgtt tgtttgtttg tttgtttttt tcttgaggca 4140
   gagtctctct ctgtcgccca ggctggagtg cagtggcgcc atctcagctc attgcaacct 4200
   ctgccacctc cgggttcaag agattctcct gcctcagcct cccgagtagc tgggattaca 4260
   ggtgtccacc accacacccg gctaattttt tgtattttta gtagaggtgg ggtttcacca 4320
   tgttggccag gctggtcttg agctcctgac ctcaggtgat ccacctgcct cggcctacca 4380
   aagagctggg ataacaggtg tgacccacca tgcccggccc attttttttt tcttattctg 4440
   ttaggagtga gagtgtaact agcagtataa tagttcaatt ttcacaacgt ggtaaaagtt 4500
   tccctataat tcaatcagat tttgctccag ggttcagttc tgttttagga aatactttta 4560
   ttttcagttt aatgatgaaa tattagagtt gtaatattgc ctttatgatt atccaccttt 4620
   ttaacctaaa agaatgaaag aaaaatatgt ttgcaatata attttatggt tgtatgttaa 4680
   cttaattcat tatgttggcc tccagtttgc tgttgttagt tatgacagca gtagtgtcat 4740
   taccatttca attcagatta cattcctata tttgatcatt gtaaactgac tgcttacatt 4800
   gtattaaaaa cagtggatat tttaaagaag ctgtacggct tatatctagt gctgtctctt 4860
   aagactatta aattgataca acatatttaa aagtaaatat tacctaaatg aatttttgaa 4920
   attacaaata cacgtgttaa aactgtcgtt gtgttcaacc atttctgtac atacttagag 4980
   ttaactgttt tgccaggctc tgtatgccta ctcataatat gataaaagca ctcatctaat 5040
   gctctgtaaa tagaagtcag tgctttccat cagactgaac tctcttgaca agatgtggat 5100
   gaaattcttt aagtaaaatt gtttactttg tcatacattt acagatcaaa tgttagctcc 5160
   caaagcaatc atatggcaaa gataggtata tcatagtttg cctattagct gctttgtatt 5220
   gctattatta taaatagact tcacagtttt agacttgctt aggtgaaatt gcaattcttt 5280
   ttactttcag tcttagataa caagtcttca attatagtac aatcacacat tgcttaggaa 5340
   tgcatcatta ggcgattttg tcattatgca aacatcatag agtgtactta cacaaaccta 5400
   gatagtatag cctttatgta cctaggccgt atggtatagt ctgttgctcc taggccacaa 5460
   acctgtacaa ctgttactgt actgaatact atagacagtt gtaacacagt ggtaaatatt 5520
   tatctaaata tatgcaaaca gagaaaaggt acagtaaaag tatggtataa aagataatgg 5580
   tatacctgtg taggccactt accacgaatg gagcttgcag gactagaagt tgctctgggt 5640
   gagtcagtga gtgagtggtg aattaatgtg aaggcctaga acactgtaca ccactgtaga 5700
   ctataaacac agtacgctga agctacacca aatttatctt aacagttttt cttcaataaa 5760
   aaattataac tttttaactt tgtaaacttt ttaatttttt aacttttaaa atacttagct 5820
   tgaaacacaa atacattgta tagctataca aaaatatttt ttctttgtat ccttattcta 5880
   gaagcttttt tctattttct attttaaatt ttttttttta cttgttagtc gtttttgtta 5940
   aaaactaaaa cacacacact ttcacctagg catagacagg attaggatca tcagtatcac 6000
   tcccttccac ctcactgcct tccacctcca catcttgtcc cactggaagg tttttagggg 6060
   caataacaca catgtagctg tcacctatga taacagtgct ttctgttgaa tacctcctga 6120
   aggacttgcc tgaggctgtt ttacatttaa cttaaaaaaa aaaaaagtag aaggagtgca 6180
   ctctaaaata acaataaaag gcatagtata gtgaatacat aaaccagcaa tgtagtagtt 6240
   tattatcaag tgttgtacac tgtaataatt gtatgtgcta tactttaaat aacttgcaaa 6300
   atagtactaa gaccttatga tggttacagt gtcactaagg caatagcata ttttcaggtc 6360
   cattgtaatc taatgggact accatcatat atgcagtcta ccattgactg aaacgttaca 6420
   tggcacataa ctgtatttgc aagaatgatt tgttttacat taatatcaca taggatgtac 6480
   ctttttagag tggtatgttt atgtggatta agatgtacaa gttgagcaag gggaccaaga 6540
   gccctgggtt ctgtcttgga tgtgagcgtt tatgttcttc tcctcatgtc tgttttctca 6600
   ttaaattcaa aggcttgaac gggccctatt tagcccttct gttttctacg tgttctaaat 6660
   aactaaagct tttaaattct agccatttag tgtagaactc tctttgcagt gatgaaatgc 6720
   tgtattggtt tcttggctag catattaaat atttttatct ttgtcttgat acttcaatgt 6780
   cgttttaaac atcaggatcg ggcttcagta ttctcataac cagagagttc actgaggata 6840
   caggactgtt tgcccatttt ttgttatggc tccagacttg tggtatttcc atgtcttttt 6900
   tttttttttt ttttttgacc ttttagcggc tttaaagtat ttctgttgtt aggtgttgta 6960
   ttacttttct aagattactt aacaaagcac cacaaactga gtggctttaa acaacagcaa 7020
   tttattctct cacaattcta gaagctagaa gtccgaaatc aaagtgttga caggggcatg 7080
   atcttcaaga gagaagactc tttccttgcc tcttcctggc ttctggtggt taccagcaat 7140
   cctgagtgtt cctttcttgc cttgtagttt caacaatcca gtatctgcct tttgtcttca 7200
   catggctgtc taccatttgt ctctgtgtct ccaaatctct ctccttataa acacagcagt 7260
   tattggatta ggccccactc taatccagta tgaccccatt ttaacatgat tacacttatt 7320
   tctagataag gtcacattca cgtacaccaa gggttaggaa ttgaacatat ctttttgggg 7380
   gacacaattc aacccacaag tgtcagtctc tagctgagcc tttcccttcc tgtttttctc 7440
   ctttttagtt gctatgggtt aggggccaaa tctccagtca tactagaatt gcacatggac 7500
   tggatatttg ggaatactgc gggtctattc tatgagcttt agtatgtaac atttaatatc 7560
   agtgtaaaga agcccttttt taagttattt ctttgaattt ctaaatgtat gccctgaata 7620
   taagtaacaa gttaccatgt cttgtaaaat gatcatatca acaaacattt aatgtgcacc 7680
   tactgtgcta gttgaatgtc tttatcctga taggagataa caggattcca catctttgac 7740
   ttaagaggac aaaccaaata tgtctaaatc atttggggtt ttgatggata tctttaaatt 7800
   gctgaaccta atcattggtt tcatatgtca ttgtttagat atctccggag catttggata 7860
   atgtgacagt tggaatgcag tgatgtcgac tctttgccca ccgccatctc cagctgttgc 7920
   caagacagag attgctttaa gtggcaaatc acctttatta gcagctactt ttgcttactg 7980
   ggacaatatt cttggtccta gagtaaggca catttgggct ccaaagacag aacaggtact 8040
   tctcagtgat ggagaaataa cttttcttgc caaccacact ctaaatggag aaatccttcg 8100
   aaatgcagag agtggtgcta tagatgtaaa gttttttgtc ttgtctgaaa agggagtgat 8160
   tattgtttca ttaatctttg atggaaactg gaatggggat cgcagcacat atggactatc 8220
   aattatactt ccacagacag aacttagttt ctacctccca cttcatagag tgtgtgttga 8280
   tagattaaca catataatcc ggaaaggaag aatatggatg cataaggtaa gtgatttttc 8340
   agcttattaa tcatgttaac ctatctgttg aaagcttatt ttctggtaca tataaatctt 8400
   atttttttaa ttatatgcag tgaacatcaa acaataaatg ttatttattt tgcatttacc 8460
   ctattagata caaatacatc tggtctgata cctgtcatct tcatattaac tgtggaaggt 8520
   acgaaatggt agctccacat tatagatgaa aagctaaagc ttagacaaat aaagaaactt 8580
   ttagaccctg gattcttctt gggagccttt gactctaata ccttttgttt ccctttcatt 8640
   gcacaattct gtcttttgct tactactatg tgtaagtata acagttcaaa gtaatagttt 8700
   cataagctgt tggtcatgta gcctttggtc tctttaacct ctttgccaag ttcccaggtt 8760
   cataaaatga ggaggttgaa tggaatggtt cccaagagaa ttccttttaa tcttacagaa 8820
   attattgttt tcctaaatcc tgtagttgaa tatataatgc tatttacatt tcagtatagt 8880
   tttgatgtat ctaaagaaca cattgaattc tccttcctgt gttccagttt gatactaacc 8940
   tgaaagtcca ttaagcatta ccagttttaa aaggcttttg cccaatagta aggaaaaata 9000
   atatctttta aaagaataat tttttactat gtttgcaggc ttacttcctt ttttctcaca 9060
   ttatgaaact cttaaaatca ggagaatctt ttaaacaaca tcataatgtt taatttgaaa 9120
   agtgcaagtc attcttttcc tttttgaaac tatgcagatg ttacattgac tgttttctgt 9180
   gaagttatct ttttttcact gcagaataaa ggttgttttg attttatttt gtattgttta 9240
   tgagaacatg catttgttgg gttaatttcc tacccctgcc cccatttttt ccctaaagta 9300
   gaaagtattt ttcttgtgaa ctaaattact acacaagaac atgtctattg aaaaataagc 9360
   aagtatcaaa atgttgtggg ttgttttttt aaataaattt tctcttgctc aggaaagaca 9420
   agaaaatgtc cagaagatta tcttagaagg cacagagaga atggaagatc aggtatatgc 9480
   aaattgcata ctgtcaaatg tttttctcac agcatgtatc tgtataaggt tgatggctac 9540
   atttgtcaag gccttggaga catacgaata agcctttaat ggagctttta tggaggtgta 9600
   cagaataaac tggaggaaga tttccatatc ttaaacccaa agagttaaat cagtaaacaa 9660
   aggaaaatag taattgcatc tacaaattaa tatttgctcc cttttttttt ctgtttgccc 9720
   agaataaatt ttggataact tgttcatagt aaaaataaaa aaaattgtct ctgatatgtt 9780
   ctttaaggta ctacttctcg aacctttccc tagaagtagc tgtaacagaa ggagagcata 9840
   tgtacccctg aggtatctgt ctggggtgta ggcccaggtc cacacaatat ttcttctaag 9900
   tcttatgttg tatcgttaag actcatgcaa tttacatttt attccataac tattttagta 9960
   ttaaaatttg tcagtgatat ttcttaccct ctcctctagg aaaatgtgcc atgtttatcc 10020
   cttggctttg aatgcccctc aggaacagac actaagagtt tgagaagcat ggttacaagg 10080
   gtgtggcttc ccctgcggaa actaagtaca gactatttca ctgtaaagca gagaagttct 10140
   tttgaaggag aatctccagt gaagaaagag ttcttcactt ttacttccat ttcctcttgt 10200
   gggtgaccct caatgctcct tgtaaaactc caatatttta aacatggctg ttttgccttt 10260
   ctttgcttct ttttagcatg aatgagacag atgatacttt aaaaaagtaa ttaaaaaaaa 10320
   aaacttgtga aaatacatgg ccataataca gaacccaata caatgatctc ctttaccaaa 10380
   ttgttatgtt tgtacttttg tagatagctt tccaattcag agacagttat tctgtgtaaa 10440
   ggtctgactt aacaagaaaa gatttccctt tacccaaaga atcccagtcc ttatttgctg 10500
   gtcaataagc agggtcccca ggaatggggt aactttcagc accctctaac ccactagtta 10560
   ttagtagact aattaagtaa acttatcgca agttgaggaa acttagaacc aactaaaatt 10620
   ctgcttttac tgggattttg ttttttcaaa ccagaaacct ttacttaagt tgactactat 10680
   taatgaattt tggtctctct tttaagtgct cttcttaaaa atgttatctt actgctgaga 10740
   agttcaagtt tgggaagtac aaggaggaat agaaacttaa gagattttct tttagagcct 10800
   cttctgtatt tagccctgta ggattttttt tttttttttt ttttttggtg ttgttgagct 10860
   tcagtgaggc tattcattca cttatactga taatgtctga gatactgtga atgaaatact 10920
   atgtatgctt aaacctaaga ggaaatattt tcccaaaatt attcttcccg aaaaggagga 10980
   gttgcctttt gattgagttc ttgcaaatct cacaacgact ttattttgaa caatactgtt 11040
   tggggatgat gcattagttt gaaacaactt cagttgtagc tgtcatctga taaaattgct 11100
   tcacagggaa ggaaatttaa cacggatcta gtcattattc ttgttagatt gaatgtgtga 11160
   attgtaattg taaacaggca tgataattat tactttaaaa actaaaaaca gtgaatagtt 11220
   agttgtggag gttactaaag gatggttttt ttttaaataa aactttcagc attatgcaaa 11280
   tgggcatatg gcttaggata aaacttccag aagtagcatc acatttaaat tctcaagcaa 11340
   cttaataata tggggctctg aaaaactggt taaggttact ccaaaaatgg ccctgggtct 11400
   gacaaagatt ctaacttaaa gatgcttatg aagactttga gtaaaatcat ttcataaaat 11460
   aagtgaggaa aaacaactag tattaaattc atcttaaata atgtatgatt taaaaaatat 11520
   gtttagctaa aaatgcatag tcatttgaca atttcattta tatctcaaaa aatttactta 11580
   accaagttgg tcacaaaact gatgagactg gtggtggtag tgaataaatg agggaccatc 11640
   catatttgag acactttaca tttgtgatgt gttatactga attttcagtt tgattctata 11700
   gactacaaat ttcaaaatta caatttcaag atgtaataag tagtaatatc ttgaaatagc 11760
   tctaaaggga atttttctgt tttattgatt cttaaaatat atgtgctgat tttgatttgc 11820
   atttgggtag attatacttt tatgagtatg gaggttaggt attgattcaa gttttcctta 11880
   cctatttggt aaggatttca aagtcttttt gtgcttggtt ttcctcattt ttaaatatga 11940
   aatatattga tgacctttaa caaatttttt ttatctcaaa ttttaaagga gatcttttct 12000
   aaaagaggca tgatgactta atcattgcat gtaacagtaa acgataaacc aatgattcca 12060
   tactctctaa agaataaaag tgagctttag ggccgggcat ggtcagaaat ttgacaccaa 12120
   cctggccaac atggcgaaac cccgtctcta ctaaaaatac aaaaatcagc cgggcatggt 12180
   ggcggcacct atagtcccag ctacttggga ggatgagaca ggagagtcac ttgaacctgg 12240
   gaggagaggt tgcagtgagc tgagatcacg ccattgcact ccagcctgag caatgaaagc 12300
   aaaactccat ctcaaaaaaa aaaaaagaaa agaaagaata aaagtgagct ttggattgca 12360
   tataaatcct ttagacatgt agtagacttg tttgatactg tgtttgaaca aattacgaag 12420
   tattttcatc aaagaatgtt attgtttgat gttattttta ttttttattg cccagcttct 12480
   ctcatattac gtgattttct tcacttcatg tcactttatt gtgcagggtc agagtattat 12540
   tccaatgctt actggagaag tgattcctgt aatggaactg ctttcatcta tgaaatcaca 12600
   cagtgttcct gaagaaatag atgtaagttt aaatgagagc aattatacac tttatgagtt 12660
   ttttggggtt atagtattat tatgtatatt attaatattc taattttaat agtaaggact 12720
   ttgtcataca tactattcac atacagtatt agccacttta gcaaataagc acacacaaaa 12780
   tcctggattt tatggcaaaa cagaggcatt tttgatcagt gatgacaaaa ttaaattcat 12840
   tttgtttatt tcattacttt tataattcct aaaagtggga ggatcccagc tcttatagga 12900
   gcaattaata tttaatgtag tgtcttttga aacaaaactg tgtgccaaag tagtaaccat 12960
   taatggaagt ttacttgtag tcacaaattt agtttcctta atcatttgtt gaggacgttt 13020
   tgaatcacac actatgagtg ttaagagata cctttaggaa actattcttg ttgttttctg 13080
   attttgtcat ttaggttagt ctcctgattc tgacagctca gaagaggaag ttgttcttgt 13140
   aaaaattgtt taacctgctt gaccagcttt cacatttgtt cttctgaagt ttatggtagt 13200
   gcacagagat tgttttttgg ggagtcttga ttctcggaaa tgaaggcagt gtgttatatt 13260
   gaatccagac ttccgaaaac ttgtatatta aaagtgttat ttcaacacta tgttacagcc 13320
   agactaattt ttttattttt tgatgcattt tagatagctg atacagtact caatgatgat 13380
   gatattggtg acagctgtca tgaaggcttt cttctcaagt aagaattttt cttttcataa 13440
   aagctggatg aagcagatac catcttatgc tcacctatga caagatttgg aagaaagaaa 13500
   ataacagact gtctacttag attgttctag ggacattacg tatttgaact gttgcttaaa 13560
   tttgtgttat ttttcactca ttatatttct atatatattt ggtgttattc catttgctat 13620
   ttaaagaaac cgagtttcca tcccagacaa gaaatcatgg ccccttgctt gattctggtt 13680
   tcttgtttta cttctcatta aagctaacag aatcctttca tattaagttg tactgtagat 13740
   gaacttaagt tatttaggcg tagaacaaaa ttattcatat ttatactgat ctttttccat 13800
   ccagcagtgg agtttagtac ttaagagttt gtgcccttaa accagactcc ctggattaat 13860
   gctgtgtacc cgtgggcaag gtgcctgaat tctctataca cctatttcct catctgtaaa 13920
   atggcaataa tagtaatagt acctaatgtg tagggttgtt ataagcattg agtaagataa 13980
   ataatataaa gcacttagaa cagtgcctgg aacataaaaa cacttaataa tagctcatag 14040
   ctaacatttc ctatttacat ttcttctaga aatagccagt atttgttgag tgcctacatg 14100
   ttagttcctt tactagttgc tttacatgta ttatcttata ttctgtttta aagtttcttc 14160
   acagttacag attttcatga aattttactt ttaataaaag agaagtaaaa gtataaagta 14220
   ttcactttta tgttcacagt cttttccttt aggctcatga tggagtatca gaggcatgag 14280
   tgtgtttaac ctaagagcct taatggcttg aatcagaagc actttagtcc tgtatctgtt 14340
   cagtgtcagc ctttcataca tcattttaaa tcccatttga ctttaagtaa gtcacttaat 14400
   ctctctacat gtcaatttct tcagctataa aatgatggta tttcaataaa taaatacatt 14460
   aattaaatga tattatactg actaattggg ctgttttaag gctcaataag aaaatttctg 14520
   tgaaaggtct ctagaaaatg taggttccta tacaaataaa agataacatt gtgcttatag 14580
   cttcggtgtt tatcatataa agctattctg agttatttga agagctcacc tacttttttt 14640
   tgtttttagt ttgttaaatt gttttatagg caatgttttt aatctgtttt ctttaactta 14700
   cagtgccatc agctcacact tgcaaacctg tggctgttcc gttgtagtag gtagcagtgc 14760
   agagaaagta aataaggtag tttattttat aatctagcaa atgatttgac tctttaagac 14820
   tgatgatata tcatggattg tcatttaaat ggtaggttgc aattaaaatg atctagtagt 14880
   ataaggaggc aatgtaatct catcaaattg ctaagacacc ttgtggcaac agtgagtttg 14940
   aaataaactg agtaagaatc atttatcagt ttattttgat agctcggaaa taccagtgtc 15000
   agtagtgtat aaatggtttt gagaatatat taaaatcaga tatataaaaa aaattactct 15060
   tctatttccc aatgttatct ttaacaaatc tgaagatagt catgtacttt tggtagtagt 15120
   tccaaagaaa tgttatttgt ttattcatct tgatttcatt gtcttcgctt tccttctaaa 15180
   tctgtccctt ctagggagct attgggatta agtggtcatt gattattata ctttattcag 15240
   taatgtttct gaccctttcc ttcagtgcta cttgagttaa ttaaggatta atgaacagtt 15300
   acatttccaa gcattagcta ataaactaaa ggattttgca cttttcttca ctgaccatta 15360
   gttagaaaga gttcagagat aagtatgtgt atctttcaat ttcagcaaac ctaatttttt 15420
   aaaaaaagtt ttacatagga aatatgttgg aaatgatact ttacaaagat attcataatt 15480
   tttttttgta atcagctact ttgtatattt acatgagcct taatttatat ttctcatata 15540
   accatttatg agagcttagt atacctgtgt cattatattg catctacgaa ctagtgacct 15600
   tattccttct gttacctcaa acaggtggct ttccatctgt gatctccaaa gccttaggtt 15660
   gcacagagtg actgccgagc tgctttatga agggagaaag gctccatagt tggagtgttt 15720
   tttttttttt ttttaaacat ttttcccatc ctccatcctc ttgagggaga atagcttacc 15780
   ttttatcttg ttttaatttg agaaagaagt tgccaccact ctaggttgaa aaccactcct 15840
   ttaacataat aactgtggat atggtttgaa tttcaagata gttacatgcc tttttatttt 15900
   tcctaataga gctgtaggtc aaatattatt agaatcagat ttctaaatcc cacccaatga 15960
   cctgcttatt ttaaatcaaa ttcaataatt aattctcttc tttttggagg atctggacat 16020
   tctttgatat ttcttacaac gaatttcatg tgtagaccca ctaaacagaa gctataaaag 16080
   ttgcatggtc aaataagtct gagaaagtct gcagatgata taattcacct gaagagtcac 16140
   agtatgtagc caaatgttaa aggttttgag atgccataca gtaaatttac caagcatttt 16200
   ctaaatttat ttgaccacag aatccctatt ttaagcaaca actgttacat cccatggatt 16260
   ccaggtgact aaagaatact tatttcttag gatatgtttt attgataata acaattaaaa 16320
   tttcagatat ctttcataag caaatcagtg gtctttttac ttcatgtttt aatgctaaaa 16380
   tattttcttt tatagatagt cagaacatta tgcctttttc tgactccagc agagagaaaa 16440
   tgctccaggt tatgtgaagc agaatcatca tttaaatatg agtcagggct ctttgtacaa 16500
   ggcctgctaa aggtatagtt tctagttatc acaagtgaaa ccacttttct aaaatcattt 16560
   ttgagactct ttatagacaa atcttaaata ttagcattta atgtatctca tattgacatg 16620
   cccagagact gacttccttt acacagttct gcacatagac tatatgtctt atggatttat 16680
   agttagtatc atcagtgaaa caccatagaa taccctttgt gttccaggtg ggtccctgtt 16740
   cctacatgtc tagcctcagg actttttttt ttttaacaca tgcttaaatc aggttgcaca 16800
   tcaaaaataa gatcatttct ttttaactaa atagatttga attttattga aaaaaaattt 16860
   taaacatctt taagaagctt ataggattta agcaattcct atgtatgtgt actaaaatat 16920
   atatatttct atatataata tatattagaa aaaaattgta tttttctttt atttgagtct 16980
   actgtcaagg agcaaaacag agaaatgtaa attagcaatt atttataata cttaaaggga 17040
   agaaagttgt tcaccttgtt gaatctatta ttgttatttc aattatagtc ccaagacgtg 17100
   aagaaatagc tttcctaatg gttatgtgat tgtctcatag tgactacttt cttgaggatg 17160
   tagccacggc aaaatgaaat aaaaaaattt aaaaattgtt gcaaatacaa gttatattag 17220
   gcttttgtgc attttcaata atgtgctgct atgaactcag aatgatagta tttaaatata 17280
   gaaactagtt aaaggaaacg tagtttctat ttgagttata catatctgta aattagaact 17340
   tctcctgtta aaggcataat aaagtgctta atacttttgt ttcctcagca ccctctcatt 17400
   taattatata attttagttc tgaaagggac ctataccaga tgcctagagg aaatttcaaa 17460
   actatgatct aatgaaaaaa tatttaatag ttctccatgc aaatacaaat catatagttt 17520
   tccagaaaat acctttgaca ttatacaaag atgattatca cagcattata atagtaaaaa 17580
   aatggaaata gcctctttct tctgttctgt tcatagcaca gtgcctcata cgcagtaggt 17640
   tattattaca tggtaactgg ctaccccaac tgattaggaa agaagtaaat ttgttttata 17700
   aaaatacata ctcattgagg tgcatagaat aattaagaaa ttaaaagaca cttgtaattt 17760
   tgaatccagt gaatacccac tgttaatatt tggtatatct ctttctagtc tttttttccc 17820
   ttttgcatgt attttcttta agactcccac ccccactgga tcatctctgc atgttctaat 17880
   ctgctttttt cacagcagat tctaagcctc tttgaatatc aacacaaact tcaacaactt 17940
   catctataga tgccaaataa taaattcatt tttatttact taaccacttc ctttggatgc 18000
   ttaggtcatt ctgatgtttt gctattgaaa ccaatgctat actgaacact tctgtcacta 18060
   aaactttgca cacactcatg aatagcttct taggataaat ttttagagat ggatttgcta 18120
   aatcagagac cattttttaa aattaaaaaa caattattca tatcgtttgg catgtaagac 18180
   agtaaatttt ccttttattt tgacaggatt caactggaag ctttgtgctg cctttccggc 18240
   aagtcatgta tgctccatat cccaccacac acatagatgt ggatgtcaat actgtgaagc 18300
   agatgccacc ctgtcatgaa catatttata atcagcgtag atacatgaga tccgagctga 18360
   cagccttctg gagagccact tcagaagaag acatggctca ggatacgatc atctacactg 18420
   acgaaagctt tactcctgat ttgtacgtaa tgctctgcct gctggtactg tagtcaagca 18480
   atatgaaatt gtgtctttta cgaataaaaa caaaacagaa gttgcattta aaaagaaaga 18540
   aatattacca gcagaattat gcttgaagaa acatttaatc aagcattttt ttcttaaatg 18600
   ttcttctttt tccatacaat tgtgtttacc ctaaaatagg taagattaac ccttaaagta 18660
   aatatttaac tatttgttta ataaatatat attgagctcc taggcactgt tctaggtacc 18720
   gggcttaata gtggccaacc agacagcccc agccccagcc cctacattgt gtatagtcta 18780
   ttatgtaaca gttattgaat ggacttatta acaaaaccaa agaagtaatt ctaagtcttt 18840
   tttttcttga catatgaata taaaatacag caaaactgtt aaaatatatt aatggaacat 18900
   ttttttactt tgcattttat attgttattc acttcttatt tttttttaaa aaaaaaagcc 18960
   tgaacagtaa attcaaaagg aaaagtaatg ataattaatt gttgagcatg gacccaactt 19020
   gaaaaaaaaa atgatgatga taaatctata atcctaaaac cctaagtaaa cacttaaaag 19080
   atgttctgaa atcaggaaaa gaattatagt atacttttgt gtttctcttt tatcagttga 19140
   aaaaaggcac agtagctcat gcctgtaaga acagagcttt gggagtgcaa ggcaggcgga 19200
   tcacttgagg ccaggagttc cagaccagcc tgggcaacat agtgaaaccc catctctaca 19260
   aaaaataaaa aagaattatt ggaatgtgtt tctgtgtgcc tgtaatccta gctattccga 19320
   aagctgaggc aggaggatct tttgagccca ggagtttgag gttacaggga gttatgatgt 19380
   gccagtgtac tccagcctgg ggaacaccga gactctgtct tatttaaaaa aaaaaaaaaa 19440
   aaaatgcttg caataatgcc tggcacatag aaggtaacag taagtgttaa ctgtaataac 19500
   ccaggtctaa gtgtgtaagg caatagaaaa attggggcaa ataagcctga cctatgtatc 19560
   tacagaatca gtttgagctt aggtaacaga cctgtggagc accagtaatt acacagtaag 19620
   tgttaaccaa aagcatagaa taggaatatc ttgttcaagg gacccccagc cttatacatc 19680
   tcaaggtgca gaaagatgac ttaatatagg acccattttt tcctagttct ccagagtttt 19740
   tattggttct tgagaaagta gtaggggaat gttttagaaa atgaattggt ccaactgaaa 19800
   ttacatgtca gtaagttttt atatattggt aaattttagt agacatgtag aagttttcta 19860
   attaatctgt gccttgaaac atttcttttt ttcctaaagt gcttagtatt ttttccgttt 19920
   tttgattggt tacttgggag cttttttgag gaaatttagt gaactgcaga atgggtttgc 19980
   aaccatttgg tatttttgtt ttgtttttta gaggatgtat gtgtatttta acatttctta 20040
   atcattttta gccagctatg tttgttttgc tgatttgaca aactacagtt agacagctat 20100
   tctcattttg ctgatcatga caaaataata tcctgaattt ttaaattttg catccagctc 20160
   taaattttct aaacataaaa ttgtccaaaa aatagtattt tcagccacta gattgtgtgt 20220
   taagtctatt gtcacagagt cattttactt ttaagtatat gtttttacat gttaattatg 20280
   tttgttattt ttaattttaa ctttttaaaa taattccagt cactgccaat acatgaaaaa 20340
   ttggtcactg gaattttttt tttgactttt attttaggtt catgtgtaca tgtgcaggtg 20400
   tgttatacag gtaaattgcg tgtcatgagg gtttggtgta caggtgattt cattacccag 20460
   gtaataagca tagtacccaa taggtagttt tttgatcctc acccttctcc caccctcaag 20520
   taggccctgg tgttgctgtt tccttctttg tgtccatgta tactcagtgt ttagctccca 20580
   cttagaagtg agaacatgcg gtagttggtt ttctgttcct ggattagttc acttaggata 20640
   atgacctcta gctccatctg gtttttatgg ctgcatagta ttccatggtg tatatgtatc 20700
   acattttctt tatccagtct accattgata ggcatttagg ttgattccct gtctttgtta 20760
   tcatgaatag tgctgtgatg aacatacaca tgcatgtgtc tttatggtag aaaaatttgt 20820
   attcctttag gtacatatag aataatgggg ttgctagggt gaatggtagt tctattttca 20880
   gttatttgag aaatcttcaa actgcttttc ataatagcta aactaattta cagtcccgcc 20940
   agcagtgtat aagtgttccc ttttctccac aaccttgcca acatctgtga ttttttgact 21000
   ttttaataat agccattcct agagaattga tttgcaattc tctattagtg atattaagca 21060
   ttttttcata tgctttttag ctgtctgtat atattcttct gaaaaatttt catgtccttt 21120
   gcccagtttg tagtggggtg ggttgttttt tgcttgttaa ttagttttaa gttccttcca 21180
   gattctgcat atccctttgt tggatacatg gtttgcagat atttttctcc cattgtgtag 21240
   gttgtctttt actctgttga tagtttcttt tgccatgcag gagctcgtta ggtcccattt 21300
   gtgtttgttt ttgttgcagt tgcttttggc gtcttcatca taaaatctgt gccagggcct 21360
   atgtccagaa tggtatttcc taggttgtct tccagggttt ttacaatttt agattttacg 21420
   tttatgtctt taatccatct tgagttgatt tttgtatatg gcacaaggaa ggggtccagt 21480
   ttcactccaa ttcctatggc tagcaattat cccagcacca tttattgaat acggagtcct 21540
   ttccccattg cttgtttttt gtcaactttg ttgaagatca gatggttgta agtgtgtggc 21600
   tttatttctt ggctctctat tctccattgg tctatgtgtc tgtttttata acagtaccct 21660
   gctgttcagg ttcctatagc cttttagtat aaaatcggct aatgtgatgc ctccagcttt 21720
   gttctttttg cttaggattg ctttggctat ttgggctcct ttttgggtcc atattaattt 21780
   taaaacagtt ttttctggtt ttgtgaagga tatcattggt agtttatagg aatagcattg 21840
   aatctgtaga ttgctttggg cagtatggcc attttaacaa tattaattct tcctatctat 21900
   gaatatggaa tgtttttcca tgtgtttgtg tcatctcttt atacctgatg tataaagaaa 21960
   agctggtatt attcctactc aatctgttcc aaaaaattga ggaggaggaa ctcttcccta 22020
   atgaggccag catcattctg ataccaaaac ctggcagaga cacaacagaa aaaagaaaac 22080
   ttcaggccaa tatccttgat gaatatagat gcaaaaatcc tcaacaaaat actagcaaac 22140
   caaatccagc agcacatcaa aaagctgatc tactttgatc aagtaggctt tatccctggg 22200
   atgcaaggtt ggttcaacat acacaaatca ataagtgtga ttcatcacat aaacagagct 22260
   aaaaacaaaa accacaagat tatctcaata ggtagagaaa aggttgtcaa taaaatttaa 22320
   catcctccat gttaaaaacc ttcagtaggt caggtgtagt gactcacacc tgtaatccca 22380
   gcactttggg aggccaaggc gggcatatct cttaagccca ggagttcaag acgagcctag 22440
   gcagcatggt gaaaccccat ctctacaaaa aaaaaaaaaa aaaaaaatta gcttggtatg 22500
   gtgacatgca cctatagtcc cagctattca ggaggttgag gtgggaggat tgtttgagcc 22560
   cgggaggcag aggttggcag cgagctgaga tcatgccacc gcactccagc ctgggcaacg 22620
   gagtgagacc ctgtctcaaa aaagaaaaat cacaaacaat cctaaacaaa ctaggcattg 22680
   aaggaacatg cctcaaaaaa ataagaacca tctatgacag acccatagcc aatatcttac 22740
   caaatgggca aaagctggaa gtattctcct tgagaaccgt aacaagacaa ggatgtccac 22800
   tctcaccact ccttttcagc atagttctgg aagtcctagc cagagcaatc aggaaagaga 22860
   aagaaagaaa gacattcaga taggaagaga agaagtcaaa ctatttctgt ttgcaggcag 22920
   tataattctg tacctagaaa atctcatagt ctctgcccag aaactcctaa atctgttaaa 22980
   aatttcagca aagttttggc attctctata ctccaacacc ttccaaagtg agagcaaaat 23040
   caagaacaca gtcccattca caatagccgc aaaacgaata aaatacctag gaatccagct 23100
   aaccagggag gtgaaagatc tctatgagaa ttacaaaaca ctgctgaaag aaatcagaga 23160
   tgacacaaac aaatggaaat gttctttttt aacaccttgc tttatctaat tcacttatga 23220
   tgaagatact cattcagtgg aacaggtata ataagtccac tcgattaaat ataagcctta 23280
   ttctctttcc agagcccaag aaggggcact atcagtgccc agtcaataat gacgaaatgc 23340
   taatattttt cccctttacg gtttctttct tctgtagtgt ggtacactcg tttcttaaga 23400
   taaggaaact tgaactacct tcctgtttgc ttctacacat acccattctc tttttttgcc 23460
   actctggtca ggtataggat gatccctacc actttcagtt aaaaactcct cctcttacta 23520
   aatgttctct taccctctgg cctgagtaga acctagggaa aatggaagag aaaaagatga 23580
   aagggaggtg gggcctggga agggaataag tagtcctgtt tgtttgtgtg tttgctttag 23640
   cacctgctat atcctaggtg ctgtgttagg cacacattat tttaagtggc cattatatta 23700
   ctactactca ctctggtcgt tgccaaggta ggtagtactt tcttggatag ttggttcatg 23760
   ttacttacag atggtgggct tgttgaggca aacccagtgg ataatcatcg gagtgtgttc 23820
   tctaatctca ctcaaatttt tcttcacatt ttttggtttg ttttggtttt tgatggtagt 23880
   ggcttatttt tgttgctggt ttgttttttg tttttttttg agatggcaag aattggtagt 23940
   tttatttatt aattgcctaa gggtctctac tttttttaaa agatgagagt agtaaaatag 24000
   attgatagat acatacatac ccttactggg gactgcttat attctttaga gaaaaaatta 24060
   catattagcc tgacaaacac cagtaaaatg taaatatatc cttgagtaaa taaatgaatg 24120
   tatattttgt gtctccaaat atatatatct atattcttac aaatgtgttt atatgtaata 24180
   tcaatttata agaacttaaa atgttggctc aagtgaggga ttgtggaagg tagcattata 24240
   tggccatttc aacatttgaa ctttttcttt ttcttcattt tcttcttttc ttcaggaata 24300
   tttttcaaga tgtcttacac agagacactc tagtgaaagc cttcctggat caggtaaatg 24360
   ttgaacttga gattgtcaga gtgaatgata tgacatgttt tcttttttaa tatatcctac 24420
   aatgcctgtt ctatatattt atattcccct ggatcatgcc ccagagttct gctcagcaat 24480
   tgcagttaag ttagttacac tacagttctc agaagagtct gtgagggcat gtcaagtgca 24540
   tcattacatt ggttgcctct tgtcctagat ttatgcttcg ggaattcaga cctttgttta 24600
   caatataata aatattattg ctatctttta aagatataat aataagatat aaagttgacc 24660
   acaactactg ttttttgaaa catagaattc ctggtttaca tgtatcaaag tgaaatctga 24720
   cttagctttt acagatataa tatatacata tatatatcct gcaatgcttg tactatatat 24780
   gtagtacaag tatatatata tgtttgtgtg tgtatatata tatagtacga gcatatatac 24840
   atattaccag cattgtagga tatatatatg tttatatatt aaaaaaaagt tataaactta 24900
   aaaccctatt atgttatgta gagtatatgt tatatatgat atgtaaaata tataacatat 24960
   actctatgat agagtgtaat atatttttta tatatatttt aacatttata aaatgataga 25020
   attaagaatt gagtcctaat ctgttttatt aggtgctttt tgtagtgtct ggtctttcta 25080
   aagtgtctaa atgatttttc cttttgactt attaatgggg aagagcctgt atattaacaa 25140
   ttaagagtgc agcattccat acgtcaaaca acaaacattt taattcaagc attaacctat 25200
   aacaagtaag tttttttttt ttttttgaga aagggaggtt gtttatttgc ctgaaatgac 25260
   tcaaaaatat ttttgaaaca tagtgtactt atttaaataa catctttatt gtttcattct 25320
   tttaaaaaat atctacttaa ttacacagtt gaaggaaatc gtagattata tggaacttat 25380
   ttcttaatat attacagttt gttataataa cattctgggg atcaggccag gaaactgtgt 25440
   catagataaa gctttgaaat aatgagatcc ttatgtttac tagaaatttt ggattgagat 25500
   ctatgaggtc tgtgacatat tgcgaagttc aaggaaaatt cgtaggcctg gaatttcatg 25560
   cttctcaagc tgacataaaa tccctcccac tctccacctc atcatatgca cacattctac 25620
   tcctacccac ccactccacc ccctgcaaaa gtacaggtat atgaatgtct caaaaccata 25680
   ggctcatctt ctaggagctt caatgttatt tgaagatttg ggcagaaaaa attaagtaat 25740
   acgaaataac ttatgtatga gttttaaaag tgaagtaaac atggatgtat tctgaagtag 25800
   aatgcaaaat ttgaatgcat ttttaaagat aaattagaaa acttctaaaa actgtcagat 25860
   tgtctgggcc tggtggctta tgcctgtaat cccagcactt tgggagtccg aggtgggtgg 25920
   atcacaaggt caggagatcg agaccatcct gccaacatgg tgaaaccccg tctctactaa 25980
   gtatacaaaa attagctggg cgtggcagcg tgtgcctgta atcccagcta cctgggaggc 26040
   tgaggcagga gaatcgcttg aacccaggag gtgtaggttg cagtgagtca agatcgcgcc 26100
   actgcacttt agcctggtga cagagctaga ctccgtctca aaaaaaaaaa aaaatatcag 26160
   attgttccta cacctagtgc ttctatacca cactcctgtt agggggcatc agtggaaatg 26220
   gttaaggaga tgtttagtgt gtattgtctg ccaagcactg tcaacactgt catagaaact 26280
   tctgtacgag tagaatgtga gcaaattatg tgttgaaatg gttcctctcc ctgcaggtct 26340
   ttcagctgaa acctggctta tctctcagaa gtactttcct tgcacagttt ctacttgtcc 26400
   ttcacagaaa agccttgaca ctaataaaat atatagaaga cgatacgtga gtaaaactcc 26460
   tacacggaag aaaaaccttt gtacattgtt tttttgtttt gtttcctttg tacattttct 26520
   atatcataat ttttgcgctt cttttttttt tttttttttt tttttttcca ttatttttag 26580
   gcagaaggga aaaaagccct ttaaatctct tcggaacctg aagatagacc ttgatttaac 26640
   agcagagggc gatcttaaca taataatggc tctggctgag aaaattaaac caggcctaca 26700
   ctcttttatc tttggaagac ctttctacac tagtgtgcaa gaacgagatg ttctaatgac 26760
   tttttaaatg tgtaacttaa taagcctatt ccatcacaat catgatcgct ggtaaagtag 26820
   ctcagtggtg tggggaaacg ttcccctgga tcatactcca gaattctgct ctcagcaatt 26880
   gcagttaagt aagttacact acagttctca caagagcctg tgaggggatg tcaggtgcat 26940
   cattacattg ggtgtctctt ttcctagatt tatgcttttg ggatacagac ctatgtttac 27000
   aatataataa atattattgc tatcttttaa agatataata ataggatgta aacttgacca 27060
   caactactgt ttttttgaaa tacatgattc atggtttaca tgtgtcaagg tgaaatctga 27120
   gttggctttt acagatagtt gactttctat cttttggcat tctttggtgt gtagaattac 27180
   tgtaatactt ctgcaatcaa ctgaaaacta gagcctttaa atgatttcaa ttccacagaa 27240
   agaaagtgag cttgaacata ggatgagctt tagaaagaaa attgatcaag cagatgttta 27300
   attggaattg attattagat cctactttgt ggatttagtc cctgggattc agtctgtaga 27360
   aatgtctaat agttctctat agtccttgtt cctggtgaac cacagttagg gtgttttgtt 27420
   tattttattg ttcttgctat tgttgatatt ctatgtagtt gagctctgta aaaggaaatt 27480
   gtattttatg ttttagtaat tgttgccaac tttttaaatt aattttcatt atttttgagc 27540
   caaattgaaa tgtgcacctc ctgtgccttt tttctcctta gaaaatctaa ttacttggaa 27600
   caagttcaga tttcactggt cagtcatttt catcttgttt tcttcttgct aagtcttacc 27660
   atgtacctgc tttggcaatc attgcaactc tgagattata aaatgcctta gagaatatac 27720
   taactaataa gatctttttt tcagaaacag aaaatagttc cttgagtact tccttcttgc 27780
   atttctgcct atgtttttga agttgttgct gtttgcctgc aataggctat aaggaatagc 27840
   aggagaaatt ttactgaagt gctgttttcc taggtgctac tttggcagag ctaagttatc 27900
   ttttgttttc ttaatgcgtt tggaccattt tgctggctat aaaataactg attaatataa 27960
   ttctaacaca atgttgacat tgtagttaca caaacacaaa taaatatttt atttaaaatt 28020
   ctggaagtaa tataaaaggg aaaatatatt tataagaaag ggataaaggt aatagagccc 28080
   ttctgccccc cacccaccaa atttacacaa caaaatgaca tgttcgaatg tgaaaggtca 28140
   taatagcttt cccatcatga atcagaaaga tgtggacagc ttgatgtttt agacaaccac 28200
   tgaactagat gactgttgta ctgtagctca gtcatttaaa aaatatataa atactacctt 28260
   gtagtgtccc atactgtgtt ttttacatgg tagattctta tttaagtgct aactggttat 28320
   tttctttggc tggtttattg tactgttata cagaatgtaa gttgtacagt gaaataagtt 28380
   attaaagcat gtgtaaacat tgttatatat cttttctcct aaatggagaa ttttgaataa 28440
   aatatatttg aaattttgcc tctttcagtt gttcattcag aaaaaaatac tatgatattt 28500
   gaagactgat cagcttctgt tcagctgaca gtcatgctgg atctaaactt tttttaaaat 28560
   taattttgtc ttttcaaaga aaaaatattt aaagaagctt tataatataa tcttatgtta 28620
   aaaaaacttt ctgcttaact ctctggattt cattttgatt tttcaaatta tatattaata 28680
   tttcaaatgt aaaatactat ttagataaat tgtttttaaa cattcttatt attataatat 28740
   taatataacc taaactgaag ttattcatcc caggtatcta atacatgtat ccaaagtaaa 28800
   aatccaagga atctgaacac tttcatctgc aaagctagga ataggtttga cattttcact 28860
   ccaagaaaaa gttttttttt gaaaatagaa tagttgggat gagaggtttc tttaaaagaa 28920
   gactaactga tcacattact atgattctca aagaagaaac caaaacttca tataatacta 28980
   taaagtaaat ataaaatagt tccttctata gtatatttct ataatgctac agtttaaaca 29040
   gatcactctt atataatact attttgattt tgatgtagaa ttgcacaaat tgatatttct 29100
   cctatgatct gcagggtata gcttaaagta acaaaaacag tcaaccacct ccatttaaca 29160
   cacagtaaca ctatgggact agttttatta cttccatttt acaaatgagg aaactaaagc 29220
   ttaaagatgt gtaatacacc gcccaaggtc acacagctgg taaaggtgga tttcatccca 29280
   gacagttaca gtcattgcca tgggcacagc tcctaactta gtaactccat gtaactggta 29340
   ctcagtgtag ctgaattgaa aggagagtaa ggaagcaggt tttacaggtc tacttgcact 29400
   attcagagcc cgagtgtgaa tccctgctgt gctgcttgga gaagttactt aacctatgca    29460
   aggttcattt tgtaaatatt ggaaatggag tgataatacg tacttcacca gaggatttaa    29520
   tgagacctta tacgatcctt agttcagtac ctgactagtg cttcataaat gctttttcat    29580
   ccaatctgac aatctccagc ttgtaattgg ggcatttaga acatttaata tgattattgg    29640
   catggtaggt taaagctgtc atcttgctgt tttctatttg ttctttttgt tttctcctta    29700
   cttttggatt tttttattct actatgtctt ttctattgtc ttattaacta tactctttga    29760
   tttattttag tggttgtttt agggttatac ctctttctaa tttaccagtt tataaccagt    29820
   ttatatacta cttgacatat agcttaagaa acttactgtt gttgtctttt tgctgttatg    29880
   gtcttaacgt ttttatttct acaaacatta taaactccac actttattgt tttttaattt    29940
   tacttataca gtcaattatc ttttaaagat atttaaatat aaacattcaa aacaccccaa    30000
   t                                                                    30001
<210> 3
   <211> 1031
   <212> DNA
   <213> Homo sapiens
<400> 3
   attcccggga tacgtaacct acggtgtccc gctaggaaag agaggtgcgt caaacagcga 60
   caagttccgc ccacgtaaaa gatgacgctt ggtgtgtcag ccgtccctgc tgcccggttg 120
   cttctctttt gggggcgggg tctagcaaga gcaggtgtgg gtttaggaga tatctccgga 180
   gcatttggat aatgtgacag ttggaatgca gtgatgtcga ctctttgccc accgccatct 240
   ccagctgttg ccaagacaga gattgcttta agtggcaaat cacctttatt agcagctact 300
   tttgcttact gggacaatat tcttggtcct agagtaaggc acatttgggc tccaaagaca 360
   gaacaggtac ttctcagtga tggagaaata acttttcttg ccaaccacac tctaaatgga 420
   gaaatccttc gaaatgcaga gagtggtgct atagatgtaa agttttttgt cttgtctgaa 480
   aagggagtga ttattgtttc attaatcttt gatggaaact ggaatgggga tcgcagcaca 540
   tatggactat caattatact tccacagaca gaacttagtt tctacctccc acttcataga 600
   gtgtgtgttg atagattaac acatataatc cggaaaggaa gaatatggat gcataaggaa 660
   agacaagaaa aatgtccaga agattatctt agaaggcaca gagagaatgg aagatcaggg 720
   tcagagtatt attccaatgc ttactggaga agtgattcct gtaatggaaa ctgctttcct 780
   ctatgaaatt cccccgggtt cctggaggaa atagatatag gctgatacag ttacccaatg 840
   atggatgaat attgggggac cgcctggtca ttgaaaggct ttcttttctc caggaaagaa 900
   atttttttcc ttttccataa aaagcttggg aatggaagac aacaattccc attctttttt 960
   tgcgttccac ccctatgtga caacagaaat ttttggggaa acaacaacga aaaaatttta 1020
   tcccgcgcgc a 1031
<210> 4
   <211> 3244
   <212> DNA
   <213> Homo sapiens
<220>
   <221> CDS
   <222> (108)..(1553)
<400> 4
   gggcggggct gcggttgcgg tgcctgcgcc cgcggcggcg gaggcgcagg cggtggcgag        60
   tggatatctc cggagcattt ggataatgtg acagttggaa tgcagtg atg tcg act         116
                                                       Met Ser Thr
                                                       1
   ctt tgc cca ccg cca tct cca gct gtt gcc aag aca gag att gct tta         164
   Leu Cys Pro Pro Pro Ser Pro Ala Val Ala Lys Thr Glu Ile Ala Leu
       5                   10                  15
   agt ggc aaa tca cct tta tta gca gct act ttt gct tac tgg gac aat         212
   Ser Gly Lys Ser Pro Leu Leu Ala Ala Thr Phe Ala Tyr Trp Asp Asn
   20                  25                  30                  35
   att ctt ggt cct aga gta agg cac att tgg gct cca aag aca gaa cag         260
   Ile Leu Gly Pro Arg Val Arg His Ile Trp Ala Pro Lys Thr Glu Gln
                   40                  45                  50
   gta ctt ctc agt gat gga gaa ata act ttt ctt gcc aac cac act cta         308
   Val Leu Leu Ser Asp Gly Glu Ile Thr Phe Leu Ala Asn His Thr Leu
               55                  60                  65
   aat gga gaa atc ctt cga aat gca gag agt ggt gct ata gat gta aag         356
   Asn Gly Glu Ile Leu Arg Asn Ala Glu Ser Gly Ala Ile Asp Val Lys
           70                  75                  80
   ttt ttt gtc ttg tct gaa aag gga gtg att att gtt tca tta atc ttt         404
   Phe Phe Val Leu Ser Glu Lys Gly Val Ile Ile Val Ser Leu Ile Phe
       85                  90                  95
   gat gga aac tgg aat ggg gat cgc agc aca tat gga cta tca att ata         452
   Asp Gly Asn Trp Asn Gly Asp Arg Ser Thr Tyr Gly Leu Ser Ile Ile
   100                 105                 110                 115
   ctt cca cag aca gaa ctt agt ttc tac ctc cca ctt cat aga gtg tgt         500
   Leu Pro Gln Thr Glu Leu Ser Phe Tyr Leu Pro Leu His Arg Val Cys
                   120                 125                 130
   gtt gat aga tta aca cat ata atc cgg aaa gga aga ata tgg atg cat         548
   Val Asp Arg Leu Thr His Ile Ile Arg Lys Gly Arg Ile Trp Met His
               135                 140                 145
   aag gaa aga caa gaa aat gtc cag aag att atc tta gaa ggc aca gag         596
   Lys Glu Arg Gln Glu Asn Val Gln Lys Ile Ile Leu Glu Gly Thr Glu
           150                 155                 160
   aga atg gaa gat cag ggt cag agt att att cca atg ctt act gga gaa         644
   Arg Met Glu Asp Gln Gly Gln Ser Ile Ile Pro Met Leu Thr Gly Glu
       165                 170                 175
   gtg att cct gta atg gaa ctg ctt tca tct atg aaa tca cac agt gtt        692
   Val Ile Pro Val Met Glu Leu Leu Ser Ser Met Lys Ser His Ser Val
   180                 185                 190                 195
   cct gaa gaa ata gat ata gct gat aca gta ctc aat gat gat gat att        740
   Pro Glu Glu Ile Asp Ile Ala Asp Thr Val Leu Asn Asp Asp Asp Ile
                   200                 205                 210
   ggt gac agc tgt cat gaa ggc ttt ctt ctc aat gcc atc agc tca cac        788
   Gly Asp Ser Cys His Glu Gly Phe Leu Leu Asn Ala Ile Ser Ser His
               215                 220                 225
   ttg caa acc tgt ggc tgt tcc gtt gta gta ggt agc agt gca gag aaa        836
   Leu Gln Thr Cys Gly Cys Ser Val Val Val Gly Ser Ser Ala Glu Lys
           230                 235                 240
   gta aat aag ata gtc aga aca tta tgc ctt ttt ctg act cca gca gag        884
   Val Asn Lys Ile Val Arg Thr Leu Cys Leu Phe Leu Thr Pro Ala Glu
       245                 250                 255
   aga aaa tgc tcc agg tta tgt gaa gca gaa tca tca ttt aaa tat gag        932
   Arg Lys Cys Ser Arg Leu Cys Glu Ala Glu Ser Ser Phe Lys Tyr Glu
   260                 265                 270                 275
   tca ggg ctc ttt gta caa ggc ctg cta aag gat tca act gga agc ttt        980
   Ser Gly Leu Phe Val Gln Gly Leu Leu Lys Asp Ser Thr Gly Ser Phe
                   280                 285                 290
   gtg ctg cct ttc cgg caa gtc atg tat gct cca tat ccc acc aca cac       1028
   Val Leu Pro Phe Arg Gln Val Met Tyr Ala Pro Tyr Pro Thr Thr His
               295                 300                 305
   ata gat gtg gat gtc aat act gtg aag cag atg cca ccc tgt cat gaa       1076
   Ile Asp Val Asp Val Asn Thr Val Lys Gln Met Pro Pro Cys His Glu
           310                 315                 320
   cat att tat aat cag cgt aga tac atg aga tcc gag ctg aca gcc ttc       1124
   His Ile Tyr Asn Gln Arg Arg Tyr Met Arg Ser Glu Leu Thr Ala Phe
       325                 330                 335
   tgg aga gcc act tca gaa gaa gac atg gct cag gat acg atc atc tac       1172
   Trp Arg Ala Thr Ser Glu Glu Asp Met Ala Gln Asp Thr Ile Ile Tyr
   340                 345                 350                 355
   act gac gaa agc ttt act cct gat ttg aat att ttt caa gat gtc tta       1220
   Thr Asp Glu Ser Phe Thr Pro Asp Leu Asn Ile Phe Gln Asp Val Leu
                   360                 365                 370
   cac aga gac act cta gtg aaa gcc ttc ctg gat cag gtc ttt cag ctg       1268
   His Arg Asp Thr Leu Val Lys Ala Phe Leu Asp Gln Val Phe Gln Leu
               375                 380                 385
   aaa cct ggc tta tct ctc aga agt act ttc ctt gca cag ttt cta ctt       1316
   Lys Pro Gly Leu Ser Leu Arg Ser Thr Phe Leu Ala Gln Phe Leu Leu
           390                 395                 400
   gtc ctt cac aga aaa gcc ttg aca cta ata aaa tat ata gaa gac gat       1364
   Val Leu His Arg Lys Ala Leu Thr Leu Ile Lys Tyr Ile Glu Asp Asp
       405                 410                 415
   acg cag aag gga aaa aag ccc ttt aaa tct ctt cgg aac ctg aag ata       1412
   Thr Gln Lys Gly Lys Lys Pro Phe Lys Ser Leu Arg Asn Leu Lys Ile
   420                 425                 430                 435
   gac ctt gat tta aca gca gag ggc gat ctt aac ata ata atg gct ctg      1460
   Asp Leu Asp Leu Thr Ala Glu Gly Asp Leu Asn Ile Ile Met Ala Leu
                   440                 445                 450
   gct gag aaa att aaa cca ggc cta cac tct ttt atc ttt gga aga cct      1508
   Ala Glu Lys Ile Lys Pro Gly Leu His Ser Phe Ile Phe Gly Arg Pro
               455                 460                 465
   ttc tac act agt gtg caa gaa cga gat gtt cta atg act ttt taa          1553
   Phe Tyr Thr Ser Val Gln Glu Arg Asp Val Leu Met Thr Phe
           470                 475                 480
   atgtgtaact taataagcct attccatcac aatcatgatc gctggtaaag tagctcagtg    1613
   gtgtggggaa acgttcccct ggatcatact ccagaattct gctctcagca attgcagtta    1673
   agtaagttac actacagttc tcacaagagc ctgtgagggg atgtcaggtg catcattaca    1733
   ttgggtgtct cttttcctag atttatgctt ttgggataca gacctatgtt tacaatataa    1793
   taaatattat tgctatcttt taaagatata ataataggat gtaaacttga ccacaactac    1853
   tgtttttttg aaatacatga ttcatggttt acatgtgtca aggtgaaatc tgagttggct    1913
   tttacagata gttgactttc tatcttttgg cattctttgg tgtgtagaat tactgtaata    1973
   cttctgcaat caactgaaaa ctagagcctt taaatgattt caattccaca gaaagaaagt    2033
   gagcttgaac ataggatgag ctttagaaag aaaattgatc aagcagatgt ttaattggaa    2093
   ttgattatta gatcctactt tgtggattta gtccctggga ttcagtctgt agaaatgtct    2153
   aatagttctc tatagtcctt gttcctggtg aaccacagtt agggtgtttt gtttatttta    2213
   ttgttcttgc tattgttgat attctatgta gttgagctct gtaaaaggaa attgtatttt    2273
   atgttttagt aattgttgcc aactttttaa attaattttc attatttttg agccaaattg      2333
   aaatgtgcac ctcctgtgcc ttttttctcc ttagaaaatc taattacttg gaacaagttc    2393
   agatttcact ggtcagtcat tttcatcttg ttttcttctt gctaagtctt accatgtacc    2453
   tgctttggca atcattgcaa ctctgagatt ataaaatgcc ttagagaata tactaactaa    2513
   taagatcttt ttttcagaaa cagaaaatag ttccttgagt acttccttct tgcatttctg    2573
   cctatgtttt tgaagttgtt gctgtttgcc tgcaataggc tataaggaat agcaggagaa    2633
   attttactga agtgctgttt tcctaggtgc tactttggca gagctaagtt atcttttgtt    2693
   ttcttaatgc gtttggacca ttttgctggc tataaaataa ctgattaata taattctaac    2753
   acaatgttga cattgtagtt acacaaacac aaataaatat tttatttaaa attctggaag    2813
   taatataaaa gggaaaatat atttataaga aagggataaa ggtaatagag cccttctgcc    2873
   ccccacccac caaatttaca caacaaaatg acatgttcga atgtgaaagg tcataatagc    2933
   tttcccatca tgaatcagaa agatgtggac agcttgatgt tttagacaac cactgaacta    2993
   gatgactgtt gtactgtagc tcagtcattt aaaaaatata taaatactac cttgtagtgt    3053
   cccatactgt gttttttaca tggtagattc ttatttaagt gctaactggt tattttcttt 3113
   ggctggttta ttgtactgtt atacagaatg taagttgtac agtgaaataa gttattaaag 3173
   catgtgtaaa cattgttata tatcttttct cctaaatgga gaattttgaa taaaatatat 3233
   ttgaaatttt g 3244
<210> 5
   <211> 761
   <212> DNA
   <213> Homo sapiens
<220>
   <221> misc_feature
   <222> (693)..(693)
   <223> n is a, c, g, or t
<220>
   <221> misc_feature
   <222> (722)..(722)
   <223> n is a, c, g, or t
<400> 5
   cacgaggctt tgatatttct tacaacgaat ttcatgtgta gacccactaa acagaagcta 60
   taaaagttgc atggtcaaat aagtctgaga aagtctgcag atgatataat tcacctgaag 120
   agtcacagta tgtagccaaa tgttaaaggt tttgagatgc catacagtaa atttaccaag 180
   cattttctaa atttatttga ccacagaatc cctattttaa gcaacaactg ttacatccca 240
   tggattccag gtgactaaag aatacttatt tcttaggata tgttttattg ataataacaa 300
   ttaaaatttc agatatcttt cataagcaaa tcagtggtct ttttacttca tgttttaatg 360
   ctaaaatatt ttcttttata gatagtcaga acattatgcc tttttctgac tccagcagag 420
   agaaaatgct ccaggttatg tgaagcagaa tcatcattta aatatgagtc agggctcttt 480
   gtacaaggcc tgctaaagga ttcaactgga agctttgtgc tgcctttccg gcaagtcatg 540
   tatgctccat atcccaccac acacatagat gtggatgtca atactgtgaa gcagatgcca 600
   ccctgtcatg aacatattta taatcagcgt agatacatga gatccgagct gacagccttc 660
   tggagagcca cttcagaaga agacatggct cangatacga tcatctacac tgacgaaagc 720
   tntactcctg atttgaatat ttttcaagat gtcttacaca g 761
<210> 6
   <211> 1901
   <212> DNA
   <213> Homo sapiens
<220>
   <221> CDS
   <222> (125)..(793)
<400> 6
   acgtaaccta cggtgtcccg ctaggaaaga gaggtgcgtc aaacagcgac aagttccgcc      60
   cacgtaaaag atgacgcttg atatctccgg agcatttgga taatgtgaca gttggaatgc     120
   agtg atg tcg act ctt tgc cca ccg cca tct cca gct gtt gcc aag aca      169
        Met Ser Thr Leu Cys Pro Pro Pro Ser Pro Ala Val Ala Lys Thr
        1               5                   10                  15
   gag att gct tta agt ggc aaa tca cct tta tta gca gct act ttt gct       217
   Glu Ile Ala Leu Ser Gly Lys Ser Pro Leu Leu Ala Ala Thr Phe Ala
                   20                  25                  30
   tac tgg gac aat att ctt ggt cct aga gta agg cac att tgg gct cca       265
   Tyr Trp Asp Asn Ile Leu Gly Pro Arg Val Arg His Ile Trp Ala Pro
               35                  40                  45
   aag aca gaa cag gta ctt ctc agt gat gga gaa ata act ttt ctt gcc       313
   Lys Thr Glu Gln Val Leu Leu Ser Asp Gly Glu Ile Thr Phe Leu Ala
           50                  55                  60
   aac cac act cta aat gga gaa atc ctt cga aat gca gag agt ggt gct       361
   Asn His Thr Leu Asn Gly Glu Ile Leu Arg Asn Ala Glu Ser Gly Ala
       65                  70                  75
   ata gat gta aag ttt ttt gtc ttg tct gaa aag gga gtg att att gtt       409
   Ile Asp Val Lys Phe Phe Val Leu Ser Glu Lys Gly Val Ile Ile Val
   80                  85                  90                  95
   tca tta atc ttt gat gga aac tgg aat ggg gat cgc agc aca tat gga       457
   Ser Leu Ile Phe Asp Gly Asn Trp Asn Gly Asp Arg Ser Thr Tyr Gly
                   100                 105                 110
   cta tca att ata ctt cca cag aca gaa ctt agt ttc tac ctc cca ctt       505
   Leu Ser Ile Ile Leu Pro Gln Thr Glu Leu Ser Phe Tyr Leu Pro Leu
               115                 120                 125
   cat aga gtg tgt gtt gat aga tta aca cat ata atc cgg aaa gga aga       553
   His Arg Val Cys Val Asp Arg Leu Thr His Ile Ile Arg Lys Gly Arg
           130                 135                 140
   ata tgg atg cat aag gaa aga caa gaa aat gtc cag aag att atc tta       601
   Ile Trp Met His Lys Glu Arg Gln Glu Asn Val Gln Lys Ile Ile Leu
       145                 150                 155
   gaa ggc aca gag aga atg gaa gat cag ggt cag agt att att cca atg       649
   Glu Gly Thr Glu Arg Met Glu Asp Gln Gly Gln Ser Ile Ile Pro Met
   160                 165                 170                 175
   ctt act gga gaa gtg att cct gta atg gaa ctg ctt tca tct atg aaa       697
   Leu Thr Gly Glu Val Ile Pro Val Met Glu Leu Leu Ser Ser Met Lys
                   180                 185                 190
   tca cac agt gtt cct gaa gaa ata gat ata gct gat aca gta ctc aat       745
   Ser His Ser Val Pro Glu Glu Ile Asp Ile Ala Asp Thr Val Leu Asn
               195                 200                 205
   gat gat gat att ggt gac agc tgt cat gaa ggc ttt ctt ctc aag taa       793
   Asp Asp Asp Ile Gly Asp Ser Cys His Glu Gly Phe Leu Leu Lys
           210                 215                 220
   gaatttttct tttcataaaa gctggatgaa gcagatacca tcttatgctc acctatgaca     853
   agatttggaa gaaagaaaat aacagactgt ctacttagat tgttctaggg acattacgta      913
   tttgaactgt tgcttaaatt tgtgttattt ttcactcatt atatttctat atatatttgg      973
   tgttattcca tttgctattt aaagaaaccg agtttccatc ccagacaaga aatcatggcc     1033
   ccttgcttga ttctggtttc ttgttttact tctcattaaa gctaacagaa tcctttcata     1093
   ttaagttgta ctgtagatga acttaagtta tttaggcgta gaacaaaatt attcatattt     1153
   atactgatct ttttccatcc agcagtggag tttagtactt aagagtttgt gcccttaaac     1213
   cagactccct ggattaatgc tgtgtacccg tgggcaaggt gcctgaattc tctatacacc     1273
   tatttcctca tctgtaaaat ggcaataata gtaatagtac ctaatgtgta gggttgttat     1333
   aagcattgag taagataaat aatataaagc acttagaaca gtgcctggaa cataaaaaca     1393
   cttaataata gctcatagct aacatttcct atttacattt cttctagaaa tagccagtat     1453
   ttgttgagtg cctacatgtt agttccttta ctagttgctt tacatgtatt atcttatatt     1513
   ctgttttaaa gtttcttcac agttacagat tttcatgaaa ttttactttt aataaaagag     1573
   aagtaaaagt ataaagtatt cacttttatg ttcacagtct tttcctttag gctcatgatg     1633
   gagtatcaga ggcatgagtg tgtttaacct aagagcctta atggcttgaa tcagaagcac     1693
   tttagtcctg tatctgttca gtgtcagcct ttcatacatc attttaaatc ccatttgact     1753
   ttaagtaagt cacttaatct ctctacatgt caatttcttc agctataaaa tgatggtatt     1813
   tcaataaata aatacattaa ttaaatgata ttatactgac taattgggct gttttaaggc     1873
   aaaaaaaaaa aaaaaaaaaa aaaaaaaa                                        1901
<210> 7
   <211> 562
   <212> DNA
   <213> Homo sapiens
<220>
   <221> misc_feature
   <222> (166)..(166)
   <223> n is a, c, g, or t
<400> 7
   agacgtaacc tacggtgtcc cgctaggaaa gagagatatc tccggagcat ttggataatg      60
   tgacagttgg aatgcagtga tgtcgactct ttgcccaccg ccatctccag ctgttgccaa     120
   gacagagatt gctttaagtg gcaaatcacc tttattagca gctacntttt gcttactggg     180
   acaatattct tggtcctaga gtaaggcaca tttgggctcc aaagacagaa caggtacttc     240
   tcagtgatgg agaaataact tttcttgcca accacactct aaatggagaa atccttcgaa     300
   atgcagagag tggtgctata gatgtaaagt tttttgtctt gtctgaaaag ggagtgatta     360
   ttgtttcatt aatctttgat ggaaactgga atggggatcg cagcacatat ggactatcaa     420
   ttatacttcc acagacagaa cttagtttct acctcccact tcatagagtg tgtgttgata     480
   gattaacaca tataatccgg aaaggaagaa tatggatgca taaggaaaga caagaaaatg     540
   tccagaagat tatcttagaa gg                                              562
<210> 8
   <211> 798
   <212> DNA
   <213> Homo sapiens
<220>
   <221> CDS
   <222> (81)..(590)
<400> 8
   gggctctctt ttgggggcgg ggtctagcaa gagcagatat ctccggagca tttggataat        60
   gtgacagttg gaatgcagtg atg tcg act ctt tgc cca ccg cca tct cca gct       113
                         Met Ser Thr Leu Cys Pro Pro Pro Ser Pro Ala
                         1               5                   10
   gtt gcc aag aca gag att gct tta agt ggc aaa tca cct tta tta gca         161
   Val Ala Lys Thr Glu Ile Ala Leu Ser Gly Lys Ser Pro Leu Leu Ala
               15                  20                  25
   gct act ttt gct tac tgg gac aat att ctt ggt cct aga gta agg cac         209
   Ala Thr Phe Ala Tyr Trp Asp Asn Ile Leu Gly Pro Arg Val Arg His
           30                  35                  40
   att tgg gct cca aag aca gaa cag gta ctt ctc agt gat gga gaa ata         257
   Ile Trp Ala Pro Lys Thr Glu Gln Val Leu Leu Ser Asp Gly Glu Ile
       45                  50                  55
   act ttt ctt gcc aac cac act cta aat gga gaa atc ctt cga aat gca         305
   Thr Phe Leu Ala Asn His Thr Leu Asn Gly Glu Ile Leu Arg Asn Ala
   60                  65                  70                  75
   gag agt ggt gct ata gat gta aag ttt ttt gtc ttg tct gaa aag gga         353
   Glu Ser Gly Ala Ile Asp Val Lys Phe Phe Val Leu Ser Glu Lys Gly
                   80                  85                  90
   gtg att att gtt tca tta atc ttt gat gga aac tgg aat ggg gat cgc         401
   Val Ile Ile Val Ser Leu Ile Phe Asp Gly Asn Trp Asn Gly Asp Arg
               95                  100                 105
   agc aca tat gga cta tca att ata ctt cca cag aca gaa ctt agt ttc         449
   Ser Thr Tyr Gly Leu Ser Ile Ile Leu Pro Gln Thr Glu Leu Ser Phe
           110                 115                 120
   tac ctc cca ctt cat aga gtg tgt gtt gat aga tta aca cat ata atc         497
   Tyr Leu Pro Leu His Arg Val Cys Val Asp Arg Leu Thr His Ile Ile
       125                 130                 135
   cgg aaa gga aga ata tgg atg cat aag gaa aga caa gaa aat gtc cag         545
   Arg Lys Gly Arg Ile Trp Met His Lys Glu Arg Gln Glu Asn Val Gln
   140                 145                 150                 155
   aag att atc tta gaa ggc aca gag aga atg gaa gat cag ggt cag             590
   Lys Ile Ile Leu Glu Gly Thr Glu Arg Met Glu Asp Gln Gly Gln
                   160                 165                 170
   agtattattc caatgcttac tggagaagtg attcctgtaa tgggactgct ttcatctatg       650
   aaatcacaca gtgttcctga agaaatagat atagctgata cagtactcca tgatgatgat       710
   atttggtgac agctgtcatg aaaggctttc ttctcaagta ggaatttttt cttttcataa       770
   aagctgggat gaagccagat tcccatct                                          798
<210> 9
   <211> 169
   <212> DNA
   <213> Homo sapiens
<400> 9
   aaacagcgac aagttccgcc cacgtaaaag atgatgcttg gtgtgtcagc cgtccctgct 60
   gcccggttgc ttctcttttg ggggcggggt ctagcaagag cagatatctc cggagcattt 120
   ggataatgtg acagttggaa tgcggtgatg tcgactcttt gcccaccgc 169
<210> 10
   <211> 176
   <212> DNA
   <213> Homo sapiens
<400> 10
   aaaacgtcat cgcacataga aaacagacag acgtaaccta cggtgtcccg ctaggaaaga 60
   gaggtgcgtc aaacagcgac aagttccgcc cacgtaaaag atgacgcttg atatctccgg 120
   agcatttgga taatgtgaca gttggaatgc agtgatgtcg actctttgcc caccgc 176
<210> 11
   <211> 576
   <212> DNA
   <213> Homo sapiens
<400> 11
   agtcgctaga ggcgaaagcc cgacacccag cttcggtcag agaaatgaga gggaaagtaa 60
   aaatgcgtcg agctctgagg agagcccccg cttctacccg cgcctcttcc cggcagccga 120
   accccaaaca gccacccgcc aggatgccgc ctcctcactc acccactcgc caccgcctgc 180
   gcctccgccg ccgcgggcgc aggcaccgca accgcagccc cgccccgggc ccgcccccgg 240
   gcccgccccg accacgcccc ggccccggcc ccggccccta gcgcgcgact cctgagttcc 300
   agagcttgct acaggctgcg gttgtttccc tccttgtttt cttctggtta atctttatca 360
   ggtcttttct tgttcaccct cagcgagtac tgtgagagca agtagtgggg agagagggtg 420
   ggaaaaacaa aaacacacac ctcctaaacc cacacctgct cttgctagac cccgccccca 480
   aaagagaagc aaccgggcag cagggacggc tgacacacca agcgtcatct tttacgtggg 540
   cggaacttgt cgctgtttga cgcacctctc tttcct 576
<210> 12
   <211> 43
   <212> DNA
   <213> Artificial sequence
<220>
   <223> Primer
<400> 12
   cgactggagc acgaggacac tgaggaaaga gaggtgcgtc aaa 43
<210> 13
   <211> 18
   <212> DNA
   <213> Artificial sequence
<220>
   <223> Primer
<400> 13
   tttttttttt tttttttt 18
<210> 14
   <211> 18
   <212> DNA
   <213> Artificial sequence
<220>
   <223> Primer
<400> 14
   aaagagaagc aaccgggc 18
<210> 15
   <211> 22
   <212> DNA
   <213> Artificial sequence
<220>
   <223> Primer
<400> 15
   cgactggagc acgaggacac tg 22
<210> 16
   <211> 17
   <212> DNA
   <213> Artificial sequence
<220>
   <223> Primer
<400> 16
   cagggacggc tgacaca 17
<210> 17
   <211> 22
   <212> DNA
   <213> Artificial sequence
<220>
   <223> Primer
<400> 17
   gtcaacggat ttggtcgtat tg 22
<210> 18
   <211> 21
   <212> DNA
   <213> Artificial sequence
<220>
   <223> Primer
<400> 18
   tggaagatgg tgatgggatt t 21
<210> 19
   <211> 20
   <212> DNA
   <213> Artificial sequence
<220>
   <223> Synthetic oligonucleotide
<400> 19
   ccttccctga aggttcctcc 20
<210> 20
   <211> 20
   <212> DNA
   <213> Artificial sequence
<220>
   <223> Synthetic oligonucleotide
<400> 20
   gccttactct aggaccaaga 20
<210> 21
<400> 21
   000
<210> 22
   <211> 20
   <212> DNA
   <213> Artificial sequence
<220>
   <223> Synthetic oligonucleotide
<400> 22
   ggaactcagg agtcgcgcgc 20
<210> 23
   <211> 20
   <212> DNA
   <213> Artificial sequence
<220>
   <223> Synthetic oligonucleotide
<400> 23
   tctggaactc aggagtcgcg 20
<210> 24
   <211> 20
   <212> DNA
   <213> Artificial sequence
<220>
   <223> Synthetic oligonucleotide
<400> 24
   gtagcaagct ctggaactca 20
<210> 25
   <211> 20
   <212> DNA
   <213> Artificial sequence
<220>
   <223> Synthetic oligonucleotide
<400> 25
   cctgtagcaa gctctggaac 20
<210> 26
   <211> 20
   <212> DNA
   <213> Artificial sequence
<220>
   <223> Synthetic oligonucleotide
<400> 26
   cagcctgtag caagctctgg 20
<210> 27
<400> 27
   000
<210> 28
   <211> 20
   <212> DNA
   <213> Artificial sequence
<220>
   <223> Synthetic oligonucleotide
<400> 28
   caaccgcagc ctgtagcaag 20
<210> 29
   <211> 20
   <212> DNA
   <213> Artificial sequence
<220>
   <223> Synthetic oligonucleotide
<400> 29
   aaacaaccgc agcctgtagc 20
<210> 30
   <211> 20
   <212> DNA
   <213> Artificial sequence
<220>
   <223> Synthetic oligonucleotide
<400> 30
   gggaaacaac cgcagcctgt 20
<210> 31
   <211> 20
   <212> DNA
   <213> Artificial sequence
<220>
   <223> Synthetic oligonucleotide
<400> 31
   ggagggaaac aaccgcagcc 20
<210> 32
   <211> 20
   <212> DNA
   <213> Artificial sequence
<220>
   <223> Synthetic oligonucleotide
<400> 32
   caaggaggga aacaaccgca 20
<210> 33
<400> 33
   000
<210> 34
   <211> 20
   <212> DNA
   <213> Artificial sequence
<220>
   <223> Synthetic oligonucleotide
<400> 34
   tgataaagat taaccagaag 20
<210> 35
   <211> 20
   <212> DNA
   <213> Artificial sequence
<220>
   <223> Synthetic oligonucleotide
<400> 35
   acctgataaa gattaaccag 20
<210> 36
   <211> 20
   <212> DNA
   <213> Artificial sequence
<220>
   <223> Synthetic oligonucleotide
<400> 36
   aagacctgat aaagattaac 20
<210> 37
   <211> 20
   <212> DNA
   <213> Artificial sequence
<220>
   <223> Synthetic oligonucleotide
<400> 37
   caagaaaaga cctgataaag 20
<210> 38
   <211> 20
   <212> DNA
   <213> Artificial sequence
<220>
   <223> Synthetic oligonucleotide
<400> 38
   gaacaagaaa agacctgata 20
<210> 39
   <211> 20
   <212> DNA
   <213> Artificial sequence
<220>
   <223> Synthetic oligonucleotide
<400> 39
   ggtgaacaag aaaagacctg 20
<210> 40
   <211> 20
   <212> DNA
   <213> Artificial sequence
<220>
   <223> Synthetic oligonucleotide
<400> 40
   gctgagggtg aacaagaaaa 20
<210> 41
   <211> 20
   <212> DNA
   <213> Artificial sequence
<220>
   <223> Synthetic oligonucleotide
<400> 41
   ctcgctgagg gtgaacaaga 20
<210> 42
   <211> 20
   <212> DNA
   <213> Artificial sequence
<220>
   <223> Synthetic oligonucleotide
<400> 42
   gtactcgctg agggtgaaca 20
<210> 43
<400> 43
   000
<210> 44
   <211> 20
   <212> DNA
   <213> Artificial sequence
<220>
   <223> Synthetic oligonucleotide
<400> 44
   ctcacagtac tcgctgaggg 20
<210> 45
<400> 45
   000
<210> 46
   <211> 20
   <212> DNA
   <213> Artificial sequence
<220>
   <223> Synthetic oligonucleotide
<400> 46
   cttgctctca cagtactcgc 20
<210> 47
   <211> 20
   <212> DNA
   <213> Artificial sequence
<220>
   <223> Synthetic oligonucleotide
<400> 47
   ctacttgctc tcacagtact 20
<210> 48
   <211> 20
   <212> DNA
   <213> Artificial sequence
<220>
   <223> Synthetic oligonucleotide
<400> 48
   ccactacttg ctctcacagt 20
<210> 49
   <211> 20
   <212> DNA
   <213> Artificial sequence
<220>
   <223> Synthetic oligonucleotide
<400> 49
   tccccactac ttgctctcac 20
<210> 50
   <211> 20
   <212> DNA
   <213> Artificial sequence
<220>
   <223> Synthetic oligonucleotide
<400> 50
   ctctccccac tacttgctct 20
<210> 51
   <211> 20
   <212> DNA
   <213> Artificial sequence
<220>
   <223> Synthetic oligonucleotide
<400> 51
   cctctctccc cactacttgc 20
<210> 52
   <211> 20
   <212> DNA
   <213> Artificial sequence
<220>
   <223> Synthetic oligonucleotide
<400> 52
   ttttgttttt cccaccctct 20
<210> 53
   <211> 20
   <212> DNA
   <213> Artificial sequence
<220>
   <223> Synthetic oligonucleotide
<400> 53
   ttaggaggtg tgtgtttttg 20
<210> 54
   <211> 20
   <212> DNA
   <213> Artificial sequence
<220>
   <223> Synthetic oligonucleotide
<400> 54
   ggtttaggag gtgtgtgttt 20
<210> 55
   <211> 20
   <212> DNA
   <213> Artificial sequence
<220>
   <223> Synthetic oligonucleotide
<400> 55
   gtgggtttag gaggtgtgtg 20
<210> 56
<400> 56
   000
<210> 57
<400> 57
   000
<210> 58
   <211> 20
   <212> DNA
   <213> Artificial sequence
<220>
   <223> Synthetic oligonucleotide
<400> 58
   agagcaggtg tgggtttagg 20
<210> 59
   <211> 20
   <212> DNA
   <213> Artificial sequence
<220>
   <223> Synthetic oligonucleotide
<400> 59
   gcaagagcag gtgtgggttt 20
<210> 60
<400> 60
   000
<210> 61
   <211> 20
   <212> DNA
   <213> Artificial sequence
<220>
   <223> Synthetic oligonucleotide
<400> 61
   ggtctagcaa gagcaggtgt 20
<210> 62
<400> 62
   000
<210> 63
<400> 63
   000
<210> 64
<400> 64
   000
<210> 65
<400> 65
   000
<210> 66
<400> 66
   000
<210> 67
<400> 67
   000
<210> 68
<400> 68
   000
<210> 69
<400> 69
   000
<210> 70
<400> 70
   000
<210> 71
<400> 71
   000
<210> 72
<400> 72
   000
<210> 73
<400> 73
   000
<210> 74
<400> 74
   000
<210> 75
<400> 75
   000
<210> 76
<400> 76
   000
<210> 77
<400> 77
   000
<210> 78
<400> 78
   000
<210> 79
<400> 79
   000
<210> 80
<400> 80
   000
<210> 81
<400> 81
   000
<210> 82
<400> 82
   000
<210> 83
<400> 83
   000
<210> 84
<400> 84
   000
<210> 85
<400> 85
   000
<210> 86
<400> 86
   000
<210> 87
<400> 87
   000
<210> 88
<400> 88
   000
<210> 89
<400> 89
   000
<210> 90
<400> 90
   000
<210> 91
<400> 91
   000
<210> 92
<400> 92
   000
<210> 93
   <211> 38001
   <212> DNA
   <213> Mus musculus
<400> 93
   tgtctctagg taaaattttg aaggaaaaaa aaaacactaa gaaggtatat tccttcaaag 60
   ttccagtctt attctgaagt gtaatgttat gttagtttga ctcacagaca ggttttaaag 120
   aagggcttac ttcaagagga caccaaacaa ataccttcta ttcctagtgg gctctggaat 180
   cacagaaaac tgacccaatc aattacattg atagctctgg cttactacag acaagcaaat 240
   tatcttaagt gtgcatgcat gcgcgtgtat gtgtgttagt acctaacacc cacctgggaa 300
   cttttcagct tttcagtgtg ggatatagta taaacgtcta ttcctcgtgt tgtggattag 360
   ctgactggcc tcactcagct gccttcctta cctgcaaact cacccacttt gactacagca 420
   tcgcactctt aaccctagcc ttccaaacat ggtcctatgc tatttctgtg tgtctggatg 480
   tatttttaac tctcagatgt atacttcatt tatgagatat acatctgaag accacggtac 540
   aaaacactgt aagaacttga tagaatgaca actgctaggt aaaaaaaaaa aaaaaaaaaa 600
   aaaaaaaaaa aaaaaaaagc atacaatacc tggtgagagt tctattttta ccgaaggtgg 660
   tattgatagg tattctgtta ttaatgcctt tcttttccct ataaatgatg aaaagttgct 720
   ggaaaataat aaacactact catctgtagt gaaaagccac aatacagtta caaaccaatc 780
   aatcaatcaa taaatcagac gtcatggtgt tcttttccca aaggttaaaa aacaaagtgc 840
   actgtgctat ttggcaaaaa tgacgtttag aagaaaacac ggtgactacg cacagagggt 900
   gggggaatca ttgtgcttgt tgcggagtga acacgtacag tgtgcacgca gacttacggc 960
   atttaaccgt gtcataggga ccaaaggaaa tccactcact cactaaatat ttgttgagca 1020
   cccactacct gccaactccc aaacaaaaca aagcaaaact acttacaacc acaaactacg 1080
   cttcgtaacc tagatagata acgcaggtga cactatctat ctaggttgag ctcagctctg 1140
   cccatgcttt tcctgagcgg ctcttggaag aaaagctaca aagcccatga cagcctccgc 1200
   ctggccagct gccactggca tctcaaggct ggcaaagcaa agtgaaagcg ccaacccgga 1260
   acttacggag tcccacgagg gaaccgcggc gcgtcaagca gagacgagtt ccgcccacgt 1320
   gaaagatggc gtttgtagtg acagccatcc caattgccct ttccttctag gtggaaagtg 1380
   gtgtctagac agtccaggga gggtgtgcga gggaggtgcg ttttggttgc ctcagctcgc 1440
   aacttaactc cacaacggtg accaaggaca aaagaaggaa acaagactgc agagatccgc 1500
   accggggagc cctgcagatt ctgggtctgc tgtggactgg gggcgggact gcgactgggc 1560
   gggcctgggg gcgtgtccgg ggcggggcgg tcccggggcg gggcccggag cgggctgcgg 1620
   ttgcggtccc tgcgccggcg gtgaaggcgc agcagcggcg agtgggtgag tgagacgcgc 1680
   gggcggaggg gggctgctgc cacggtcggc tcgcgggccg gccggctccg ggtaccagcg 1740
   gggttttttt ctccttcgag gtgaactcct ccctgtcccc cgggcgaaag agcccttggc 1800
   cttgcaggag ttgcgggggc cgcggcggtg cggaggggat ggggatgggc ctcatctttg 1860
   ctgtccgccc gcgctccccg atcccgaccc ggagcgtctc ccgggccctt gagggaaccc 1920
   tccgggagta cggcgagcgc ggcccccacc gccacaagcc tgggccccag gggcctggcc 1980
   cggcgacagc tggtgggtcc tgcgacccag tcaggtctcc cgagggtccc cgcccgggag 2040
   gagaaagcgc cggtgggatg gagtaaggac ggacagaaca acacgcaggc aggatttcgc 2100
   agaagtttgc aaggagtgcg gatgcccact tacatgggct gctactctta ccaggttgtt 2160
   ccccagttct gtgggacgtg acctggttgc ctcacagctc cgcggttgta cagacttatt 2220
   aaaggaagtg accattgtga cttgggcatc acttgactga tggtaatcag ttgcagagag 2280
   agaagtgcac tgattaagtc tgtccacaca gggtctgtct ggccaggagt gcatttgcct 2340
   gggagggatt ggttgcgctt tctggtgtgg ggactattag gctcttgtag agttttgtcc 2400
   cggcagatgg ataaatttct tgttacactg ttcccgttcg tcaccagttg agaaaaacgg 2460
   gtacacagtc tgtctcagta gtacttttac tttatattaa gggcccaaaa gggactggaa 2520
   aatactttaa gatagaatcg ttagtccact tggaaaactt aaaatatgag agagagaggg 2580
   gggggggaga gagagagaga gagagagaga gaaaggaagg aagaaggagg aagaggagga 2640
   ggaaagagat tgagattatg ttaataatat ggaatcagaa tatttgaaat atagtaagcg 2700
   tcccctcagt taaagaggac attccaggag gcccccagta tagcctgaaa tctcaggaaa 2760
   cgcctacata cacccatcgt gtggatatag gtgttttccc ttcattacat ttcatacaca 2820
   gatgttaaag tttagaaagt aggcacaata agagattaca aataactgat aataaagtcg 2880
   agccattgca gctgctctgt aaaagtcctg tgaatgtgat cgctttgtgt ttcaaagtaa 2940
   cttactgtac ttcacccctg ttaagcaaaa caagattcac ctgaacgcag gcaccttggt 3000
   accttggcag acaccagatc tgataaccaa gaggatggag aagtagtggc agacagtgtg 3060
   gagagcatga atatgctaga caaaagggtg aatcataacc taggagcaga aagcaggtat 3120
   ttcatcatcc tccacagtaa aaacctatgt cacgtaaaaa acctacaagt agtttttctt 3180
   ttactctttt tgaatgaaag cttgctacag gcactgaaag ttaaaataat ctgtggatca 3240
   ggaggaacag gggttttctg tctgagtcac tgctgactag cacctcagtg accattggca 3300
   ctgtgggaaa ccccagagtc agttggaaac ttcgaaacta aaggtgacgg tgttcttatt 3360
   tcatagaaca caaaaaataa gaggggttac agcctgcgct gcagactgga cattcaacaa 3420
   gcatttaaat ttctgggaga caaatgtaaa tataacttta aaagttggta aaatactctg 3480
   tttggctatg ttggccatcc aatgtttgct tttagaaaat gactgaatgg ataaaacgtc 3540
   tatcttttga gcctgcccta gacccccatg ttgagtgaat actgtccaag tgttaggtta 3600
   gccggcctga gaaacttgga tctaggcaag atggcacagt cctggtgtca tgagtatgca 3660
   tgtgagtttt ggctgaaatt gaacatttgt agagaatgac aaaggctggt ctggcaagta 3720
   gtccactgtc tttacagtgg tcttggttag ttcctgtttg gctgagaggg ctggttgatg 3780
   gctgtcctgc ccctcttccc acaagtggaa gccttatggt ataattcttg atcacagtag 3840
   cagtaggcaa atgaacttcc tcaaagcagc ctggaaagct gatttttttt tctttctttc 3900
   tctttttttt ttttttttca caaggttaaa gaaaaaacaa agggcttcaa atgtgccagt 3960
   ctgctaacag tgttaacatg tttattaaca taaataaact ttattagttt ttggaagtat 4020
   tggttaagcc ctcgtgaccc ctgaactcgg tttatagagt gatgagtcgt agcctcactc 4080
   tggtttggac tctggcttct ctcagaagac tctgtggcta atgttaacct tctgaagtag 4140
   ccagaaaaca tataagcaaa agtctgtgag gttgaaatga attttttggc cacatttgta 4200
   tatgggttcc caccaatgct aacttcaggt gttagtaata tcagactcac agcttccctg 4260
   attacacttc gctataagac tttatttttt aggtcatagg aatttcccct ttttcatgat 4320
   tcctaaatca tgaaataaca tagtctaaaa atacggtatt cctgaaataa acaatttcta 4380
   agttttaagc tgcgtgctat tctgaacagt ctgatgccct cttgtagctt ttactgtgtc 4440
   ctaccccggg catggttgat tcctttgtcc aaacatctgt ctgttgtatc cacactggat 4500
   tgcaccacct gcgtgctagt cagtcactca gacattttag ttataaggta gcttatattt 4560
   actccttatt ttatttaata atggcctcat agcaaggcgg taatgatact ggtaatttgg 4620
   gtttgcttaa gaggagccat gaagtagttt taaatgaaaa ggtgaaaatt cccactatag 4680
   tttggagggg gaggctatac tggtactact acgattcacg gtaagactaa atcttctgtg 4740
   aaattatgaa ggagaaaaag ttacactggt ctggtcttgc tgttggatta attttatagt 4800
   tataaccact gtacatgata aataacccta aaacaatgaa tttgtaggtg gatggcataa 4860
   tctgaaaacc atgttctgag cagttgatgg cagcaggctg tgctggaagt gttaggcata 4920
   tttatagatt tcagcccaag ttctgaagag gctggagaga tggctcagtg gttaagagtg 4980
   cttgctattg cagaggacct aggttcctct acaggcacca ggcaagcgtg ggacacactg 5040
   agatacatac agacaaaaca taaaattaaa taaattgtgc ataataatac tagtaatata 5100
   tgagtaaaat aaggataaat acacatcata attaaataaa taaattgtaa agttccctag 5160
   aagtgagggt caccaagcca ttcacaagat ggctgcgctg atgcagggat atatgtgaac 5220
   tagaaaaagg tcaaacttaa cagagaagtt ccaaggcatg ctactgcagg cttggctagc 5280
   atgcttgacc tgcagaaatg ctgacggcca ctgggaggtt ttcacaaatg aggaattaga 5340
   agaacttttt ttactaatct ccagaaaaaa aaaagggaag aagaaactga agcagcctgt 5400
   gatgtggacc agaaacgcag tgacagtaac atgtgtgaca ttgcaaaggc atgaaaggac 5460
   agagctgtgg aatacagacc tcaggtggag ctcagcatag agtcattcgg ggattatgcc 5520
   tgctgcagca acaaaaggat gagctcaaaa gagacaccga cttctgaatg cagtgggtgt 5580
   ttgttttgtt ttgtttcaaa tgaattgggc agaaaacttt ccagctgtgg aagcttctga 5640
   accgtccctt gctgctgaca tctaagcgtc cgctgtgtcc cagctcagtg atctagggtc 5700
   ttccaaacag atggtccggt gctgagcact ttgaatctca atcctgagtt tctaccacgc 5760
   ctttggccat ttaattccca gataaaagac acatacaacc tttatattta taataaacct 5820
   tagtcagcac aagagctgag caaatatctg tcctctatgc tattatatct attacccagc 5880
   caataacccc attctataat ttgctgtgct tcatctgggc tgctcttaac ttcagtcagc 5940
   cagcccacgt ggccattatt ttaagatttt tttaccccat agtgtcttct cactttactt 6000
   tacatttttc tctctctcct catggttctc ctctgacccc aagcctagga accctaaacc 6060
   ccacccatgt ctcttctgcc catctattgg ctgtaggcat ctttattcac caatcaggat 6120
   aacttggagg caaggttaag tagtctcctg ggtctaggtg ctgtctctgg gagcaaccag 6180
   tatttagcat agcaaaagac cagacctcca caatgatcac tctgaccatc ggggcagaag 6240
   gcacctacta gcctgtgcca ctcacctcac tttgttgaat cacatcttat cctgtagtgt 6300
   gtatcactgc ctgttatcac aggaaaaagt gagtcccatc aaataagatg tttcagaaag 6360
   agaccatgtt catataatta tcattctggt aagcttttaa tggttatatt ttgttattaa 6420
   tctctttgtt cctattttgc aaattatacc ttacagtaaa tatatatgca tccaatgggg 6480
   tctttgaatt cctccccggg gagtaggagg actctttgag gatgggctgc atttaaagct 6540
   aaacaacgca acatgacctt tagtccttat agatagccta gagatgagac taaataaaag 6600
   aaatggtata taatgcttta agtttcccaa tcagcttaaa agcttttcct ataaatcttt 6660
   aagattatgc tctggggctc aatactgctt caagaagggc ttttcttttg tatttagaat 6720
   tattcacctt tttaaacaaa aggagaaaat ggaatagaaa tatgtttgca acataatttt 6780
   atgactatgt gtttatttcg cgtgttctgt gggcctgcag tttgctgctg ttaatgagga 6840
   caacagtggc accaatacag tttccactca gattacattc tctgttccct ttctgaaagc 6900
   tgccctctcc actgggccca aaagagtcag tatcttaaac aagctgtaca acttagataa 6960
   ccatggtctc ttcagactag ttaattgaca tatattaaaa agtaaatagt accaaagtga 7020
   atttctgaaa ttaaaaatga acatttaaaa actctaggta aactattcct tagagttaag 7080
   tgttttgcca agttctgtaa tcataatatg atagaaacgc tcactcagca ttctaaatat 7140
   agaagttact ccttcgcatg acactctaat tcttgataag gtggagaaag agagagagag 7200
   agggggagag acagaaaata tggtggttca aggaccattt gagggaatta gttatgttct 7260
   tccgtcctct gtggatctta ggggttgaat acagtcattg agctcggtgg atggctgtcc 7320
   tgttgaaagg tctgcccagc agagcaaata gactttttta tttacatgga catccgtttg 7380
   tgactaatct aatgttcact cccaaagtaa tcacacagac agagaggtag cttccttcag 7440
   tactcttacc ttacatgaat cctaccattt tgttattttt tttccacttt aaatctttga 7500
   ttatgtgttt ttaattagaa aatttgcata caaatttcca tacagtatgt agaattgact 7560
   gtgtttgaat gggtgaagat ccacatgtgt aaccctagct ctggactggc tctgagcttg 7620
   tttgctcttc tcttttgtgt tctgagtaac tgaaactctt tcattttagc agcttagtat 7680
   gcgcccttca cattgctgtg ctgcctgctg cactaacatt actcctttgc ttatgttccc 7740
   cttcctgatt cagtgtcatt ttaagcagta gtactggacc tcagtacctt agccggagct 7800
   cactgaggtg acagggctga ggctctgctg ctgtcttttg agcttacctc tttttaatgt 7860
   tttatggtat ttctgctgcc aggtttgggg gttttgtttt gttttgtttt ttgttttttg 7920
   ttttttttaa ttttctagga acacctagaa aacacaaact aggaaactta aaagagcagc 7980
   gtcttgttcc ctgcgttcta gaaagtccaa gcctaatgcc agtgtcatgg ttgtcaggaa 8040
   catgagcctc tgaaggcttc ttgggaaacc tttcttgtct caacacctct ggtggcaagc 8100
   agtagtccat ggtactctct ctgtccacgg tcagcatccc agtccctgcc ctttatcttt 8160
   gtgcagccga ccagctttgc tttagtctgt ctccttctca ggtctccttc cccgctcctc 8220
   ttaagcacag cagtcattgg attagagccc atccttccct cggatggccc atttgaccta 8280
   attttacgta tttgtaacta aggtcccatt tacttacaca gggccctccc cttcctgttt 8340
   tgttctttag ctgaaatggt ttggagacca aatatccaat cattacaatt gtgcacaagc 8400
   tatgttcatt tggaggtaat aaaggctcat tctttgcttc tattggtatg tgacattttt 8460
   ctaagtcact tggggtttga tagatatctt taaatggctg aacctgatca ctgttctttt 8520
   gtatgtccct gtttagctat tgcaagcgtt cggataatgt gagacctgga atgcagtgag 8580
   acctgggatg cagggatgtc gactatctgc cccccaccat ctcctgctgt tgccaagaca 8640
   gagattgctt taagtggtga atcacccttg ttggcggcta cctttgctta ctgggataat 8700
   attcttggtc ctagagtaag gcatatttgg gctccaaaga cagaccaagt gcttctcagt 8760
   gatggagaaa taacttttct tgccaaccac actctaaatg gagaaattct tcgaaatgca 8820
   gagagtgggg ctatagatgt aaaatttttt gtcttatctg aaaaaggggt aattattgtt 8880
   tcattaatct tcgacggaaa ctggaatgga gatcggagca cttatggact atcaattata 8940
   ctgccgcaga cagagctgag cttctacctc ccacttcaca gagtgtgtgt tgacaggcta 9000
   acacacatta ttcgaaaagg aagaatatgg atgcataagg taaggggctt ttgagcttga 9060
   tcatggtagc ctggccaatg aaagtttttt tctggtacag ttacacttaa gttttggaaa 9120
   ttatatgctg ctaacaccag acagctgtta tgttgtgtct cctgggcaca gaaagccctg 9180
   ctctcatgcc tggggtcttc acagtcctaa tggaaagtaa gatcttataa acattgtgtc 9240
   tgagtttgtt ctggaagctg tgactctacc ttcttgtttt cctttccctg tgtgactttg 9300
   tcctttgctt acaacagtgc aaaagtataa atattctcag attttgataa gctgtcagcc 9360
   acacagcctt agtaactaag ctgctgtccc acgctcccag ttctgtataa cgaggatgga 9420
   ccaattagat tctaaggagt tattcctttc aatttgcaaa tttagctaaa ggaaatattg 9480
   ttttctcctg atatttacat tgcttttcat tttcagcata tctaaagaac aaacctaatt 9540
   ctccttccta ctttctagtt taatataatc ctaaaaatcc attaaaacat gactaattct 9600
   ataaggcctc taacctacaa agggaagtag cattttgaaa agaatagttt tctctattat 9660
   acctattcat gcagacttcc ttccttattt ctgacatact taacaaaaat catttagatt 9720
   caaacagttt agctgcaggt gatattacag acaagtaatc ccagtgctct atctagtctg 9780
   aggcaaaagg atttgagctc agtgccagcc tgttctatct acctggtgag ttccagtccc 9840
   ataaataaac aaactaaaac aaccgttcct ctgttcctca gatgcgagtc gatcttgttt 9900
   gatttaaata gtgtgtaatt attttctttt gaagctgcag gtgttatgtg ggctgtttta 9960
   gactaaattc tctctttact gtggagtaaa gggtgctgtg attgtatttc atgttctctg 10020
   cgagagcttg aacttgttgg gctaatcgct tgtctccatc ctgtctcccc acctgcgtaa 10080
   aaagtatttt cctgtgagct gtacatgata gagcatatct acattgaaaa atgaacgagc 10140
   atcaaaatgg atttgttaaa gtaaattttc tttttcttag gaaagacaag aaaatgtcca 10200
   gaaaattgtc ttggaaggca cagagaggat ggaagatcag gtacagtgca tatcacatgc 10260
   tgcctgtggc aggtcctctt tgcttatgtc ggtataaagt tggtgggtac ttctggtaag 10320
   gacctgagga tacattcatt tgacggaagg agcctgaaaa tgagtattct tgttaagctg 10380
   tatagaatga actgaataaa aatttctgca gcctaagttt gaattttaaa aaaatttaat 10440
   tacatctaca aattagtatt tggccaccct ttttcaatca gcaagaatat gtttgaggtc 10500
   atttatttgt agtaaaattg catgcagttt atttatttta ttgaaaatag gttttttaaa 10560
   ctatattttc tgattatggt tttccctcct ctgaatcctc ctagaacctc cacctaccca 10620
   aatctatatc tgttctttct ctctctcatt aggatacaat caggcatgta aaataatagt 10680
   agtagtagta gtaataataa tgtaaaataa gttaaagtaa aaacaaacca gagtaggaca 10740
   acataaatag aagtagaaaa gagccaaata agaaattcaa gaaacacata tagacacaga 10800
   cacaatattt gcatacacag aaattgcata aaaccgcaag actggaaacc ataatatgta 10860
   tgtaaggtgg agtgggaagc cctgacagca cagtgagtaa agcactttca aaaacaccac 10920
   tgactttgtg ttgtgttgcc tgtctgctgg gcatgaggcc tggccttaga gagtggtgtg 10980
   tatacccagg aagacttaca taaacactta gcttttcatt tgtgacctga tagcaattgg 11040
   aaatagtgtc tgggctaggc attccggctt attgccactt cccctcagca ctgaggcccc 11100
   atctgaatcg gatccgtgca acccttgtgc atatgcagtt ttaaaagtta tcccttctgc 11160
   aactatgctc acaggagttg ccgtcttaag ggagtgagca cacccctgag gcatggctcc 11220
   aggggtgcag agccagccat aggcacagtt ttttttaaaa ggtttatgtt gtagttttga 11280
   aactcaaatt tatgtgtatt tgtggcagat tgtttgaatg ttgaaatttg ccagtaacat 11340
   cttttatctt cttcccttta gcctggcatg ccacccaccc tcatttgtcc ttgtcaaact 11400
   ccagtaatta aacatggcta tgtggccttt tctctcattt tccttagcat ggctaaggag 11460
   aatgggactt aaaaaataat aatcatcatt ttaagtatgt ctgagggttt gaggatatag 11520
   tggtagaata tctgcctagc ttccatagct tgatcctaca tttgatccct ggcaaaacac 11580
   acacacacac acatatacac acacataaaa tgacttttat aaagttagtg tgctgtgctg 11640
   tgatgaacag tgccatagga aatattcttg gaaaagacct gaaactaaat gctctaaaag 11700
   gtctaatctt tacttgcttg ctgatcgtta agcagagtct ccaagtataa agtcactttc 11760
   accaacctct gcactggatt tctggagtaa ttagggagag tcatttcaat ataagaaaat 11820
   ttagtaccaa ataaaatttt cattcagtga aattttgttt ttgaaagtaa gagcccactg 11880
   tggtggtttg aatatgcttg gcccagggag tgtcctgtaa gatttttgtt gttgttgaac 11940
   tccattgaga cttatgttga caataaatgc ctgagagtcc atgtctaaaa tgctgtacct 12000
   gtctgaaccc aacggagata aaacttacca tttctgaaaa ggatgaggtg ttttatttac 12060
   atagctgatg taatgtgctt gcaacagctc tattatgaat cttaatacta cttcagtata 12120
   tcacagcact tcaggaaatt taacatacat tgtttaattc catgtcttaa ttgtatttgt 12180
   aaacagacat ttcagcagtt actctaaaaa gtagaaataa tgagtggttg cttctggtca 12240
   ttaggatgaa atattgaaat gataaaattt tctgggctgg agagatggct cagaggttaa 12300
   gagcactgac tgctcttcca gagatcctga gttcaattcc cagcaaccac atggtagctc 12360
   acaaccatct gtaatgggga tctgatgccc tcttctggtg tgtctgaaga caactacagt 12420
   gaactcatac aaataaaaat aaataaatct ttttttaaaa atctatatct gcataggcat 12480
   ttctagatta ggataaattt tccaaaggaa ataagcacct ccatgataag ggcattggaa 12540
   atgaagcccc cgcccccacc cccggtctgc acgtgtgttg aggatgagat ctagggcctc 12600
   cttatacatg ccaggcagct gttctgtcac caagtggaat ataatcctca acccttaatt 12660
   tgaggttcta actttaaaat agatgtgagg ggtttaaata atcatttcat gaaacttaaa 12720
   tgagcaagtt tattactgag gtgagtataa gtaattgata attttaaata tatttagctg 12780
   agattgatag acacttggca atgtcagcat cttatttagg tgatcataaa ctgatgggag 12840
   aaatggtaaa tgttaggggg tgtcgctcat gtcacacacc gcagttatgc tgcaaacaag 12900
   atgccgggaa atagaaattc aaggtcttgt tttgcgggtg cagactcttc tgtctcactg 12960
   attctatgtg gtaacttcag tatgcatttg gatagattat gtcccatttt gaatgtggaa 13020
   gctggctgtt gagaggagac ttcctggtga attccttttt ctaagcatta ccatctgtct 13080
   tagtcagggt ttctattcct gcacaaacat tatgaccaag aagcacttgg ggaggaaagg 13140
   gtttattcag cttacacttc cacactgctg ttcatcacca aggaagtcag gactggaact 13200
   taagcaggtc aggaagcagg agctgatgca gaggccacgg agggatgttc tttactggct 13260
   tgcttccctg gcttgctcag cctgctgtct tatagaaccc aagactacta gcctagggat 13320
   ggcaccaccc acaatgggcc ctcccccctt gatcactaat tgagaaaatg ccccacagct 13380
   ggatctcatg gaggcatttc ctcaactgaa actcctttct ctgtgataac tccagcctgt 13440
   gtcaagttga cacacaaaac cagccagtac aacatctttt cacatttaat ttttctcact 13500
   ttaaacgtgg cctttaacaa gcgcttataa aaatgcttaa gcttaaatgt tatttaagct 13560
   taatatactt aatatacagc actgtagctt aaatgttgca tgtgagagta tatgataagc 13620
   catgctcacc aaggaaaaga agcttaaaga gcataaaaac cctgacagcg gtttctgagt 13680
   gggaggctcg gggactgtgc tgagcaattc caaccaaggg tgttttactc tctgcctcca 13740
   tttgaaatgt ttttcctgca caacctaccc accctgtgat ttcgttcact cgattatgtt 13800
   tgatctaggg tcagagtatc attcccatgc ttactgggga agtcattcct gtaatggagc 13860
   tgcttgcatc tatgaaatcc cacagtgttc ctgaagacat tgatgtaagt gtcatgtatc 13920
   ttttatgggt tcccttgagt ggtgagtggg tggatgtgtg gtgcatgtgc gtgtgtgtgc 13980
   ttgcatactg ggaattgaac ccaagtcctc aggaagagca gccggtgctc ttaagcactg 14040
   agccatctct tcagaacctc ttccaccagt ttctttgacc atttgttgag aatattccag 14100
   tcacacattt tccgtgagta aatctctcta atgctgattt gtcattaagc tcagtctcct 14160
   aattctgata gctaagaagg gtaaattatt aaaaagtgcc ctttactctt cctggccaat 14220
   tcccctttgt tcttctgaaa agtgcataga cagcatcact ttatagatca ccttgatgct 14280
   cgtgagaggg ctggctcgtg ctggctctag acttcggcac acttattaag agttctccca 14340
   acactgtaaa cagactaatt tttatattgt gcattttaga tagctgatac agtgctcaat 14400
   gatgatgaca ttggtgacag ctgtcacgaa ggctttcttc tcaagtaaga attttacttc 14460
   tttttctgaa tgctaagtaa agcagattaa aaatcttaat gctcacccat gacaagattt 14520
   acagggaaaa gatggtagaa aacctacttc ctccaattat ttagggtcaa catggcacat 14580
   ttgagcttac acgtgttgtt ctcacccata caacagtggc atatctgaca ttactcttcc 14640
   cacagtctaa aaaggcagag tttccgtagt acccagggaa gttctggtct gtgtttgggt 14700
   ctggtttctt ctttcaattc tcactaagta taacccttag gaatctatca agttgagttg 14760
   cattttaaat tcctgtgaat tcttcaggtc tagaaatgga aatcattcat attttagact 14820
   gacatttttc atcttcttgt gtaatttaac atttaagaac ttgagctcta atatcagact 14880
   gtctaggtta caactgggaa aacttggtga agctacccaa agctgaacct ccattttctt 14940
   acctgtgaaa tgtgaacagt gataacagct agtttcttgg gtccttgtag gcaccaaatg 15000
   acaggataat ataaagcacc taggacagtg gagccaatga gccaggagcc agtgtgccct 15060
   attatatctg ctctaagaaa gacagtaagt ggaatagcca atactgactg tcttagtcag 15120
   gctttctatt cctgaacaaa aaacatcatg accaagaagc aagctgggga ggaaagggtt 15180
   tattcagctt acacttccac gttgctgttc ctcaccaaag gaagtcagga ctggaactca 15240
   gatcaggaaa caggagcaga tgcagaggcc atggaggaat gttacttact agcttgcttt 15300
   cttatagacc ccaagactac cagcccagag atggtcccac ccacaaggga ccctgccccc 15360
   ttgatcacta attgagaaaa tgccccacag ctggatctca tggaggcatt tccccaactg 15420
   aaactccttt ctctatgata actccagcct gtttcaagtt gacacaaaac cagccagtac 15480
   gctgaccgag cagctgtgtg ttcctctgca gggctgtgtt ctctgtttgt ccctcatctc 15540
   ctgttgtagt ctcctttaca gttacagact gtcatcagta acgagagaga agtgaatagg 15600
   attttgttaa agtgtttact tctatgtcac attcccttcc tataataagc tcacagtgaa 15660
   ataccaggtg accgtgctta acggcatcta ttacctaact ggggtatctt tttccttaaa 15720
   atggatttaa ttttatgtgt gtttgaatac ctgcatatgt gtatgtacac catatttatg 15780
   tatgcctggt acctgaaaaa gggaaaagag ggctttggct ttcttgaaac tagatggttg 15840
   tgagtctcca tgtgggttct ggattgtctc tgcaagagcg gcaggcacac tttagcagtg 15900
   agccgctcct gtcccgagtt gtcttaagac ctgtgaaagg tccctaaaaa atgcagggtt 15960
   ttacccgaat aaaagatgac atcatgcaga tggctttggt gttcatcaag ctcttgtgtg 16020
   ttgtcctaac cttgctgggc tttgtcgttg tgaagctgta actccgtcaa tgttttcctt 16080
   acctacagtg ccatcagctc acacctgcag acctgtggct gttccgttgt agttggcagc 16140
   agtgcagaga aagtaaataa ggtaattcgt tctacagttg aacatgatct gacttttatc 16200
   atcactagca tatcatacat tatcatctaa acagtaggct gcaattgaaa taaccccata 16260
   gtataaggaa gcaatgtaat tttaccaaat ttctctgaca ccctctagca gaactgactc 16320
   taatagaatg agtaagaatt caattaccaa attaattttg atactctttt ttatttttgt 16380
   tattactttt ttattttatt ttaattaggt attttcttca tttacatttc caatgctatc 16440
   ccaaaagttt cccataccct cccacccact cccactcccc tatccaccca ctcccctttg 16500
   gccttggcgt tcacctgtac tgagacatat aaaatttgca agaccaatgg gcctctcttt 16560
   ccaatgatgg ccaactagac catcttctga tacatatgca gctagagaca cgagctccag 16620
   ggggtactgg ttagttcata ttgttgttcc acctaaaggg ttgcagaccc ctttagctcc 16680
   ttaggtactt tctctagctc ctccattggg ggccctgtga tccatccaat agctgactgt 16740
   gagcatccac ttctctgttt gctaggcccc agcatagcct cacaagagac agctatatca 16800
   gggtcctttt agcaaaatct tgctagtgtg tgcaatggtg tcagcgtttg gaagctgatt 16860
   atgagatgga tccccaggat ggcagtatct agatcgtcca tcctttcgtc tcagttccaa 16920
   actttgtctc tgtaactcct tccatgggtg ttttgttccc aattctaaga agggacaaag 16980
   tgtccacact ttggttttca ttcttcttga atttcatgtg ttttgcaaat tgtatcttat 17040
   atcttgggta tcctaagttt ctgggctaat atccacttat cagtgagtac atattgtgtg 17100
   agttcctttg tgattgggtt acctcactca ggatgatgcc ctccaagtcc atccatttgc 17160
   ctaggaattt cataaattca ttctttttaa tagctgagta gtactccatt gtataaatgt 17220
   accacatttt ctgtatccat tcctctgttg aaggacatct gggttctttc cagcttctgg 17280
   ctattataaa taaggctgct atgaacatag tggagcatgt gaccttctta ccggttggaa 17340
   catcttctgg atatatgccc aggagaggta ttgtgggatc ctccggtagt actatgtcca 17400
   attttctgag gaacggccag actgatttcc agagtggttg tacaagcttg caattccacg 17460
   aacaatggag gagtattcct atttctccac atcctcgcca gcatctgctg tcacctgaat 17520
   ttttcatcgt agccattctg actggtgtga ggtggaatct cagggttgtt ttgatttgca 17580
   tttacctgat gattaaggat gctgagtttt tttttcaggt gcttctctgc cattcggtat 17640
   tcctcaggtg agaattcttg gtttagctct gagccccatt tttaatgggg ttatttgatt 17700
   ttctggagtc caccttcttg agttctttat atatattgga tattagtccc ctatctgatt 17760
   taggataggt aaagatcctt tccaaatctg ttggtgacct ttttgtctta ttgatggtgt 17820
   cttttgcctt acagaagctt tgcaatttta tgaggtacca tttgtcgatt ctcgctctta 17880
   cagcacaagc cattgatgtt ctattcagga atttttcccc tgagccaata tcttcgaggc 17940
   tgttccccac tctctcctct ataagcttca ctgtctctgg ttttatgtgg agttccttga 18000
   tccacatgga tttgacatta gtacaaggaa ataggaatgg attaatttgc attcttctac 18060
   atgatatccg ccagttgtgc tagcaccatt tgttgaaaat gctttttccc actggatggt 18120
   tttagctccc ttgtcaaaga tcaagtgacc ataggtgtgt gggttcattt ctgggtcttc 18180
   aattctattc cattggtcta cttgtctgta tataccacta ccatgcagtt tttatcacaa 18240
   ttgccctgta gtacagcttt aggtcaggca tggtgattcc accagaggat cttttatcct 18300
   tgagaagagt ttttgctatc ctaggttttt tgttattcca gatgaatttg catattgccc 18360
   tttctaattc gttgaagaat tgagttggaa ttttgatggg gattgcattg aatctgtaga 18420
   ttgcttttgg caagatagcc atttttacaa tgttgatcct gccaatccat gagcatggga 18480
   gatctttcca tcttctgaga tcttctttaa tttctttctt cagagacttt aagttcttgt 18540
   catacagatc tttcacttcc ttagagtcac gccaaggtat tttatattat ttgtgactat 18600
   tgagaagggt gttgttttcc taatttcttt ctcagcctgt ttatcctttg tatagagaaa 18660
   ggccattact tgtttgagtt aattttatat ccagctactt cattgaagct gtttatcaga 18720
   tttaggagtt ctctggtgga attcttaggg tcacttatat atactaccat atcatctgca 18780
   aaaagtgata ttttgacttc ttcctttcca atttgtatcc ccttgatctc ctcttgttat 18840
   cgaattgctc tggctaagac ttcaagtaca gtgttgaata gggaggaaga aagtggacag 18900
   ccttgtctag tccctgattt tagtggggtt gcttccagct tctcaccatt tactttgatg 18960
   ttggctactg gtttgctgta gattgctttt atcatgttta ggtatgggcc ttgaattcct 19020
   gatctttcca agacttttat catgaatggg tgttggattt tgacaaatgc tttctcctca 19080
   tctaacgaga tgatcatgtg gtttttgtct ttgagtttat ataatggatt acattgatgg 19140
   atttccgtat attgaaccat ctctgcatcc ctggaataaa acctacttgg tcaggatgga 19200
   tgattgtttt gatgagttct tggattcagt tagtgagaat tttactgagt atttttgcat 19260
   caatattcat aagggaaatt ggtctgaagt tctctatctt tgttggttct ttctgtggtt 19320
   taggtatcag agtaattgtg gcttcataga atgagttggg tagagtacct tctgcttctg 19380
   ttttgtggaa tagtttgtga agaactggaa ttagatcttc tttgaaggtc tgatagaact 19440
   ctgcactaaa cccatctggt cctgggattt tttttggttg ggagactatt aatgactgct 19500
   tctatttctt taggggatat aggactgttt agatcattaa cctgatcttg atttaacttt 19560
   ggtacctggt atctgtctag aaacttgtcc atttcatcca ggttctccag ttttgttgag 19620
   tatagccttt tgtagaagga tctgatggtg ttttggattt cttcaggatc tgttgttatg 19680
   tctccctttt catttctgat tttgttaatt agaatacttt ccctgtggcc tctagtgagt 19740
   ctggctaagg gtttatctat cttgttgatt ttctctaaga accagctcct tgattggttg 19800
   attctttgaa tagttcttct tgtttccact tggttgattt cacccctgag tttgattgtt 19860
   tcctgccgtc tactcctctt gggtgaattt gcttcttttt gttctagagc ttttaggtgt 19920
   gttgtcaagc tgctaatgtg tgctctctct agtttccttt tggaggcact cagagctatg 19980
   agttttcccc ttagaaatgc tatcattgtg tcccataagt ttgggtatgt agtggcttca 20040
   ttttcattaa actccaaaaa gtccttaatt tctttcttca ttccttcctt gaccaaggta 20100
   tcattgagaa gactgttgtt cagtttccac gtgaatgttg gctttctatt atttattttg 20160
   ttattgaaga tcagccttag tccatggtga tctgatagga tgcatgggac aatttcaata 20220
   tttttgtata tgttgaggct tgtttttctg accaattatg tggtcaattt tggagaaggt 20280
   accatgaggt gctgagaaga aggtatatcc ttttgtttta ggataaaatg ttctgtagat 20340
   atctgtcaga tccatttgtt tcattacttc tgttagtttc actgtgtccc tgtttagttt 20400
   ctgtttccac gatctgtcca ttggtgaaag tggccatctt tatagtcact gaagacatac 20460
   aaatacatat tcatatcaac tggaacaaac ctaatttctt tttaaatgtt ttacatggaa 20520
   ataagttagg ggttgttatt tgcattacaa agttactcat ccctttcctt cttttctttt 20580
   tttttttttt tttttttttg agaacaagcc tgtgtactta tatgaacttt aatttgccaa 20640
   attcataatt cttattcaat catttatgac agaatgctaa aactctcatt atattttagc 20700
   taggcattta gagctgttat gtgtaacccc aaaaagtagc tttccacttg agatgctgaa 20760
   ggccttgggt tccgtgggct gtcatcatgg ttggctgtat gaaaagagaa aggctccatt 20820
   gtttgggcat cacttaaata ttttttcacc tttcatcttc ttttaggtta agtagcttgt 20880
   ccttgatcat ttcatttttg agagacaact tgccactact ctagttgaaa agtgctgtct 20940
   tgacgctgtc tctggctgtg gtcagagtcc agcagagctg cacagctggt tacctttctc 21000
   tgtacagctc taggccaact cttcttactg gcgaccattt ctaaatccac cattcacttg 21060
   ttccccatga aagtgagtag ggttttttct gtggaagatt ttgggcagtc ctgttgccac 21120
   tttgcatcag acaatagttc cctcattgaa acacgcagtt tattctccag agcggtctgc 21180
   ccactccaaa ggcagtaggt gctgggtaga gatatgccaa gtatcacact aggctatgac 21240
   tgctcactca gatcactcgg atgaagcttt catggccaaa tacagttgag aaagaacaaa 21300
   tattcttcac ttagagagca acaagagtta ttcaagtgta acaagttctg agattccatg 21360
   cagttgattt accagctact tcctaaactt aactggccac aaaatccctt tgtaagcagt 21420
   atgttgtttt gacccatgcc ctgtcaaagg atactcctta cttgggaact gttttaatga 21480
   tggcaacaaa aatttctatt taaatttatt tcataagcaa gcaaagatct ttttacttca 21540
   cattccaatg ttgactcttt tcctctagat agtaagaacg ctgtgccttt ttctgacacc 21600
   agcagagagg aaatgctcca ggctgtgtga agcagaatcg tcctttaagt acgaatcggg 21660
   actctttgtg caaggcttgc taaaggtaca cttgccgatc atttatcatg tgtgacgcaa 21720
   caagtagaga tggagggtac aaataatcac tgagaggctt tggaaagtat attgttagca 21780
   tttaatgtct catagtttta gttgtctggg tactggtttg ttttcatcat tctgagcatg 21840
   aagtgtatgt cttagggatt tatagttcgt atcatgtatg aaacaccatg gggtaatatt 21900
   tatatttcac ttggttccct ctagctatgt gtctggcccc agtgctttcc ttgtaaatgc 21960
   atgcttgaat cagactgagc tgatatgata atgttgatgc tccttttgct tactgagtgg 22020
   ctatgaatat gcaccatact tactcattgt aagaaattaa aatgtctctt aaggatgtaa 22080
   acatagcaaa atgaagcaaa acaaaagcga tgctgtttta ggtaccctaa ctgaccttgt 22140
   gtattcaagg agcattccta cttctgtgat gcaaaagctg tctacactgg gcagatctac 22200
   aaccagcatt aaaccaaata gggaatcact gaaatcacgt tatcaaagat gagaaacaag 22260
   ataataatgt ctactttcac ggcttttatt caggtctagt gctataagtt tttgccaaaa 22320
   caaaaatgaa aacatagact ctgggctgag gctttccctt agcagaaaag tgcttacttg 22380
   ttgtgtccgg ccagcagatc acagcctggg ttctagcctg gaaaggcatt ttggaaacct 22440
   ggaagagaag aggggctagg taacgagaga aagaacggag ccaagtcaaa agcaactctg 22500
   atcaaagctc aattttacta tatcagcacg cagttataaa ggaggggaag ggggggccaa 22560
   tagcaaggcg gcaggttcca gcagtgggcg tggcagaccg attgagccgg caagctcctt 22620
   ccaggtgtaa acagtggagc cctaaggctg ggggagggga ggctacactt agcatgcctg 22680
   atgccctaga tgccacctaa atgacaaatc cagtccagta caggatgtag agcacccccc 22740
   cccaaaaaat tatttttttt gtataccaga aatgaaattg ctgagaaaaa aaaatgaaga 22800
   ccataattat actcccagta gctacaaact aaacagcccc atagatgaag tgagtgatgt 22860
   ctgctgtgac aattatgaaa tgaaagaagt aaagatgaac aaatgaaggg aagacatcca 22920
   gtactcagga ctgaaagact gctgctaaaa tgcctatcca acccagagct ctctgcagac 22980
   tctggacaga tccgctctag atgtgaagat ggtctttttt tttttttttt ttttttggtt 23040
   tttcgagaca gggtttctct gtgtagccct ggctgtccag gaactcactc tgtagaccag 23100
   gctggcctcg aactcagaaa tccgcctgcc tctgcctccc gagtgctggg attaaaggcg 23160
   tgcaccacca tacctggctt tttgtgaaga tgttcttaac agaactagaa agaagtaccc 23220
   cttggtttgc tgcccttctg atgcagtatc cccaaaggct cgcatgcact gaacatttca 23280
   tcttacctgg tgccactgtt gggaagtgat ggaaatgcga ggaattgtag cctcgttgag 23340
   atgtttctca ttaaggcact gggggcatac ctatggagca tacagtagga acctggtttg 23400
   caacctctcc cctctccatc caggctctcc cctgtgcacc tggccttggt gttctgccac 23460
   tccatgaacc caaagtaaag tggactatgc ccttagactg taacagtgag tcagaagaaa 23520
   catttcctct ttaaagctga gttttctggg tgctttgtca tgttaatgga gtctgattag 23580
   tacagaccct gagtaggcag ggcaatctta tgcagaaaca tcaaagctgg tagcatagac 23640
   atacctaatt tcacaataga cactgatgga ctcagtctgg agtacttaca gtaagaatat 23700
   acagcagaga tacggagctc tcttacagtg gtgctctggg agaactggcc gtcctgtgaa 23760
   gaaaagccag agtggctcat tctcaccaga cacaaactga gctcataaga cgcttgaacc 23820
   tgagatcctg gtcagcagcc actagaagaa aacttaggag aaaccattca acacgtcagt 23880
   ctggggaaaa gggtggtttt ggttttggtt ttggtttttt agtatattcc ccaaatcaaa 23940
   aacaacaaaa cccaaacttg acagatgaca tcacactgca aagcttttgc acaaccaaga 24000
   aagcaacctg cagagtgcag taataaccca cagaaggaga ggagatactt gtgggcagtt 24060
   catcacacag gtcaatataa gcaagtactg atagtgtggc catctccaaa gaagatatga 24120
   aaataactgg tatatatgaa gtagtactta gcattgctgc gtatatggta aattcaaaac 24180
   catgatgaga tattgcccca cttagatgga tattatcaaa acaacatcaa aaagtgacaa 24240
   atgctttcaa ggatatgggg aaagtgtact tgcaggaatt taaattatta atttgccatt 24300
   caagaggata ggatggcagt ttaaattaaa aaactagaag tggtagagca gtcgcctaga 24360
   acatacaagg ttcagcacta taataaatga gcaattagac atttgaagca acaatctcac 24420
   cactaggcaa gtcctaaaag aaatggactc gcttcttctt cttcgggaaa acaccaaatg 24480
   gcagatgacg ccggtgcagc gggagggccc agaggacctg ggggctcagg attaggaggc 24540
   cgcggcggct tccacggagg attcggcagc ggtcttaggg gccgtggtcg tggccgaggc 24600
   cgtggccgtg gtcgaggccg cggggctcgt ggaggtaaag ctgaagacaa ggagtggatc 24660
   cccgtcacca agctgggccg cctggttaag gacatgaaga tcaagtcctt ggaggagatc 24720
   tacctgttct ccctgcgcat taaggagtct gagatcattg atttcttcct gggtgcgtcc 24780
   ctaaaggatg aggttctgaa aatcatgcca gtgcagaagc agactcgggc tggccagcgg 24840
   accaggttca aggctttcgt cgctattggg gactacaatg gtcacgttgg tcttggtgtt 24900
   aagtgctcca aggaggttgc tactgccatc cgaggggcca tcatcttggc caagctttcc 24960
   atcgtccctg tgcggagagg ctactggggg aacaagattg gcaagcccca cactgttcca 25020
   tgcaaggtga caggccgctg tggctctgtg ctggtgcgtc tcatccctgc ccccagaggc 25080
   actggcattg tctctgctcc tgaagctcct gatgatggcc ggtatagatg actgctacac 25140
   ttcagccaga ggctgcactg ccaccctggg caactttgct aaggccacct ttgatgccat 25200
   ctccaagact tacagctacc tgacccccga cctctggaaa gagactgtct tcaccaagtc 25260
   tccttatcag gaattcacgg atcatcttgt gaaaacccac accagagtct ctgttcagag 25320
   gacccaggct ccagctgtgg ctaccacata agggttttta tatgagaaaa ataaaagaat 25380
   taagtctgct gaaaaaaaaa aaaaaagaaa gaaagaaaga aaagaaatgg actcggtatg 25440
   tggatgaagc ccaggcacct tcatctgtgt tgcagcacga gtcaccatgc aggatcagtc 25500
   taaacgccca tgcacaaatg aatggtacat agccacagtg aagtgtttga ccacaaaaag 25560
   gaaagtcagt tgtgataagt gaaacaagcc aggcacagaa agataaatgc tgcatgttat 25620
   cattatgtgt aaaggctaaa acgtttatct catacaagta gaaggtaaat acggagacta 25680
   ccagaactta taaagagttc taggaaaaag ctatagagag gctcagggtt gaataactaa 25740
   aattatacct aaaataacta aaaggatagc ttacaatatt ctgtagcact gtagaataat 25800
   tgtgacagtt tgttgtattt ttctggtttg tgtatgtggg agagaaagta tgtggacaga 25860
   ggttgatatc aagtgtctga ctctgcactg cattatttta ggcagggtct ctctctaacc 25920
   attgaatgga ctggctaggc agtggtgccc taacatctac ctgtccgtac atctcccaat 25980
   actaggttat aagtacactg ggttttaagt acaggctata ggtatagata taggctacag 26040
   gtatagatat aggctgctgc aactgattac atgggtgctg ggaacctaac ataggttggg 26100
   tcctcatgtt tacacagaaa tcagtactgt gcctactgag tcatttcccc agttctagta 26160
   tttgtttttt aaatagctag taattggaat tgtgaatgtt cctaacaaaa gaaaatgata 26220
   actatctgag atgctagtta tgataccctg agtgaatcac actttgtgtg catgtactga 26280
   aattcattgt accctgaaaa tacaaaaatt gctctgtgtt gattggctag atgcatgtgt 26340
   attagtcagc aatctctaga gtaataaaac ttagatatat gggatgtatt agacttttgg 26400
   ccttacaggc caagatccag ctaatccatc agtggcaggc tgtgaacagt aagtctaaga 26460
   atccaatagt tgttcagtcc acaaggccgg gtggctcagc tgccttctgt atacagtgga 26520
   atcccaaaga aataggcgcc aaagctagtg aggaatggtc ttgctagcaa agcgaaggtg 26580
   aaggtaatca ggcagaagac aagaccttcc tttttccgtg tccttatata ggctcctagc 26640
   agaacaagtg gcccagacta gatgtggatt aaatgttttg ggtttggttt ggtttgattt 26700
   ggtttggttt ggtttggttt ggtttggttt ggtttggttt ggtttggctt ttcgagacag 26760
   ggtttctctg tatagccctg gctgtcctgg gttgtagacc aggctggcct caaactcaga 26820
   aatctcttgc ctctgcttcc caagtgctgg gattaaaggc gtgcacacca ctacgcccgg 26880
   ctcaatagca ttaaatggca tgtcttttcc tatctcaaat gatctggatt aaaagagtgt 26940
   cttcctacct caaaggtctg gattagaagt ggatctttct acttcagatt aagttaaact 27000
   ctctcacagg tgtgccctct acttttggat ttttggttct agatggagtc aacatgacaa 27060
   ccaaaagtaa ctattacaag tccacccaat atcaacttga tacacaatca tatctcctta 27120
   tgtcataatt aatttccaaa tgaaaacaat aaccatgtca taaaaacacc taaacatgaa 27180
   taactattcc acatacaatc agaaatgcat tcattatata tttaaccaag tcctaattat 27240
   gcctaacgtg atataactat tcttcataca acagcaaaca tgataaattt acaataggtg 27300
   gcaatgtctt attcttttaa tatctcaaac ttaaatatga taaccattga tgttatctta 27360
   attgatgtta tatcatatga taaagaaatt gatgaaagaa agcacaaatg tctgtataaa 27420
   tgctttctta agaaaatagg acagaaactc tgtcaattat aatcatcttt tctgcaacta 27480
   gtcatgtggc cttagtattt ataactacct tcctctgcta aaccattttg tattttctcc 27540
   acccttggca agaacctcag caggtcttgg ctcttttcct ggaggagtga cccatacctt 27600
   cattccttac atgtatgtgc cctttgtcat cctgcctgga ccaggttgtt gtaacattga 27660
   ctttaatcac aggacatcgt agcaccaaca catgccccaa aggatctcct gccctataga 27720
   cataaccttt cttacctcca tagtggggag gcagtcccag tcctccttgg tagtctgcat 27780
   cagtcacgcc tcctaacact gttattcctt tcttagccgg ttgacttaag ggcatcagaa 27840
   ggccaaagtt gccagaggaa aatctgagct tccagttcaa tgaatgtaat gttgttctag 27900
   gcaagcagaa ctgaaggtct caggaatagg aagcaaacac ttcccatgga tcactacagg 27960
   gtgagagtga gtagaattat tctcttttct accacttgac tcctggacct atggatcctg 28020
   gtatcaaaga aaatgtctca tatattgtac actgattcag agcatgcctt ctggaaaacc 28080
   ctgccccagc ccttcatact gctgccatca aattgtcacc tgtgtcttcc tggtaccaac 28140
   ttttgtcctg gttagggtta ctattgctgt gaggaaacac catgagcacc aaagcaactt 28200
   ggggagaaat gggtttattc agcttatgcg tctacatcac agctcatcat caaaggaagt 28260
   cagaacagga gctcaagcag ggcaggaatc tggaggccgt ggaggaaagc tgctgactgg 28320
   ctcgctccct aggcttgctc agactgctta tagaactcag gaccaccagc tccagggtgg 28380
   ccccaccccg caatggattg ggccctccct caggaatcac aattgcccca cagacttacc 28440
   tacagcctag gcattttgga ggctttgagt ctgcctcctc tctgatgatt ctagcttttg 28500
   tcaagttgaa gcaaaagtag acaggcctta aactcacaac aacccacctg cctcaatttt 28560
   ctgagtgcta atattatatc aatttaaaat ttaaatataa catataaagg gcaatagaaa 28620
   ggactagatt catgtaatgg atacaagtta tggaagatgt gtgtgtgtgt gtctgtctgt 28680
   ctgtgtgtgt gtttctagtt taattctgtc atgatttttt tcttgtaggt ggtaggtgag 28740
   tgcatggaat acatttgata ctgaaagggt aaattgaatg tggagcctca cagcttctgt 28800
   tccacatgcc tatgataacc gtagaaattc atggattagt atagacgttg agtctggtta 28860
   attttggtgt gtgatattta tatatatatg tatatatata tgtgtgtgta tgtatgtatg 28920
   tatgtatata tatatatgta tatgtgtata tatatatata tatatatata tatgcaagat 28980
   ttcttataat taagtttaca aaattaaaaa ctatcttaaa aattgaattc ttgcaaataa 29040
   aaatttagct tttggtgatt ggattcttaa tatggttgat gtttacctag aaagttaaaa 29100
   gccctgagtt cagtctccac tttcaccccc aaaatgaaaa tcagcttttg ggtttcagat 29160
   catgagctca gaattaaaga aaacacattt ctaactttgc ttttacaaat cttaatttta 29220
   ccaatttcct ttaaagtcac aatgagatac acagtacttc ctagcacccc ttgttcaatt 29280
   agataatgtg atttctgaaa gagctccctc tacacagggc acagggcagg tgcaaaactg 29340
   tgattgggtg aaatacctgc gagctctcca agcaaagcca ggcctatttg ctttagctgc 29400
   cacatcgggt tcttagaccc gacatccctt cccacctgta tcctccctaa ttccttccaa 29460
   ccccacaaca ctaggtagga gagaaagaag gttagtggtg gaagtttgca cacatctttt 29520
   tagactattt cctactgatt aggggtgtta ggtccttgag acaagtccag tcttcattgt 29580
   caggatatct ccaacttctt cttctcatct ctttgctcac aaagtttatc acaagttgat 29640
   aaactacaac aacaggaacc agcagtagca aggacatcag agttgtatag ctttccagaa 29700
   aatactttga tatacagtaa ttatcctagc ctttaagagt gaaagatttg gcagcctctg 29760
   tgttctacac tcagcataat accttgtata ctgcaggtat ttgctgcatg gtaagtggct 29820
   gcccagctac ctagaaagag gtaaatactt ttctattaac atacatattc atttagatat 29880
   aggaagaaga taaaacaatg gagaaaggca gtcataattt tacagaccag caagtaaacg    29940
   cattaacttg gcataggtct ttgtagtctt tttctgcagt gcgtatttcc tgcagtgccc    30000
   acaccctaca gttggattgc acgtggcatg ttctgaccca ctttttatgg tatactgtgt    30060
   actgtcactg tcaacacaaa tggtagtggc tggattttta tacagtatca gcttgaaggt    30120
   tatttctgaa caagccctgt accagattca caggaatatg catctcttat cattactata    30180
   ttcttttaac aattgcttct ctcagttggc atgtggtcag tgagttctct cttccttctg    30240
   acaggatgca acaggcagtt ttgtcctacc cttccggcaa gttatgtatg ccccgtaccc    30300
   caccacgcac attgatgtgg atgtcaacac tgtcaagcag atgccaccgt gtcatgaaca    30360
   tatttataat caacgcagat acatgaggtc agagctgaca gccttctgga gggcaacttc    30420
   agaagaggac atggcgcagg acaccatcat ctacacagat gagagcttca ctcctgattt    30480
   gtatgtgacg cttggcctta ggtgtcattg ttaaacaaca taaaacttct catttatgag    30540
   taaaaacagt gcaagttgta tttaaaagaa aagaaatatg acaagcacat actcaggcac    30600
   tttttcttta ttttcttaac tttaaggttt tttttttttt aagatttatt tattattata    30660
   tctaagtaca ctgtagctgt cttcagacac accagaagag ggcgtcagat ctcattacaa    30720
   atggttgtga gccaccatgt ggttgctggg atttgaactc aggacctttg gaagagcagt    30780
   cagtgctctt acctgctgag ccatcttgcc acccccaact ttaaattttt tatactatta    30840
   tttttagaca gtctcactgg gcctaatgac ttacataggt ggcctggaac tcactatata    30900
   gatcaggcta gccttcaact cccagatatc cacctgcctc tgccacccaa atacttggat    30960
   taaaggcgtg tgcctccata cctagcctaa atcttcattt cttaaaatac tgttttgcta    31020
   agataggtaa agatttcctc ttaaaaataa atacttagca aatatatacc gatctcctaa    31080
   ttacttaatg aagggccagc ttaatagtta tcagtcagtt atcagtgcca gcccctactg    31140
   ctgggaattt agtgtataac gttcattgta tggtagactg aagtaattct aagtattttt    31200
   ttcttgggtg tgactatcaa acacagaaaa gtatttgaaa tttataaaga gaacaggttt    31260
   tttctttgca ttttatattt tgctatttat ttcttaccag aagatgcgag cagcaaagta    31320
   aaaggcagta agtgctgatg ggtttggagg aacttgggat tttaattata aaacttcaag    31380
   aaagcatttc aatggtgttc tagagtctaa aaaagaatag tgagacccta ttcctgttct    31440
   ctccgatcaa ccaagagctt gaaatggtgc tagtccttag tatacactga aaagacgcta    31500
   agtgtggtca tcccggttgg agggctttag gaagcagtga ccctggacca atgggtgtca    31560
   ccgtgtgtct gaagaagaaa gcagagctga aacaagaggc gcatggtagg gacaccagca    31620
   gccacagtaa actgctgccc agaggtccct gtgtggggct gcagaattaa aagaacccat    31680
   tctacacagc tctgctgtgc tctgttagtg ctgagaaagg ttgagaggaa ttgtttcaga    31740
   agaggaatcg ttcaaattga actcttatgt cactagttca catactggca atcttggaaa 31800
   acatagaaat tttctcactg agtctgcgtg cctgcgtctt cctcgtgact aatatacttg 31860
   aagtcctgtt tattttttta gttgattgtt tagaatctct tctcaggaaa tgaggtaaac 31920
   ttgaatggat ttgcaccatg ttagtgtttt tgttttgaat atgtttgttt ggaagatttg 31980
   aagaaaaagc aattgttcag ctattctggc atgacaaaat catgtcatga attttagaat 32040
   tttatttcca gttctaagta aatgttttga atataaaatt gtcagaaata ttttcagcca 32100
   caagattata tcttctatta ttgtgggctc atgatagtat cagtgtggtt taaataatat 32160
   tcacttttga gtctgggagg tttgaggttt cagattcagg gactcacaca ctgggcaatt 32220
   actgtaccac tatgcagttg cttattagta ccacagagta attcccagtt aagttacttt 32280
   taattttaac ctttttaaga taaaagcagt ctgatgatac attaaagtcg gacatttcct 32340
   tgaagatagt ctttcctttt ccagcttttg tgatccagat ctcattcagt aaagcagaaa 32400
   ttgggaaata gtggacttaa gttctaaggg acccacaaac cccgtgactg tgctgtccgt 32460
   tttcagccag taaccatgaa gtgctggcgt cccttccagc gcccctttct ccatttggtg 32520
   cactcatccc tcaaggctga gaggcgtgct gctctcctgt ctatttccct cttccccatg 32580
   gttcctgggc agtgatgttg tgatctctac catctgagtc ttgctttgca tttatcttac 32640
   tgtgaaaaat gttatatttt ccctctgaca tgaatataat agcctaggga aagacagaag 32700
   taaaacactg aaagggaatg ggggctgaga aaaaaacagt cattagcttc tgtctggcca 32760
   gcatgctgaa gtgggtcacc tcagttggcc attttgtctg aacgttacat gccagccaac 32820
   cttagctgcg gtagtaataa gttatgctgc tggctcatac ttacagatgg taagtctctt 32880
   gacctgaggc aaacgtgtaa ggtgacggtt ctaaacacac tgatggacag gcacatgccc 32940
   tgcctggata gcctcaaaac acaaacagtg tacaaatgta cccttgcgtt aaagtggatc 33000
   tatgtgcgtt tgtgtttatt ttctgtgcat taagtatgta tatgtatgtg tgtttatatt 33060
   gtgcacattg agtatatgca tgtgtgttta cactgaatac tgaacccacg gcctcctgca 33120
   aactaagtat gcattccaaa tgcacacatc tgtcttctta cacatctgtt tataaaactt 33180
   caactttttt actagagcaa gaagttgtgg aatgtaactc tgtaaaaccg tttaatatct 33240
   gaaccttttt cttcttagga atattttcca agatgtctta cacagagaca ctctagtgaa 33300
   agccttcctg gatcaggtaa atatgatgcc acccattgcc agacaaaaga acatcatata 33360
   ttttctttta aaatatgtcc cacagtgcct acagaatata taaaaagcac caaagaatta 33420
   aagtgctaga ggcctttcta aagtctgtaa acggattcct ctttgaatta ttaatgggaa 33480
   atagcctgta tattaaccgt taaagcagca ttctccatcc tagtggctgc ttcaggtcca 33540
   accctctgcc tttagaattt ttgtggttgg tgaagacagg ggtgtgcttt catttgtgtt 33600
   aattgaattg aaaatattct taaaacttag gttgcttctg cttaaatggt agcatcctta 33660
   ttgtctctgt ttttaaaagt atctgatgag taaacatctg gagatggtac tggattctat 33720
   gcgacttgtt tctatacgta agcagagctt tgtcataata gcatgctggg aatcaggcca 33780
   agatcctgtg ccatagacat agagttgaga tgaggagaac ctcgtgttca ctgggacttg 33840
   tgggtctggg tctgtgtgag gtgaggacag cctgtaatcc caagtctctg aagctgaaaa 33900
   gtcccctcct ctactccaca caacctgaag tcattgactt agttatttcc ataataaaat 33960
   aaggagatat tttaaggtag aatacaagat ctaagtgcat taaactaggg aatctgaaaa 34020
   ggggacagtg ggtttccaga catttgccgc taccagagtc ttgccctttg gaaatcggaa 34080
   gaaatggctg taatgggtgt tgtgtgtcag atcctgtcaa caatgtcgcg gaagctgcac 34140
   tgtcttgtgt ccctgcaggt cttccatttg aagcctggcc tgtctctcag gagtactttc 34200
   cttgcacagt tcctcctcat tcttcacaga aaagccttga cactaatcaa gtacatcgag 34260
   gatgatacgt gagtcctgct cctctagagg aaagccttta tgcattgaca gttgctgttc 34320
   gttccctttg aacattgtct gtattataat gcgggggttt ttgtctcttt tgttttgttt 34380
   ataggcagaa ggggaaaaag ccctttaagt ctcttcggaa cctgaagata gatcttgatt 34440
   taacagcaga gggcgatctt aacataataa tggctctagc tgagaaaatt aagccaggcc 34500
   tacactcttt catctttggg agacctttct acactagtgt acaagaacgt gatgttctaa 34560
   tgaccttttg accgtgtggt ttgctgtgtc tgtctcttca cagtcacacc tgctgttaca 34620
   gtgtctcagc agtgtgtggg cacatccttc ctcccgagtc ctgctgcagg acagggtaca 34680
   ctacacttgt cagtagaagt ctgtacctga tgtcaggtgc atcgttacag tgaatgactc 34740
   ttcctagaat agatgtactc ttttagggcc ttatgtttac aattatccta agtactattg 34800
   ctgtctttta aagatatgaa tgatggaata tacacttgac cataactgct gattggtttt 34860
   ttgttttgtt ttgtttgttt tcttggaaac ttatgattcc tggtttacat gtaccacact 34920
   gaaaccctcg ttagctttac agataaagtg tgagttgact tcctgcccct ctgtgttctg 34980
   tggtatgtcc gattacttct gccacagcta aacattagag catttaaagt ttgcagttcc 35040
   tcagaaagga acttagtctg actacagatt agttcttgag agaagacact gatagggcag 35100
   agctgtaggt gaaatcagtt gttagccctt cctttataga cgtagtcctt cagattcggt 35160
   ctgtacagaa atgccgaggg gtcatgcatg ggccctgagt atcgtgacct gtgacaagtt 35220
   ttttgttggt ttattgtagt tctgtcaaag aaagtggcat ttgtttttat aattgttgcc 35280
   aacttttaag gttaattttc attatttttg agccgaatta aaatgcgcac ctcctgtgcc 35340
   tttcccaatc ttggaaaata taatttcttg gcagagggtc agatttcagg gcccagtcac 35400
   tttcatctga ccaccctttg cacggctgcc gtgtgcctgg cttagattag aagtccttgt 35460
   taagtatgtc agagtacatt cgctgataag atctttgaag agcagggaag cgtcttgcct 35520
   ctttcctttg gtttctgcct gtactctggt gtttcccgtg tcacctgcat cataggaaca 35580
   gcagagaaat ctgacccagt gctatttttc taggtgctac tatggcaaac tcaagtggtc 35640
   tgtttctgtt cctgtaacgt tcgactatct cgctagctgt gaagtactga ttagtggagt 35700
   tctgtgcaac agcagtgtag gagtatacac aaacacaaat atgtgtttct atttaaaact 35760
   gtggacttag cataaaaagg gagaatatat ttatttttta caaaagggat aaaaatgggc 35820
   cccgttcctc acccaccaga tttagcgaga aaaagctttc tattctgaaa ggtcacggtg 35880
   gctttggcat tacaaatcag aacaacacac actgaccatg atggcttgtg aactaactgc 35940
   aaggcactcc gtcatggtaa gcgagtaggt cccacctcct agtgtgccgc tcattgcttt 36000
   acacagtaga atcttatttg agtgctaatt gttgtctttg ctgctttact gtgttgttat 36060
   agaaaatgta agctgtacag tgaataagtt attgaagcat gtgtaaacac tgttatatat 36120
   cttttctcct agatggggaa ttttgaataa aatacctttg aaattctgtg tatgttttag 36180
   ttcattattt agggaaaacg ctgctgtgaa agggggcgtg atcagcttcc tattctgcga 36240
   cagtcgtgtt gaacggaacc cattggtttt catcttcgct ccccccccct tggtttttcg 36300
   agacagggtt tctctgtata gccctggctg tcctggacct cactctgtag accaggctgg 36360
   cctcgaactc agaaatctac ctgcctctgc ctcccaagtg ctgggaggca gttgccccac 36420
   caactagtct tcttttttca aagaagatat ttaaagctaa cgaataatgc tagactctta 36480
   catcttaaaa aaaaaagaag agaaaagaaa agaaaaggta atcacactgc ccagtgtgta 36540
   gtgcatgctt ctacttccgg tccttgggag atgggggcag gatgagacgc tccagaccgg 36600
   cttccaatac agagttcaag acccactgag ctacgtgagg ctacacgagc ctgcctttaa 36660
   aaacataaag ctaaagcttt cttcttaact tccagtattg caccttgatt cccccttcaa 36720
   atttcacata caaaataatt cttaaattct cttttgaaaa atgttctact gaggccagag 36780
   agacagttcg cttggtaaag gtgcctgttg ccaaacgtga taacctgagt taaatcatag 36840
   ccccacatgg gggaggaaga aacccccgca gcttgccctc tgatgccatg tatgcactaa 36900
   aacacgcacg tgtgtgcgca cacatttttt aagttcctat tacattgata gtaatataat 36960
   ttaaactgat ttattctccc caagtcattg atacgggtgt ccaacgtaaa atccagcggc 37020
   tgaacaaagc acttttaggc gctttaagtt ggaaagcaag aaacggagat tgacactgtc 37080
   actccaagag aaaactcttc gtagtagcga gatcggctgt ggagtgaaga tgctcagagg 37140
   ctgggaacgc acacagctca ggagtggata gcatccccca gcctcaactc ctaacactgg 37200
   gaaagcgtag ggctctcaga tgaggaaaca aaaccataca aagctgctgc aagctaaaca 37260
   gaaaaatagt ggcattacac taactgttgt ggaattgtac agaccgattc tcctcccaat 37320
   ctgccgagtg tgggcggctt gagagaatga agagagctac tggcctcagg taacagtgct 37380
   tcccacagga ctgtctcagg ctgccaccac cataaatagc attttagacg tgacagagct 37440
   aaggcttgac acacagccaa aagctactca cattccattt catccccagc tgttctgtca 37500
   tcgctaagca cagagcattc agcacagctc ttccctgtgg tgggtactca gcactgttga 37560
   gttgaaagga ttgaaaaaac tcaagactat gttctcaaac atttttttaa gctcttttta 37620
   aaaccacctt agaatgaaag cttttgactt cttattaaca tgcactaact tcatatacac 37680
   atttagtgtt attgtacagg cacgaagcat actctggtca gaacctgtct cctttggtcc 37740
   accctcccca ccgttttcag cttctattcc accttccata cgtctcaaga tccacatgtg 37800
   agagggaaca ctcagagcct tgtctttctg tatctgggat atctcactta acatgatatt 37860
   ctccagttct gttccatcca tttcattgca aagagcaaga tttcactcta cagccaaata 37920
   acacatttgt ccatgtatat ccgtattttt ccttattcat ctgttgaatg gcacaagact 37980
   gatatcatgg gtaatatcta t 38001
<210> 94
   <211> 3435
   <212> DNA
   <213> Rattus norvegicus
<220>
   <221> CDS
   <222> (370)..(1815)
<400> 94
   cgtttgtagt gtcagccatc ccaattgcct gttccttctc tgtgggagtg gtgtctagac       60
   agtccaggca gggtatgcta ggcaggtgcg ttttggttgc ctcagatcgc aacttgactc      120
   cataacggtg accaaagaca aaagaaggaa accagattaa aaagaaccgg acacagaccc      180
   ctgcagaatc tggagcggcc gtggttgggg gcggggctac gacggggcgg actcgggggc      240
   gtgggagggc ggggccgggg cggggcccgg agccggctgc ggttgcggtc cctgcgccgg      300
   cggtgaaggc gcagcggcgg cgagtggcta ttgcaagcgt ttggataatg tgagacctgg      360
   gatgcaggg atg tcg act atc tgc ccc cca cca tct cct gct gtt gcc aag      411
             Met Ser Thr Ile Cys Pro Pro Pro Ser Pro Ala Val Ala Lys
             1               5                   10
   aca gag att gct tta agt ggt gaa tca ccc ttg ttg gcg gct acc ttt        459
   Thr Glu Ile Ala Leu Ser Gly Glu Ser Pro Leu Leu Ala Ala Thr Phe
   15                  20                  25                  30
   gct tac tgg gat aat att ctt ggt cct aga gta agg cac att tgg gct        507
   Ala Tyr Trp Asp Asn Ile Leu Gly Pro Arg Val Arg His Ile Trp Ala
                   35                  40                  45
   cca aag aca gac caa gta ctc ctc agt gat gga gaa atc act ttt ctt        555
   Pro Lys Thr Asp Gln Val Leu Leu Ser Asp Gly Glu Ile Thr Phe Leu
               50                  55                  60
   gcc aac cac act ctg aat gga gaa att ctt cgg aat gcg gag agt ggg        603
   Ala Asn His Thr Leu Asn Gly Glu Ile Leu Arg Asn Ala Glu Ser Gly
           65                  70                  75
   gca ata gat gta aag ttt ttt gtc tta tct gaa aag ggc gtc att att        651
   Ala Ile Asp Val Lys Phe Phe Val Leu Ser Glu Lys Gly Val Ile Ile
       80                  85                  90
   gtt tca tta atc ttc gac ggg aac tgg aac gga gat cgg agc act tac        699
   Val Ser Leu Ile Phe Asp Gly Asn Trp Asn Gly Asp Arg Ser Thr Tyr
   95                  100                 105                 110
   gga cta tca att ata ctg ccg cag acg gag ctg agt ttc tac ctc cca        747
   Gly Leu Ser Ile Ile Leu Pro Gln Thr Glu Leu Ser Phe Tyr Leu Pro
                   115                 120                 125
   ctg cac aga gtg tgt gtt gac agg cta acg cac atc att cga aaa gga        795
   Leu His Arg Val Cys Val Asp Arg Leu Thr His Ile Ile Arg Lys Gly
               130                 135                 140
   agg ata tgg atg cac aag gaa aga caa gaa aat gtc cag aaa att gtc        843
   Arg Ile Trp Met His Lys Glu Arg Gln Glu Asn Val Gln Lys Ile Val
           145                 150                 155
   ttg gaa ggc acc gag agg atg gaa gat cag ggt cag agt atc atc cct        891
   Leu Glu Gly Thr Glu Arg Met Glu Asp Gln Gly Gln Ser Ile Ile Pro
       160                 165                 170
   atg ctt act ggg gag gtc atc cct gtg atg gag ctg ctt gcg tct atg        939
   Met Leu Thr Gly Glu Val Ile Pro Val Met Glu Leu Leu Ala Ser Met
   175                 180                 185                 190
   aga tca cac agt gtt cct gaa gac ctc gat ata gct gat aca gta ctc        987
   Arg Ser His Ser Val Pro Glu Asp Leu Asp Ile Ala Asp Thr Val Leu
                   195                 200                 205
   aat gat gat gac att ggt gac agc tgt cat gaa ggc ttt ctt ctc aat       1035
   Asn Asp Asp Asp Ile Gly Asp Ser Cys His Glu Gly Phe Leu Leu Asn
               210                 215                 220
   gcc atc agc tca cat ctg cag acc tgc ggc tgt tct gtg gtg gta ggc       1083
   Ala Ile Ser Ser His Leu Gln Thr Cys Gly Cys Ser Val Val Val Gly
           225                 230                 235
   agc agt gca gag aaa gta aat aag ata gta aga aca ctg tgc ctt ttt       1131
   Ser Ser Ala Glu Lys Val Asn Lys Ile Val Arg Thr Leu Cys Leu Phe
       240                 245                 250
   ctg aca cca gca gag agg aag tgc tcc agg ctg tgt gaa gcc gaa tcg       1179
   Leu Thr Pro Ala Glu Arg Lys Cys Ser Arg Leu Cys Glu Ala Glu Ser
   255                 260                 265                 270
   tcc ttt aaa tac gaa tct gga ctc ttt gta caa ggc ttg cta aag gat       1227
   Ser Phe Lys Tyr Glu Ser Gly Leu Phe Val Gln Gly Leu Leu Lys Asp
                   275                 280                 285
   gcg act ggc agt ttt gta cta cct ttc cgg caa gtt atg tat gcc cct       1275
   Ala Thr Gly Ser Phe Val Leu Pro Phe Arg Gln Val Met Tyr Ala Pro
               290                 295                 300
   tat ccc acc aca cac atc gat gtg gat gtc aac act gtc aag cag atg       1323
   Tyr Pro Thr Thr His Ile Asp Val Asp Val Asn Thr Val Lys Gln Met
           305                 310                 315
   cca ccg tgt cat gaa cat att tat aat caa cgc aga tac atg agg tca       1371
   Pro Pro Cys His Glu His Ile Tyr Asn Gln Arg Arg Tyr Met Arg Ser
       320                 325                 330
   gag ctg aca gcc ttc tgg agg gca act tca gaa gag gac atg gct cag      1419
   Glu Leu Thr Ala Phe Trp Arg Ala Thr Ser Glu Glu Asp Met Ala Gln
   335                 340                 345                 350
   gac acc atc atc tac aca gat gag agc ttc act cct gat ttg aat att      1467
   Asp Thr Ile Ile Tyr Thr Asp Glu Ser Phe Thr Pro Asp Leu Asn Ile
                   355                 360                 365
   ttc caa gat gtc tta cac aga gac act cta gtg aaa gcc ttt ctg gat      1515
   Phe Gln Asp Val Leu His Arg Asp Thr Leu Val Lys Ala Phe Leu Asp
               370                 375                 380
   cag gtc ttc cat ttg aag cct ggc ctg tct ctc agg agt act ttc ctt      1563
   Gln Val Phe His Leu Lys Pro Gly Leu Ser Leu Arg Ser Thr Phe Leu
           385                 390                 395
   gca cag ttc ctc ctc att ctt cac aga aaa gcc ttg aca cta atc aag      1611
   Ala Gln Phe Leu Leu Ile Leu His Arg Lys Ala Leu Thr Leu Ile Lys
       400                 405                 410
   tac ata gag gat gac acg cag aag ggg aaa aag ccc ttt aag tct ctt      1659
   Tyr Ile Glu Asp Asp Thr Gln Lys Gly Lys Lys Pro Phe Lys Ser Leu
   415                 420                 425                 430
   cgg aac ctg aag ata gat ctt gat tta aca gca gag ggc gac ctt aac      1707
   Arg Asn Leu Lys Ile Asp Leu Asp Leu Thr Ala Glu Gly Asp Leu Asn
                   435                 440                 445
   ata ata atg gct cta gct gag aaa att aag cca ggc cta cac tct ttc      1755
   Ile Ile Met Ala Leu Ala Glu Lys Ile Lys Pro Gly Leu His Ser Phe
               450                 455                 460
   atc ttc ggg aga cct ttc tac act agt gtc caa gaa cgt gat gtt cta      1803
   Ile Phe Gly Arg Pro Phe Tyr Thr Ser Val Gln Glu Arg Asp Val Leu
           465                 470                 475
   atg act ttt taa acatgtggtt tgctccgtgt gtctcatgac agtcacactt          1855
   Met Thr Phe
       480
   gctgttacag tgtctcagcg ctttggacac atccttcctc cagggtcctg ccgcaggaca    1915
   cgttacacta cacttgtcag tagaggtctg taccagatgt caggtacatc gttgtagtga    1975
   atgtctcttt tcctagacta gatgtaccct cgtagggact tatgtttaca accctcctaa    2035
   gtactagtgc tgtcttgtaa ggatacgaat gaagggatgt aaacttcacc acaactgctg    2095
   gttggttttg ttgtttttgt tttttgaaac ttataattca tggtttacat gcatcacact    2155
   gaaaccctag ttagcttttt acaggtaagc tgtgagttga ctgcctgtcc ctgtgttctc    2215
   tggcctgtac gatctgtggc gtgtaggatc acttttgcaa caactaaaaa ctaaagcact    2275
   ttgtttgcag ttctacagaa agcaacttag tctgtctgca gattcgtttt tgaaagaaga    2335
   catgagaaag cggagtttta ggtgaagtca gttgttggat cttcctttat agacttagtc    2395
   ctttagatgt ggtctgtata gacatgccca accatcatgc atgggcactg aatatcgtga    2455
   actgtggtat gctttttgtt ggtttattgt acttctgtca aagaaagtgg cattggtttt     2515
   tataattgtt gccaagtttt aaggttaatt ttcattattt ttgagccaaa ttaaaatgtg     2575
   cacctcctgt gcctttccca atcttggaaa atataatttc ttggcagaag gtcagatttc     2635
   agggcccagt cactttcgtc tgacttccct ttgcacagtc cgccatgggc ctggcttaga     2695
   agttcttgta aactatgcca gagagtacat tcgctgataa aatcttcttt gcagagcagg     2755
   agagcttctt gcctctttcc tttcatttct gcctggactt tggtgttctc cacgttccct     2815
   gcatcctaag gacagcagga gaactctgac cccagtgcta tttctctagg tgctattgtg     2875
   gcaaactcaa gcggtccgtc tctgtccctg taacgttcgt accttgctgg ctgtgaagta     2935
   ctgactggta aagctccgtg ctacagcagt gtagggtata cacaaacaca agtaagtgtt     2995
   ttatttaaaa ctgtggactt agcataaaaa gggagactat atttattttt tacaaaaggg     3055
   ataaaaatgg aaccctttcc tcacccacca gatttagtca gaaaaaaaca ttctattctg     3115
   aaaggtcaca gtggttttga catgacacat cagaacaacg cacactgtcc atgatggctt     3175
   atgaactcca agtcactcca tcatggtaaa tgggtagatc cctccttcta gtgtgccaca     3235
   ccattgcttc ccacagtaga atcttattta agtgctaagt gttgtctctg ctggtttact     3295
   ctgttgtttt agagaatgta agttgtatag tgaataagtt attgaagcat gtgtaaacac     3355
   tgttatacat cttttctcct agatggggaa tttggaataa aataccttta aaattcaaaa     3415
   aaaaaaaaaa aaaaaaaaaa                                                   3435

## Description

### Field

Provided are compositions and methods for inhibiting expression of C9ORF72 antisense transcript in an animal. Such compositions and methods are useful to treat, prevent, or ameliorate neurodegenerative diseases, including amyotrophic lateral sclerosis (ALS), frontotemporal dementia (FTD), corticalbasal degeneration syndrome (CBD), atypical Parkinsonian syndrome, and olivopontocerellar degeneration (OPCD).

### Background

Amyotrophic lateral sclerosis (ALS) is a fatal neurodegenerative disease characterized clinically by progressive paralysis leading to death from respiratory failure, typically within two to three years of symptom onset (Rowland and Shneider, N. Engl. J. Med., 2001, 344, 1688-1700). ALS is the third most common neurodegenerative disease in the Western world (Hirtz et al., Neurology, 2007, 68, 326-337), and there are currently no effective therapies. Approximately 10% of cases are familial in nature, whereas the bulk of patients diagnosed with the disease are classified as sporadic as they appear to occur randomly throughout the population (Chio et al., Neurology, 2008, 70, 533-537). There is growing recognition, based on clinical, genetic, and epidemiological data, that ALS and frontotemporal dementia (FTD) represent an overlapping continuum of disease, characterized pathologically by the presence of TDP-43 positive inclusions throughout the central nervous system (Lillo and Hodges, J. Clin. Neurosci., 2009, 16, 1131-1135; Neumann et al., Science, 2006, 314, 130-133).

To date, a number of genes have been discovered as causative for classical familial ALS, for example, SOD1, TARDBP, FUS, OPTN, and VCP (Johnson et al., Neuron, 2010, 68, 857-864; Kwiatkowski et al., Science, 2009, 323, 1205-1208; Maruyama et al., Nature, 2010, 465, 223-226; Rosen et al., Nature, 1993, 362, 59-62; Sreedharan et al., Science, 2008, 319, 1668-1672; Vance et al., Brain, 2009, 129, 868-876). Recently, linkage analysis of kindreds involving multiple cases of ALS, FTD, and ALS-FTD had suggested that there was an important locus for the disease on the short arm of chromosome 9 (Boxer et al., J. Neurol. Neurosurg. Psychiatry, 2011, 82, 196-203; Morita et al., Neurology, 2006, 66, 839-844; Pearson et al. J. Nerol., 2011, 258, 647-655; Vance et al., Brain, 2006, 129, 868-876). This mutation has been found to be the most common genetic cause of ALS and FTD. It is postulated that the ALS-FTD causing mutation is a large hexanucleotide (GGGGCC) repeat expansion in the first intron of the C9ORF72 gene (Renton et al., Neuron, 2011, 72, 257-268; DeJesus-Hernandez et al., Neuron, 2011, 72, 245-256). A founder haplotype, covering the C9ORF72 gene, is present in the majority of cases linked to this region (Renton et al., Neuron, 2011, 72, 257-268). This locus on chromosome 9p21 accounts for nearly half of familial ALS and nearly one-quarter of all ALS cases in a cohort of 405 Finnish patients (Laaksovirta et al, Lancet Neurol., 2010, 9, 978-985).

There are currently no effective therapies to treat such neurodegenerative diseases. Therefore, it is an object to provide compositions and methods for the treatment of such neurodegenerative diseases.

Ciura et al., 2013, Annals of Neurology., 74(2), 180-187 reports that loss of function of C9orf72 causes motor deficits in a zebrafish model of amyotrophic lateral sclerosis.

### Summary

The invention provides a compound comprising a modified oligonucleotide consisting of 12 to 50 linked nucleosides wherein the nucleobase sequence of the modified oligonucleotide is at least 90% complementary to an equal length portion of a C9ORF72 antisense transcript, wherein the C9ORF72 antisense transcript is encoded by SEQ ID NO: 11, wherein the modified oligonucleotide comprises at least one modification selected from a modified sugar, a sugar surrogate, and a modified internucleoside linkage, and wherein the modified oligonucleotide is capable of reducing the level of expression of the C9ORF72 antisense transcript.

The invention also provides a modified oligonucleotide consisting of 12 to 50 linked nucleosides and having a nucleobase sequence comprising at least 8 contiguous nucleobases of a sequence selected from among SEQ ID NOs: 19, 20, 22-26, 28-32, 34-42, 44, 46-55, 58, 59, and 61, wherein the modified oligonucleotide is capable of reducing the level of expression of a C9ORF72 antisense transcript, and wherein the C9ORF72 antisense transcript is encoded by SEQ ID NO: 11.

The invention also provides a composition comprising a compound of the invention, a double stranded compound of the invention, a conjugated compound of the invention, or a modified oligonucleotide of the invention and a pharmaceutically acceptable diluent or carrier, optionally wherein the pharmaceutically acceptable diluent is phosphate-buffered saline (PBS); and/or the modified oligonucleotide of the compound is a sodium salt.

The invention also provides a compound of the invention, a double stranded compound of the invention, a conjugated compound of the invention, a composition of the invention, or a modified oligonucleotide of the invention for use in treating a neurodegenerative disease.

Described herein are compositions and methods for modulating levels of C9ORF72 antisense transcript in cells, tissues, and animals. In certain embodiments of the disclosure, C9ORF72 antisense transcript specific inhibitors modulate expression of C9ORF72 antisense transcript. In certain embodiments of the disclosure, C9ORF72 antisense transcript specific inhibitors are nucleic acids, proteins, or small molecules.

In certain embodiments, modulation can occur in a cell or tissue. In certain embodiments, the cell or tissue is in an animal. In certain embodiments, the animal is a human. In certain embodiments, C9ORF72 antisense transcript levels are reduced. In certain embodiments, C9ORF72 antisense transcript associated RAN translation products are reduced. In certain embodiments, the C9ORF72 antisense transcript associated RAN translation products are poly-(proline-alanine), poly-(proline-arginine), and poly-(proline-glycine). In certain embodiments, the C9ORF72 antisense transcript contains a hexanucleotide repeat expansion. In certain embodiments, the hexanucleotide repeat is transcribed in the antisense direction from the C9ORF72 gene. In certain embodiments, the hexanucleotide repeat expansion is associated with a C9ORF72 associated disease. In certain embodiments, the hexanucleotide repeat expansion is associated with a C9ORF72 hexanucleotide repeat expansion associated disease. In certain embodiments, the hexanucleotide repeat expansion comprises at least 24 GGCCCC, CCCCCC, GCCCCC, and/or CGCCCC repeats. In certain embodiments, the hexanucleotide repeat expansion is associated with nuclear foci. In certain embodiments, C9ORF72 antisense transcript associated RAN translation products are associated with nuclear foci. In certain embodiments, the antisense transcript associated RAN translation products are poly-(proline-alanine) and/or poly-(proline-arginine). In certain embodiments, the compositions and methods described herein are useful for reducing C9ORF72 antisense transcript levels, C9ORF72 antisense transcript associated RAN translation products, and nuclear foci. Such reduction can occur in a time-dependent manner or in a dose-dependent manner.

Also described are methods useful for preventing, treating, ameliorating, and slowing progression of diseases and conditions associated with C9ORF72. In certain embodiments, such diseases and conditions associated with C9ORF72 are neurodegenerative diseases. In certain embodiments, the neurodegenerative disease is amyotrophic lateral sclerosis (ALS), frontotemporal dementia (FTD), corticalbasal degeneration syndrome (CBD), atypical Parkinsonian syndrome, or olivopontocerellar degeneration (OPCD).

Such diseases and conditions can have one or more risk factors, causes, or outcomes in common. Certain risk factors and causes for development of a neurodegenerative disease, and, in particular, ALS and FTD, include genetic predisposition and older age.

Described herein are methods of treatment include administering a C9ORF72 antisense transcript specific inhibitor to an individual in need thereof. In certain embodiments, the C9ORF72 antisense transcript specific inhibitor is a nucleic acid. In certain embodiments, the nucleic acid is an antisense compound. In certain embodiments, the antisense compound is an antisense oligonucleotide. In certain embodiments, the antisense oligonucleotide is complementary to a C9ORF72 antisense transcript. In certain embodiments, the antisense oligonucleotide is a modified antisense oligonucleotide.

### Detailed Description

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Additionally, as used herein, the use of "and" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings: "2'-O-methoxyethyl" (also 2'-MOE and 2'-OCH₂CH₂-OCH₃ and MOE) refers to an O-methoxyethyl modification of the 2' position of a furanose ring. A 2'-O-methoxyethyl modified sugar is a modified sugar.

"2'-MOE nucleoside" (also 2'-O-methoxyethyl nucleoside) means a nucleoside comprising a MOE modified sugar moiety.

"2'-substituted nucleoside" means a nucleoside comprising a substituent at the 2'-position of the furanose ring other than H or OH. In certain embodiments, 2'-substituted nucleosides include nucleosides with bicyclic sugar modifications.

"5-methylcytosine" means a cytosine modified with a methyl group attached to the 5' position. A 5-methylcytosine is a modified nucleobase.

"About" means within ±7% of a value. For example, if it is stated, "the compounds affected at least about 70% inhibition of C90RF72 antisense trascript", it is implied that the C90RF72 antisense transcript levels are inhibited within a range of 63% and 77%.

"Administered concomitantly" refers to the co-administration of two pharmaceutical agents in any manner in which the pharmacological effects of both are manifest in the patient at the same time. Concomitant administration does not require that both pharmaceutical agents be administered in a single pharmaceutical composition, in the same dosage form, or by the same route of administration. The effects of both pharmaceutical agents need not manifest themselves at the same time. The effects need only be overlapping for a period of time and need not be coextensive.

"Administering" means providing a pharmaceutical agent to an animal, and includes, but is not limited to administering by a medical professional and self-administering.

"Amelioration" refers to a lessening, slowing, stopping, or reversing of at least one indicator of the severity of a condition or disease. The severity of indicators may be determined by subjective or objective measures, which are known to those skilled in the art.

"Animal" refers to a human or non-human animal, including, but not limited to, mice, rats, rabbits, dogs, cats, pigs, and non-human primates, including, but not limited to, monkeys and chimpanzees.

"Antibody" refers to a molecule characterized by reacting specifically with an antigen in some way, where the antibody and the antigen are each defined in terms of the other. Antibody may refer to a complete antibody molecule or any fragment or region thereof, such as the heavy chain, the light chain, Fab region, and Fc region.

"Antisense activity" means any detectable or measurable activity attributable to the hybridization of an antisense compound to its target nucleic acid. In certain embodiments, antisense activity is a decrease in the amount or expression of a target nucleic acid or protein product encoded by such target nucleic acid.

"Antisense compound" means an oligomeric compound that is capable of undergoing hybridization to a target nucleic acid through hydrogen bonding. Examples of antisense compounds include single-stranded and double-stranded compounds, such as, antisense oligonucleotides, siRNAs, shRNAs, ssRNAs, and occupancy-based compounds.

"Antisense inhibition" means reduction of target nucleic acid levels in the presence of an antisense compound complementary to a target nucleic acid compared to target nucleic acid levels or in the absence of the antisense compound.

"Antisense mechanisms" are all those mechanisms involving hybridization of a compound with a target nucleic acid, wherein the outcome or effect of the hybridization is either target degradation or target occupancy with concomitant stalling of the cellular machinery involving, for example, transcription or splicing.

"Antisense oligonucleotide" means a single-stranded oligonucleotide having a nucleobase sequence that permits hybridization to a corresponding segment of a target nucleic acid.

"Base complementarity" refers to the capacity for the precise base pairing of nucleobases of an antisense oligonucleotide with corresponding nucleobases in a target nucleic acid (i.e., hybridization), and is mediated by Watson-Crick, Hoogsteen or reversed Hoogsteen hydrogen binding between corresponding nucleobases.

"Bicyclic sugar" means a furanose ring modified by the bridging of two atoms. A bicyclic sugar is a modified sugar.

"Bicyclic nucleoside" (also BNA) means a nucleoside having a sugar moiety comprising a bridge connecting two carbon atoms of the sugar ring, thereby forming a bicyclic ring system. In certain embodiments, the bridge connects the 4'-carbon and the 2'-carbon of the sugar ring.

"C90RF72 antisense transcript" means transcripts produced from the non-coding strand (also antisense strand and template strand) of the C90RF72 gene. The C90RF72 antisense transcript differs from the canonically transcribed "C90RF72 sense transcript", which is produced from the coding strand (also sense strand) of the C90RF72 gene.

"C90RF72 antisense transcript associated RAN translation products" means aberrant peptide or di-peptide polymers translated through RAN translation (i.e., repeat-associated, and non-ATG-dependent translation). In certain embodiments, the C90RF72 antisense transcript associated RAN translation products are any of poly-(proline-alanine), poly-(proline-arginine), and poly-(proline-glycine).

"C90RF72 antisense transcript specific inhibitor" refers to any agent capable of specifically inhibiting the expression of C90RF72 antisense transcript and/or its expression products at the molecular level. For example, C90RF72 specific antisense transcript inhibitors include nucleic acids (including antisense compounds), siRNAs, aptamers, antibodies, peptides, small molecules, and other agents capable of inhibiting the expression of C90RF72 antisense transcript and/or its expression products, such as C90RF72 antisense transcript associated RAN translation products.

"C90RF72 associated disease" means any disease associated with any C90RF72 nucleic acid or expression product thereof, regardless of which DNA strand the C90RF72 nucleic acid or expression product thereof is derived from. Such diseases may include a neurodegenerative disease. Such neurodegenerative diseases may include ALS and FTD.

"C90RF72 foci" means nuclear foci comprising a C90RF72 transcript. In certain embodiments, a C90RF72 foci comprises at least one C90RF72 sense transcript (herein "C90RF72 sense foci"). In certain embodiments, C90RF72 sense foci comprise C90RF72 sense transcripts comprising any of the following hexanucleotide repeats: GGGGCC, GGGGGG, GGGGGC, and/or GGGGCG. In certain embodiments, a C90RF72 foci comprises at least one C90RF72 antisense transcript (herein "C90RF72 antisense foci"). In certain embodiments, C90RF72 antisense foci comprise C90RF72 antisense transcripts comprising any of the following hexanucleotide repeats: GGCCCC, CCCCCC, GCCCCC, and/or CGCCCC. In certain embodiments, C90RF72 foci comprise both C90RF72 sense transcripts and C90RF72 antisense transcripts.

"C90RF72 hexanucleotide repeat expansion associated disease" means any disease associated with a C90RF72 nucleic acid containing a hexanucleotide repeat expansion. In certain embodiments, the hexanucleotide repeat expansion may comprise any of the following hexanucleotide repeats: GGGGCC, GGGGGG, GGGGGC, GGGGCG, GGCCCC, CCCCCC, GCCCCC, and/or CGCCCC. In certain embodiments, the hexanucleotide repeat is repeated at least 24 times. Such diseases may include a neurodegenerative disease. Such neurodegenerative diseases may include ALS and FTD.

"C90RF72 nucleic acid" means any nucleic acid derived from the C90RF72 locus, regardless of which DNA strand the C90RF72 nucleic acid is derived from. In certain embodiments, a C90RF72 nucleic acid includes a DNA sequence encoding C90RF72, an RNA sequence transcribed from DNA encoding C90RF72 including genomic DNA comprising introns and exons (i.e., pre-mRNA), and an mRNA sequence encoding C90RF72. "C90RF72 mRNA" means an mRNA encoding a C90RF72 protein. In certain embodiments, a C90RF72 nucleic acid includes transcripts produced from the coding strand of the C90RF72 gene. C90RF72 sense transcripts are examples of C90RF72 nucleic acids.In certain embodiments, a C90RF72 nucleic acid includes transcripts produced from the non-coding strand of the C90RF72 gene. C90RF72 antisense transcripts are examples of C90RF72 nucleic acids.

"C90RF72 pathogenic associated mRNA variant" means the C90RF72 mRNA variant processed from a C90RF72 pre-mRNA variant containing the hexanucleotide repeat. A C90RF72 pre-mRNA contains the hexanucleotide repeat when transcription of the pre-mRNA begins in the region from the start site of exon 1A to the start site of exon 1B, e.g., nucleotides 1107 to 1520 of the genomic sequence (SEQ ID NO: 2, the complement of GENBANK Accession No. NT_008413.18 truncated from nucleosides 27535000 to 27565000). In certain embodiments, the level of a C90RF72 pathogenic associated mRNA variant is measured to determine the level of a C90RF72 pre-mRNA containting the hexanucleotide repeat in a sample.

"C90RF72 transcript" means an RNA transcribed from C90RF72. In certain embodiments, a C90RF72 transcript is a C90RF72 sense transcript. In certain embodiments, a C90RF72 transcript is a C90RF72 antisense transcript.

"Cap structure" or "terminal cap moiety" means chemical modifications, which have been incorporated at either terminus of an antisense compound.

"cEt" or "constrained ethyl" means a bicyclic nucleoside having a sugar moiety comprising a bridge connecting the 4'-carbon and the 2'-carbon, wherein the bridge has the formula: 4'-CH(CH₃)-O-2'.

"Constrained ethyl nucleoside" (also cEt nucleoside) means a nucleoside comprising a bicyclic sugar moiety comprising a 4'-CH(CH₃)-O-2' bridge.

"Chemically distinct region" refers to a region of an antisense compound that is in some way chemically different than another region of the same antisense compound. For example, a region having 2'-O-methoxyethyl nucleosides is chemically distinct from a region having nucleosides without 2'-O-methoxyethyl modifications.

"Chimeric antisense compound" means an antisense compound that has at least two chemically distinct regions, each position having a plurality of subunits.

"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses parallel or sequential administration.

"Complementarity" means the capacity for pairing between nucleobases of a first nucleic acid and a second nucleic acid.

"Comprise," "comprises," and "comprising" will be understood to imply the inclusion of a stated step or element or group of steps or elements but not the exclusion of any other step or element or group of steps or elements.

"Contiguous nucleobases" means nucleobases immediately adjacent to each other.

"Designing" or"designed to" refer to the process of designing an oligomeric compound that specifically hybridizes with a selected nucleic acid molecule.

"Diluent" means an ingredient in a composition that lacks pharmacological activity, but is pharmaceutically necessary or desirable. For example, in drugs that are injected, the diluent may be a liquid, e.g. saline solution.

"Dose" means a specified quantity of a pharmaceutical agent provided in a single administration, or in a specified time period. In certain embodiments, a dose may be administered in one, two, or more boluses, tablets, or injections. For example, in certain embodiments where subcutaneous administration is desired, the desired dose requires a volume not easily accommodated by a single injection, therefore, two or more injections may be used to achieve the desired dose. In certain embodiments, the pharmaceutical agent is administered by infusion over an extended period of time or continuously. Doses may be stated as the amount of pharmaceutical agent per hour, day, week, or month.

"Effective amount" in the context of modulating an activity or of treating or preventing a condition means the administration of that amount of pharmaceutical agent to a subject in need of such modulation, treatment, or prophylaxis, either in a single dose or as part of a series, that is effective for modulation of that effect, or for treatment or prophylaxis or improvement of that condition. The effective amount may vary among individuals depending on the health and physical condition of the individual to be treated, the taxonomic group of the individuals to be treated, the formulation of the composition, assessment of the individual's medical condition, and other relevant factors.

"Efficacy" means the ability to produce a desired effect.

"Expression" includes all the functions by which a gene's coded information, regardless of which DNA strand the coded information is derived from, is converted into structures present and operating in a cell. Such structures include, but are not limited to the products of transcription and translation, including RAN translation.

"Fully complementary" or "100% complementary" means each nucleobase of a first nucleic acid has a complementary nucleobase in a second nucleic acid. In certain embodiments, a first nucleic acid is an antisense compound and a target nucleic acid is a second nucleic acid.

"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap" and the external regions may be referred to as the "wings."

"Gap-narrowed" means a chimeric antisense compound having a gap segment of 9 or fewer contiguous 2'-deoxyribonucleosides positioned between and immediately adjacent to 5' and 3' wing segments having from 1 to 6 nucleosides.

"Gap-widened" means a chimeric antisense compound having a gap segment of 12 or more contiguous 2'-deoxyribonucleosides positioned between and immediately adjacent to 5' and 3' wing segments having from 1 to 6 nucleosides.

"Hexanucleotide repeat expansion" means a series of six bases (for example, GGGGCC, GGGGGG, GGGGGC, GGGGCG, GGCCCC, CCCCCC, GCCCCC, and/or CGCCCC) repeated at least twice. In certain embodiments, the hexanucleotide repeat expansion may be located in intron 1 of a C90RF72 nucleic acid. In certain embodiments, the hexanucleotide repeat may be transcribed in the antisense direction from the C90RF72 gene. In certain embodiments, a pathogenic hexanucleotide repeat expansion includes at least 24 repeats of GGGGCC, GGGGGG, GGGGGC, GGGGCG, GGCCCC, CCCCCC, GCCCCC, and/or CGCCCC in a C90RF72 nucleic acid and is associated with disease. In certain embodiments, the repeats are consecutive. In certain embodiments, the repeats are interrupted by 1 or more nucleobases. In certain embodiments, a wild-type hexanucleotide repeat expansion includes 23 or fewer repeats of GGGGCC, GGGGGG, GGGGGC, GGGGCG, GGCCCC, CCCCCC, GCCCCC, and/or CGCCCC in a C90RF72 nucleic acid. In certain embodiments, the repeats are consecutive. In certain embodiments, the repeats are interrupted by 1 or more nucleobases.

"Hybridization" means the annealing of complementary nucleic acid molecules. In certain embodiments, complementary nucleic acid molecules include, but are not limited to, an antisense compound and a target nucleic acid. In certain embodiments, complementary nucleic acid molecules include, but are not limited to, an antisense oligonucleotide and a nucleic acid target.

"Identifying an animal having a C90RF72 associated disease" means identifying an animal having been diagnosed with a C90RF72 associated disease or predisposed to develop a C90RF72 associated disease. Individuals predisposed to develop a C90RF72 associated disease include those having one or more risk factors for developing a C90RF72 associated disease, including, having a personal or family history or genetic predisposition of one or more C90RF72 associated diseases. In certain embodiments, the C90RF72 associated disease is a C90RF72 hexanucleotide repeat expansion associated disease. Such identification may be accomplished by any method including evaluating an individual's medical history and standard clinical tests or assessments, such as genetic testing.

"Immediately adjacent" means there are no intervening elements between the immediately adjacent elements.

"Individual" means a human or non-human animal selected for treatment or therapy.

"Inhibiting expression of a C90RF72 antisense transcript" means reducing the level or expression of a C90RF72 antisense transcript and/or its expression products (e.g., RAN translation products). In certain embodiments, C90RF72 antisense transcripts are inhibited in the presence of an antisense compound targeting a C90RF72 antisense transcript, including an antisense oligonucleotide targeting a C90RF72 antisense transcript, as compared to expression of C90RF72 antisense transcript levels in the absence of a C90RF72 antisense compound, such as an antisense oligonucleotide.

"Inhibiting expression of a C90RF72 sense transcript" means reducing the level or expression of a C90RF72 sense transcript and/or its expression products (e.g., a C90RF72 mRNA and/or protein). In certain embodiments, C90RF72 sense transcripts are inhibited in the presence of an antisense compound targeting a C90RF72 sense transcript, including an antisense oligonucleotide targeting a C90RF72 sense transcript, as compared to expression of C90RF72 sense transcript levels in the absence of a C90RF72 antisense compound, such as an antisense oligonucleotide.

"Inhibiting the expression or activity" refers to a reduction or blockade of the expression or activity and does not necessarily indicate a total elimination of expression or activity.

"Internucleoside linkage" refers to the chemical bond between nucleosides.

"Linked nucleosides" means adjacent nucleosides linked together by an internucleoside linkage.

"Locked nucleic acid" or "LNA" or "LNA nucleosides" means nucleic acid monomers having a bridge connecting two carbon atoms between the 4' and 2'position of the nucleoside sugar unit, thereby forming a bicyclic sugar. Examples of such bicyclic sugar include, but are not limited to A) α-L-Methyleneoxy (4'-CH₂-O-2') LNA, (B) β-D-Methyleneoxy (4'-CH₂-O-2') LNA, (C) Ethyleneoxy (4'-(CH₂)₂-O-2') LNA, (D) Aminooxy (4'-CH₂-O-N(R)-2') LNA and (E) Oxyamino (4'-CH₂-N(R)-O-2') LNA, as depicted below.

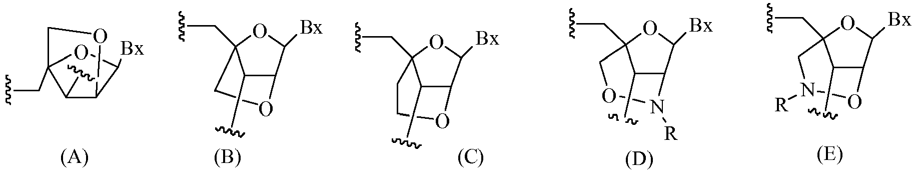

As used herein, LNA compounds include, but are not limited to, compounds having at least one bridge between the 4' and the 2' position of the sugar wherein each of the bridges independently comprises 1 or from 2 to 4 linked groups independently selected from -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- and -N(R₁)-; wherein: x is 0, 1, or 2; n is 1, 2, 3, or 4; each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, a heterocycle radical, a substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

Examples of 4'- 2' bridging groups encompassed within the definition of LNA include, but are not limited to one of formulae: -[C(R₁)(R₂)]ₙ-, -[C(R₁)(R₂)]ₙ-O-, -C(R₁R₂)-N(R₁)-O- or-C(R₁R₂)-O-N(R₁)-. Furthermore, other bridging groups encompassed with the definition of LNA are 4'-CH₂-2', 4'-(CH₂)₂-2', 4'-(CH₂)₃-2', 4'-CH₂-O-2', 4'-(CH₂)₂-O-2', 4'-CH₂-O-N(R₁)-2' and 4'-CH₂-N(R₁)-O-2'- bridges, wherein each R₁ and R₂ is, independently, H, a protecting group or C₁-C₁₂ alkyl.

Also included within the definition of LNA according to the invention are LNAs in which the 2'-hydroxyl group of the ribosyl sugar ring is connected to the 4' carbon atom of the sugar ring, thereby forming a methyleneoxy (4'-CH₂-O-2') bridge to form the bicyclic sugar moiety. The bridge can also be a methylene (-CH₂-) group connecting the 2' oxygen atom and the 4' carbon atom, for which the term methyleneoxy (4'-CH₂-O-2') LNA is used. Furthermore; in the case of the bicylic sugar moiety having an ethylene bridging group in this position, the term ethyleneoxy (4'-CH₂CH₂-O-2') LNA is used. α-L-methyleneoxy (4'-CH₂-O-2'), an isomer of methyleneoxy (4'-CH₂-O-2') LNA is also encompassed within the definition of LNA, as used herein.

"Mismatch" or "non-complementary nucleobase" refers to the case when a nucleobase of a first nucleic acid is not capable of pairing with the corresponding nucleobase of a second or target nucleic acid.

"Modified internucleoside linkage" refers to a substitution or any change from a naturally occurring internucleoside bond (*i.e.,* a phosphodiester internucleoside bond).

"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).

"Modified nucleoside" means a nucleoside having, independently, a modified sugar moiety and/or modified nucleobase.

"Modified nucleotide" means a nucleotide having, independently, a modified sugar moiety, modified internucleoside linkage, and/or modified nucleobase.

"Modified oligonucleotide" means an oligonucleotide comprising at least one modified internucleoside linkage, modified sugar, and/or modified nucleobase.

"Modified sugar" means substitution and/or any change from a natural sugar moiety.

"Monomer" means a single unit of an oligomer. Monomers include, but are not limited to, nucleosides and nucleotides, whether naturally occurring or modified.

"Motif' means the pattern of unmodified and modified nucleoside in an antisense compound.

"Natural sugar moiety" means a sugar moiety found in DNA (2'-H) or RNA (2'-OH).

"Naturally occurring internucleoside linkage" means a 3' to 5' phosphodiester linkage.

"Non-complementary nucleobase" refers to a pair of nucleobases that do not form hydrogen bonds withone another or otherwise support hybridization.

"Nucleic acid" refers to molecules composed of monomeric nucleotides. A nucleic acid includes, but is not limited to, ribonucleic acids (RNA), deoxyribonucleic acids (DNA), single-stranded nucleic acids, double-stranded nucleic acids, small interfering ribonucleic acids (siRNA), and microRNAs (miRNA).

"Nucleobase" means a heterocyclic moiety capable of pairing with a base of another nucleic acid.

"Nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

"Nucleobase sequence" means the order of contiguous nucleobases independent of any sugar, linkage, and/or nucleobase modification.

"Nucleoside" means a nucleobase linked to a sugar.

"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo, or tricyclo sugar mimetics, e.g., non furanose sugar units. Nucleotide mimetic includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage). Sugar surrogate overlaps with the slightly broader term nucleoside mimetic but is intended to indicate replacement of the sugar unit (furanose ring) only. The tetrahydropyranyl rings provided herein are illustrative of an example of a sugar surrogate wherein the furanose sugar group has been replaced with a tetrahydropyranyl ring system. "Mimetic" refers to groups that are substituted for a sugar, a nucleobase, and/or internucleoside linkage. Generally, a mimetic is used in place of the sugar or sugar-internucleoside linkage combination, and the nucleobase is maintained for hybridization to a selected target.

"Nucleotide" means a nucleoside having a phosphate group covalently linked to the sugar portion of the nucleoside.

"Off-target effect" refers to an unwanted or deleterious biological effect assocaited with modulation of RNA or protein expression of a gene other than the intended target nucleic acid.

"Oligomeric compound" or "oligomer" means a polymer of linked monomeric subunits which is capable of hybridizing to at least a region of a nucleic acid molecule.

"Oligonucleotide" means a polymer of linked nucleosides each of which can be modified or unmodified, independent one from another.

"Parenteral administration" means administration through injection (e.g., bolus injection) or infusion. Parenteral administration includes subcutaneous administration, intravenous administration, intramuscular administration, intraarterial administration, intraperitoneal administration, or intracranial administration, e.g., intrathecal or intracerebroventricular administration.

"Peptide" means a molecule formed by linking at least two amino acids by amide bonds. Without limitation, as used herein, peptide refers to polypeptides and proteins.

"Pharmaceutical agent" means a substance that provides a therapeutic benefit when administered to an individual. In certain embodiments, an antisense oligonucleotide targeted to C90RF72sense transcript is a pharmaceutical agent. In certain embodiments, an antisense oligonucleotide targeted to C90RF72antisense transcript is a pharmaceutical agent.

"Pharmaceutical composition" means a mixture of substances suitable for administering to as subject. For example, a pharmaceutical composition may comprise an antisense oliognucleotide and a sterile aqueous solution.

"Pharmaceutically acceptable derivative" encompasses pharmaceutically acceptable salts, conjugates, prodrugs or isomers of the compounds described herein.

"Pharmaceutically acceptable salts" means physiologically and pharmaceutically acceptable salts of antisense compounds, *i.e.,* salts that retain the desired biological activity of the parent oligonucleotide and do not impart undesired toxicological effects thereto.

"Phosphorothioate linkage" means a linkage between nucleosides where the phosphodiester bond is modified by replacing one of the non-bridging oxygen atoms with a sulfur atom. A phosphorothioate linkage is a modified internucleoside linkage.

"Portion" means a defined number of contiguous (*i.e.,* linked) nucleobases of a nucleic acid. In certain embodiments, a portion is a defined number of contiguous nucleobases of a target nucleic acid. In certain embodiments, a portion is a defined number of contiguous nucleobases of an antisense compound.

"Prevent" or "preventing" refers to delaying or forestalling the onset or development of a disease, disorder, or condition for a period of time from minutes to days, weeks to months, or indefinitely.

"Prodrug" means a therapeutic agent that is prepared in an inactive form that is converted to an active form within the body or cells thereof by the action of endogenous enzymes or other chemicals or conditions.

"Prophylactically effective amount" refers to an amount of a pharmaceutical agent that provides a prophylactic or preventative benefit to an animal.

"Region" is defined as a portion of the target nucleic acid having at least one identifiable structure, function, or characteristic.

"Ribonucleotide" means a nucleotide having a hydroxy at the 2' position of the sugar portion of the nucleotide. Ribonucleotides may be modified with any of a variety of substituents.

"Salts" mean a physiologically and pharmaceutically acceptable salts of antisense compounds, i.e., salts that retain the desired biological activity of the parent oligonucleotide and do not impart undesired toxicological effects thereto.

"Segments" are defined as smaller or sub-portions of regions within a target nucleic acid.

"Shortened" or "truncated" versions of antisense oligonucleotides taught herein have one, two or more nucleosides deleted.

"Side effects" means physiological responses attributable to a treatment other than desired effects. In certain embodiments, side effects include, without limitation, injection site reactions, liver function test abnormalities, renal function abnormalities, liver toxicity, renal toxicity, central nervous system abnormalities, and myopathies.

"Single-stranded oligonucleotide" means an oligonucleotide which is not hybridized to a complementary strand.

"Sites," as used herein, are defined as unique nucleobase positions within a target nucleic acid.

"Slows progression" means decrease in the development of the disease.

"Specifically hybridizable" refers to an antisense compound having a sufficient degree of complementarity between an antisense oligonucleotide and a target nucleic acid to induce a desired effect, while exhibiting minimal or no effects on non-target nucleic acids under conditions in which specific binding is desired, *i.e.,* under physiological conditions in the case of *in vivo* assays and therapeutic treatments.

"Stringent hybridization conditions" or "stringent conditions" refer to conditions under which an oligomeric compound will hybridize to its target sequence, but to a minimal number of other sequences.

"Subject" means a human or non-human animal selected for treatment or therapy.

"Targeting" or "targeted" means the process of design and selection of an antisense compound that will specifically hybridize to a target nucleic acid and induce a desired effect.

"Target nucleic acid," "target RNA," and "target RNA transcript" and "nucleic acid target" all mean a nucleic acid capable of being targeted by antisense compounds.

"Target region" means a portion of a target nucleic acid to which one or more antisense compounds is targeted.

"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.

"Therapeutically effective amount" means an amount of a pharmaceutical agent that provides a therapeutic benefit to an individual.

"Treat" or "treating" or "treatment" means administering a composition to effect an alteration or improvement of a disease or condition.

"Unmodified nucleobases" means the purine bases adenine (A) and guanine (G), and the pyrimidine bases (T), cytosine (C), and uracil (U).

"Unmodified nucleotide" means a nucleotide composed of naturally occuring nucleobases, sugar moieties, and internucleoside linkages. In certain embodiments, an unmodified nucleotide is an RNA nucleotide (*i.e.* β-D-ribonucleosides) or a DNA nucleotide (*i.e.* β-D-deoxyribonucleoside).

"Wing segment" means a plurality of nucleosides modified to impart to an oligonucleotide properties such as enhanced inhibitory activity, increased binding affinity for a target nucleic acid, or resistance to degradation by in vivo nucleases.

### Certain Embodiments

Described herein are compounds comprising a C9ORF72 antisense transcript specific inhibitor.

In certain embodiments, the C9ORF72 antisense transcript specific inhibitor is a C9ORF72 antisense transcript specific antisense compound.

In certain embodiments, the C9ORF72 antisense transcript specific antisense compound comprises an antisense oligonucleotide.

In certain embodiments, the antisense oligonucleotide consists of 12-30 linked nucleosides.

In certain embodiments, the antisense oligonucleotide consists of 16-25 linked nucleosides.

In certain embodiments, the antisense oligonucleotide consists of 18-22 linked nucleosides

In certain embodiments, the antisense oligonucleotide has a nucleobase sequence that is at least 80%, at least 85%, at least 90%, at least 91%, at least 92%, at least 93%, at least 94%, at least 95%, at least 96%, at least 97%, at least 98%, at least 99%, or 100% complementary to a C9ORF72 antisense transcript.

The antisense oligonucleotide of the invention has a nucleobase sequence that is at least 90% complementary to a C9ORF72 antisense transcript.

In certain embodiments, the antisense oligonucleotide has a nucleobase sequence that is at least 95% complementary to a C9ORF72 antisense transcript.

In certain embodiments, the antisense oligonucleotide has a nucleobase sequence that is 100% complementary to a C9ORF72 antisense transcript.

In certain embodiments, the C9ORF72 antisense transcript is SEQ ID NO: 11.

In certain embodiments, the antisense oligonucleotide has a nucleobase sequence comprising at least 8, at least 9, at least 10, at least 11, at least 12, at least 13, at least 14, at least 15, at least 16, at least 17, at least 18, at least 19, or 20 contiguous nucleobases of a sequence selected from among SEQ ID NO: 19-20, 22-26, 28-32, 34-42, 44, 46-55, 58-59, and 61.

In certain embodiments, the antisense oligonucleotide is a modified antisense oligonucleotide.

In certain embodiments, the modified antisense oligonucleotide comprises at least one modified internucleoside linkage.

In certain embodiments, the at least one modified internucleoside linkage is a phosphorothioate internucleoside linkage.

In certain embodiments, each modified internucleoside linkage is a phosphorothioate internucleoside linkage.

In certain embodiments, the modified antisense oligonucleotide comprises at least one phosphodiester internucleoside linkage.

In certain embodiments, the at least one nucleoside of the modified antisense oligonucleotide comprises a modified nucleobase.

In certain embodiments, the modified nucleobase is a 5-methylcytosine.

In certain embodiments, the at least one nucleoside of the modified antisense oligonucleotide comprises a modified sugar.

In certain embodiments, the at least one modified sugar is a bicyclic sugar.

In certain embodiments, the bicyclic sugar comprises a chemical bridge between the 2' and 4' position of the sugar, wherein the chemical bridge is selected from: 4'-CH₂-O-2'; 4'-CH(CH₃)-O-2'; 4'-(CH₂)₂-O-2'; and 4'-CH₂-N(R)-O-2' wherein R is, independently, H, C₁-C₁₂ alkyl, or a protecting group.

In certain embodiments, the at least one modified sugar comprises a 2'-O-methoxyethyl group.

In certain embodiments, the antisense oligonucleotide is a gapmer.

In certain embodiments, the C9ORF72 antisense transcript specific antisense compound comprises at least one conjugate.

In certain embodiments, the C9ORF72 antisense transcript specific antisense compound consists of an antisense oligonucleotide.

Provided herein are pharmaceutical compositions comprising a compound or modified oligonucleotide of the invention and a pharmaceutically acceptable diluent or carrier.

Described herein are pharmaceutical compositions comprising a C9ORF72 antisense transcript specific inhibitor.

Described herein are pharmaceutical compositions comprising a C9ORF72 antisense transcript specific inhibitor and a C9ORF sense transcript specific inhibitior.

In certain embodiments, the C9ORF72 sense transcript specific inhibitor is a C9ORF72 sense transcript specific antisense compound.

In certain embodiments, the C9ORF72 antisense transcript specific inhibitor is a C9ORF72 antisense transcript specific antisense compound.

In certain embodiments, the C9ORF72 sense transcript specific antisense compound is an antisense oligonucleotide.

In certain embodiments, the C9ORF72 antisense transcript specific antisense compound is an antisense oligonucleotide.

In certain embodiments, the sense transcript is any of SEQ ID NO: 1-10.

Described herein are uses of any compound described herein for the manufacture of a medicament for treating a neurodegenerative disease.

### Antisense Compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound may be "antisense" to a target nucleic acid, meaning that is is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain embodiments, an antisense compound targeted to a C9ORF72 nucleic acid is 12 to 30 subunits in length. In other words, such antisense compounds are from 12 to 30 linked subunits. In certain embodiments, the antisense compound is 8 to 80, 12 to 50, 15 to 30, 18 to 24, 19 to 22, or 20 linked subunits. In certain embodiments, the antisense compounds are 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked subunits in length, or a range defined by any two of the above values. In some embodiments the antisense compound is an antisense oligonucleotide, and the linked subunits are nucleosides.

In certain embodiments antisense oligonucleotides targeted to a C9ORF72 nucleic acid may be shortened or truncated. For example, a single subunit may be deleted from the 5' end (5' truncation), or alternatively from the 3' end (3' truncation). A shortened or truncated antisense compound targeted to a C9ORF72 nucleic acid may have two subunits deleted from the 5' end, or alternatively may have two subunits deleted from the 3' end, of the antisense compound. Alternatively, the deleted nucleosides may be dispersed throughout the antisense compound, for example, in an antisense compound having one nucleoside deleted from the 5' end and one nucleoside deleted from the 3' end.

When a single additional subunit is present in a lengthened antisense compound, the additional subunit may be located at the 5' or 3' end of the antisense compound. When two or more additional subunits are present, the added subunits may be adjacent to each other, for example, in an antisense compound having two subunits added to the 5' end (5' addition), or alternatively to the 3' end (3' addition), of the antisense compound. Alternatively, the added subunits may be dispersed throughout the antisense compound, for example, in an antisense compound having one subunit added to the 5' end and one subunit added to the 3' end.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL *in vitro* and *in vivo.* Furthermore, this oligonucleotide demonstrated potent anti-tumor activity *in vivo.*

Maher and Dolnick (Nuc. Acid. Res. 16:3341-3358,1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Antisense Compound Motifs

In certain embodiments, antisense compounds targeted to a C90RF72 nucleic acid have chemically modified subunits arranged in patterns, or motifs, to confer to the antisense compounds properties such as enhanced inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound may optionally serve as a substrate for the cellular endonuclease RNase H, which cleaves the RNA strand of an RNA:DNA duplex.

Antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer may in some embodiments include β-D-ribonucleosides, β-D-deoxyribonucleosides, 2'-modified nucleosides (such 2'-modified nucleosides may include 2'-MOE, and 2'-O-CH₃, among others), and bicyclic sugar modified nucleosides (such bicyclic sugar modified nucleosides may include those having a 4'-(CH₂)n-O-2' bridge, where n=1 or n=2 and 4'-CH₂-O-CH₂-2'). Preferably, each distinct region comprises uniform sugar moieties. The wing-gap-wing motif is frequently described as "X-Y-Z", where "X" represents the length of the 5' wing region, "Y" represents the length of the gap region, and "Z" represents the length of the 3' wing region. As used herein, a gapmer described as "X-Y-Z" has a configuration such that the gap segment is positioned immediately adjacent to each of the 5' wing segment and the 3' wing segment. Thus, no intervening nucleotides exist between the 5' wing segment and gap segment, or the gap segment and the 3' wing segment. Any of the antisense compounds described herein can have a gapmer motif. In some embodiments, X and Z are the same, in other embodiments they are different. In a preferred embodiment, Y is between 8 and 15 nucleotides. X, Y or Z can be any of 1, 2, 3, 4, 5, 6, 7, 8, 9, 10, 11, 12, 13, 14, 15, 16, 17, 18, 19, 20, 25, 30 or more nucleotides. Thus, gapmers described herein include, but are not limited to, for example 5-10-5, 5-10-4, 4-10-4, 4-10-3, 3-10-3, 2-10-2, 5-9-5, 5-9-4, 4-9-5, 5-8-5, 5-8-4, 4-8-5, 5-7-5, 4-7-5, 5-7-4, or 4-7-4.

In certain embodiments, the antisense compound has a "wingmer" motif, having a wing-gap or gap-wing configuration, i.e. an X-Y or Y-Z configuration as described above for the gapmer configuration. Thus, wingmer configurations described herein include, but are not limited to, for example 5-10, 8-4, 4-12, 12-4, 3-14, 16-2, 18-1, 10-3, 2-10, 1-10, 8-2, 2-13, 5-13, 5-8, or 6-8.

In certain embodiments, an antisense compound targeted to a C90RF72 nucleic acid has a gap-narrowed motif. In certain embodiments, a gap-narrowed antisense oligonucleotide targeted to a C90RF72 nucleic acid has a gap segment of 9, 8, 7, or 6 2'-deoxynucleotides positioned immediately adjacent to and between wing segments of 5, 4, 3, 2, or 1 chemically modified nucleosides. In certain embodiments, the chemical modification comprises a bicyclic sugar. In certain embodiments, the bicyclic sugar comprises a 4' to 2' bridge selected from among: 4'-(CH₂)ₙ-0-2' bridge, wherein n is 1 or 2; and 4'-CH₂-O-CH₂-2'. In certain embodiments, the bicyclic sugar is comprises a 4'-CH(CH₃)-O-2' bridge. In certain embodiments, the chemical modification comprises a non-bicyclic 2'-modified sugar moiety. In certain embodiments, the non-bicyclic 2'-modified sugar moiety comprises a 2'-O-methylethyl group or a 2'-O-methyl group.

### Target Nucleic Acids, Target Regions and Nucleotide Sequences

Nucleotide sequences that encode C90RF72 include, without limitation, the following: the complement of GENBANK Accession No. NM_001256054.1 (incorporated herein as SEQ ID NO: 1), GENBANK Accession No. NT_008413.18 truncated from nucleobase 27535000 to 27565000 (incorporated herein as SEQ ID NO: 2), GENBANK Accession No. BQ068108.1 (incorporated herein as SEQ ID NO: 3), GENBANK Accession No. NM_018325.3 (incorporated herein as SEQ ID NO: 4), GENBANK Accession No. DN993522.1 (incorporated herein as SEQ ID NO: 5), GENBANK Accession No. NM_145005.5 (incorporated herein as SEQ ID NO: 6), GENBANK Accession No. DB079375.1 (incorporated herein as SEQ ID NO: 7), GENBANK Accession No. BU194591.1 (incorporated herein as SEQ ID NO: 8), Sequence Identifier 4141_014_A (incorporated herein as SEQ ID NO: 9), and Sequence Identifier 4008_73_A (incorporated herein as SEQ ID NO: 10).

Nucleotide sequences that encode the C90RF72 antisense transcript include, without limitation, the following: SEQ ID NO: 11 is a sequence that is complementary to nucleotides 1159 to 1734 of SEQ ID NO: 2 (the complement of GENBANK Accession No. NT_008413.18 truncated from nucleotides 27535000 to 27565000).

It is understood that the sequence set forth in each SEQ ID NO in the Examples contained herein is independent of any modification to a sugar moiety, an internucleoside linkage, or a nucleobase. As such, antisense compounds defined by a SEQ ID NO may comprise, independently, one or more modifications to a sugar moiety, an internucleoside linkage, or a nucleobase. Antisense compounds described by Isis Number (Isis No) indicate a combination of nucleobase sequence and motif.

In certain embodiments, a target region is a structurally defined region of the target nucleic acid. For example, a target region may encompass a 3' UTR, a 5' UTR, an exon, an intron, an exon/intron junction, a coding region, a translation initiation region, translation termination region, or other defined nucleic acid region. The structurally defined regions for C9ORF72 can be obtained by accession number from sequence databases such as NCBI. In certain embodiments, a target region may encompass the sequence from a 5' target site of one target segment within the target region to a 3' target site of another target segment within the same target region.

Targeting includes determination of at least one target segment to which an antisense compound hybridizes, such that a desired effect occurs. In certain embodiments, the desired effect is a reduction in mRNA target nucleic acid levels. In certain embodiments, the desired effect is reduction of levels of protein encoded by the target nucleic acid or a phenotypic change associated with the target nucleic acid.

A target region may contain one or more target segments. Multiple target segments within a target region may be overlapping. Alternatively, they may be non-overlapping. In certain embodiments, target segments within a target region are separated by no more than about 300 nucleotides. In certain emodiments, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceeding values. In certain embodiments, target segments within a target region are separated by no more than, or no more than about, 5 nucleotides on the target nucleic acid. In certain embodiments, target segments are contiguous. Contemplated are target regions defined by a range having a starting nucleic acid that is any of the 5' target sites or 3' target sites listed herein.

Suitable target segments may be found within a 5' UTR, a coding region, a 3' UTR, an intron, an exon, or an exon/intron junction. Target segments containing a start codon or a stop codon are also suitable target segments. A suitable target segment may specifcally exclude a certain structurally defined region such as the start codon or stop codon.

The determination of suitable target segments may include a comparison of the sequence of a target nucleic acid to other sequences throughout the genome. For example, the BLAST algorithm may be used to identify regions of similarity amongst different nucleic acids. This comparison can prevent the selection of antisense compound sequences that may hybridize in a non-specific manner to sequences other than a selected target nucleic acid (i.e., non-target or off-target sequences).

There may be variation in activity (e.g., as defined by percent reduction of target nucleic acid levels) of the antisense compounds within a target region. In certain embodiments, reductions in C90RF72 mRNA levels are indicative of inhibition of C90RF72 expression. Reductions in levels of a C90RF72 protein are also indicative of inhibition of target mRNA expression. Reduction in the presence of expanded C90RF72 RNA foci are indicative of inhibition of C90RF72 epxression. Further, phenotypic changes are indicative of inhibition of C90RF72 expression. For example, improved motor function and respiration may be indicative of inhibition of C90RF72 expression.

### Hybridization

In some embodiments, hybridization occurs between an antisense compound disclosed herein and a C90RF72 nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art. In certain embodiments, the antisense compounds provided herein are specifically hybridizable with a C90RF72 nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as a C90RF72 nucleic acid).

Non-complementary nucleobases between an antisense compound and a C9ORF72 nucleic acid may be tolerated provided that the antisense compound remains able to specifically hybridize to a target nucleic acid. Moreover, an antisense compound may hybridize over one or more segments of a C9ORF72 nucleic acid such that intervening or adjacent segments are not involved in the hybridization event (e.g., a loop structure, mismatch or hairpin structure).

The antisense compounds described herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a C9ORF72 nucleic acid, a target region, target segment, or specified portion thereof. Percent complementarity of an antisense compound with a target nucleic acid can be determined using routine methods.

For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds provided herein, or specified portions thereof, are fully complementary (*i.e.,* 100% complementary) to a target nucleic acid, or specified portion thereof. For example, an antisense compound may be fully complementary to a C9ORF72 nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e., linked) or non-contiguous. In one embodiment, a non-complementary nucleobase is located in the wing segment of a gapmer antisense oligonucleotide.

In certain embodiments, antisense compounds that are, or are up to 12, 13, 14, 15, 16, 17, 18, 19, or 20 nucleobases in length comprise no more than 4, no more than 3, no more than 2, or no more than 1 non-complementary nucleobase(s) relative to a target nucleic acid, such as a C90RF72 nucleic acid, or specified portion thereof.

In certain embodiments, antisense compounds that are, or are up to 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, such as a C90RF72 nucleic acid, or specified portion thereof.

The antisense compounds provided herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain embodiments, the antisense compounds, are complementary to at least an 8 nucleobase portion of a target segment. In certain embodiments, the antisense compounds are complementary to at least a 9 nucleobase portion of a target segment. In certain embodiments, the antisense compounds are complementary to at least a 10 nucleobase portion of a target segment. In certain embodiments, the antisense compounds, are complementary to at least an 11 nucleobase portion of a target segment. In certain embodiments, the antisense compounds, are complementary to at least a 12 nucleobase portion of a target segment. In certain embodiments, the antisense compounds, are complementary to at least a 13 nucleobase portion of a target segment. In certain embodiments, the antisense compounds, are complementary to at least a 14 nucleobase portion of a target segment. In certain embodiments, the antisense compounds, are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identity

The antisense compounds provided herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds provided herein also are contemplated. The non-identical bases may be adjacent to each other or dispersed throughout the antisense compound. Percent identity of an antisense compound is calculated according to the number of bases that have identical base pairing relative to the sequence to which it is being compared.

In certain embodiments, the antisense compounds, or portions thereof, are at least 70%, 75%, 80%, 85%, 90%, 95%, 96%, 97%, 98%, 99% or 100% identical to one or more of the antisense compounds or SEQ ID NOs, or a portion thereof, disclosed herein.

In certain embodiments, a portion of the antisense compound is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

In certain embodiments, a portion of the antisense oligonucleotide is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a C90RF72 nucleic acid comprise one or more modified internucleoside linkages. In certain embodiments, the modified internucleoside linkages are interspersed throughout the antisense compound. In certain embodiments, the modified internucleoside linkages are phosphorothioate linkages. In certain embodiments, each internucleoside linkage of an antisense compound is a phosphorothioate internucleoside linkage. In certain embodiments, the antisense compounds targeted to a C90RF72 nucleic acid comprise at least one phosphodiester linkage and at least one phosphorothioate linkage.

### Modified Sugar Moieties

Antisense compounds can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (*R* or *S*), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each Rₗ, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include without limitation nucleosides comprising a bridge between the 4' and the 2' ribosyl ring atoms. In certain embodiments, antisense compounds provided herein include one or more bicyclic nucleosides comprising a 4' to 2' bridge. Examples of such 4' to 2' bridged bicyclic nucleosides, include but are not limited to one of the formulae: 4'-(CH₂)-O-2' (LNA); 4'-(CH₂)-S-2'; 4'-(CH₂)₂-O-2' (ENA); 4'-CH(CH₃)-O-2' and 4'-CH(CH₂OCH₃)-0-2' (and analogs thereof see U.S. Patent 7,399,845, issued on July 15, 2008); 4'-C(CH₃)(CH₃)-O-2' (and analogs thereof see published International Application WO/2009/006478, published January 8, 2009); 4'-CH₂-N(OCH₃)-2' (and analogs thereof see published International Application WO/2008/150729, published December 11, 2008); 4'-CH₂-O-N(CH₃)-2' (see published U.S. Patent Application US2004-0171570, published September 2, 2004); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C-(=CH₂)-2' (and analogs thereof see published International Application WO 2008/154401, published on December 8, 2008).

Further reports related to bicyclic nucleosides can also be found in published literature (see for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 2007, 129(26) 8362-8379; Elayadi et al., Curr. Opinion Invest. Drugs, 2001, 2, 558-561; Braasch et al., Chem. Biol., 2001, 8, 1-7; and Orum et al., Curr. Opinion Mol. Ther., 2001, 3, 239-243; U.S. Patent Nos. 6,268,490; 6,525,191; 6,670,461; 6,770,748; 6,794,499; 7,034,133; 7,053,207; 7,399,845; 7,547,684; and 7,696,345; U.S. Patent Publication No. US2008-0039618; US2009-0012281; U.S. Patent Serial Nos. 60/989,574; 61/026,995; 61/026,998; 61/056,564; 61/086,231; 61/097,787; and 61/099,844; Published PCT International applications WO 1994/014226; WO 2004/106356; WO 2005/021570; WO 2007/134181; WO 2008/150729; WO 2008/154401; and WO 2009/006478. Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

In certain embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from -[C(Rₐ)(R_{b})]ₙ-, -C(Rₐ)=C(R_{b})-, -C(Rₐ)=N-, -C(=O)-, -C(=NRₐ)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain embodiments, the bridge of a bicyclic sugar moiety is -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each R is, independently, H, a protecting group or C₁-C₁₂ alkyl.

In certain embodiments, bicyclic nucleosides are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') BNA's have been incorporated into antisense oligonucleotides that showed antisense activity (Frieden et al., Nucleic Acids Research, 2003, 21, 6365-6372).

In certain embodiments, bicyclic nucleosides include, but are not limited to, (A) α-L-methyleneoxy (4'-CH₂-O-2') BNA , (B) β-D-methyleneoxy (4'-CH₂-O-2') BNA, (C) ethyleneoxy (4'-(CH₂)₂-O-2') BNA , (D) aminooxy (4'-CH₂-O-N(R)-2') BNA, (E) oxyamino (4'-CH₂-N(R)-O-2') BNA, and (F) methyl(methyleneoxy) (4'-CH(CH₃)-O-2') BNA, (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, and (J) propylene carbocyclic (4'-(CH₂)₃-2') BNA as depicted below. 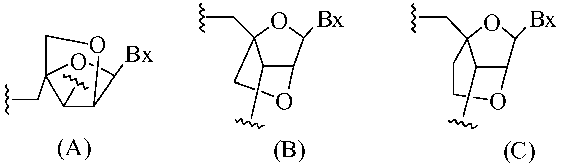 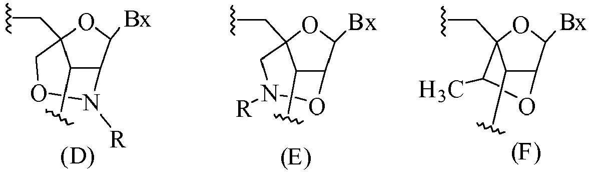 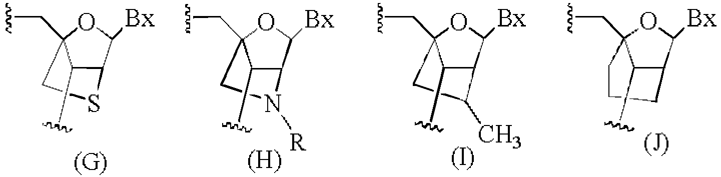 wherein Bx is the base moiety and R is independently H, a protecting group or C₁-C₁₂ alkyl.

In certain embodiments, bicyclic nucleosides are provided having Formula I: 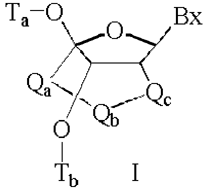 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or-N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides are provided having Formula II: 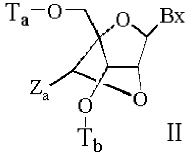 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thio.

In one embodiment, each of the substituted groups is, independently, mono or poly substituted with substituent groups independently selected from halogen, oxo, hydroxyl, OJ_{c}, NJ_{c}J_{d}, SJ_{c}, N₃, OC(=X)J_{c}, and NJₑC(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides are provided having Formula III: 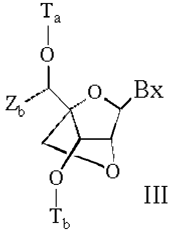 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides are provided having Formula IV: 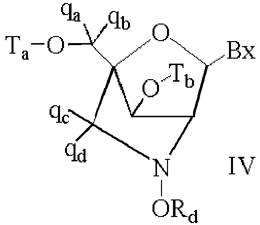 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;

In certain embodiments, bicyclic nucleosides are provided having Formula V: 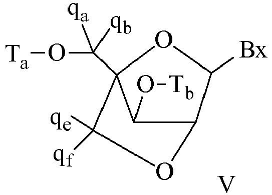 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (Wengel et al., WO 99/14226). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides are provided having Formula VI: 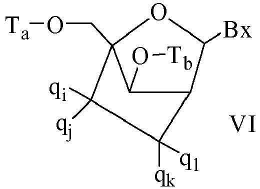 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and qₗ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qᵢ and qⱼ or qₗ and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Freier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc., 2007, 129(26), 8362-8379).

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

As used herein, "monocylic nucleosides" refer to nucleosides comprising modified sugar moieties that are not bicyclic sugar moieties. In certain embodiments, the sugar moiety, or sugar moiety analogue, of a nucleoside may be modified or substituted at any position.

As used herein, "2'-modified sugar" means a furanosyl sugar modified at the 2' position. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₙO]ₘCH₃, O(CH₂)ₙNH₂, O(CH₂)ₙCH₃, O(CH₂)ₙF, O(CH₂)ₙONH₂, OCH₂C(=O)N(H)CH₃, and O(CH₂)ₙON[(CH₂)ₙCH₃]₂, where n and m are from 1 to about 10. Other 2'- substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain embodiments, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'- *O*-methyl, *O*-propyl, and *O*-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854), fluoro HNA (F-HNA) or those compounds having Formula VII: 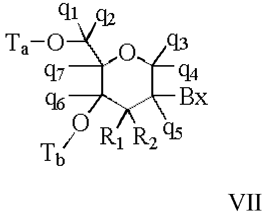 wherein independently for each of said at least one tetrahydropyran nucleoside analog of Formula VII:
Bx is a heterocyclic base moiety;
Tₐ and T_{b} are each, independently, an internucleoside linking group linking the tetrahydropyran nucleoside analog to the antisense compound or one of Tₐ and T_{b} is an internucleoside linking group linking the tetrahydropyran nucleoside analog to the antisense compound and the other of Tₐ and T_{b} is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and each of R₁ and R₂ is selected from hydrogen, hydroxyl, halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is fluoro. In certain embodiments, R₁ is fluoro and R₂ is H; R₁ is methoxy and R₂ is H, and R₁ is H and R₂ is methoxyethoxy.

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position.

As used herein, "2'-OMe" or "2'-OCH₃" or "2'-O-methyl" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

As used herein, "oligonucleotide" refers to a compound comprising a plurality of linked nucleosides. In certain embodiments, one or more of the plurality of nucleosides is modified. In certain embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds (see for example review article: Leumann, Bioorg. Med. Chem., 2002, 10, 841-854).
Such ring systems can undergo various additional substitutions to enhance activity.

Methods for the preparations of modified sugars are well known to those skilled in the art.

In nucleotides having modified sugar moieties, the nucleobase moieties (natural, modified or a combination thereof) are maintained for hybridization with an appropriate nucleic acid target.

In certain embodiments, antisense compounds comprise one or more nucleosides having modified sugar moieties. In certain embodiments, the modified sugar moiety is 2'-MOE. In certain embodiments, the 2'-MOE modified nucleosides are arranged in a gapmer motif. In certain embodiments, the modified sugar moiety is a bicyclic nucleoside having a (4'-CH(CH₃)-O-2') bridging group. In certain embodiments, the (4'-CH(CH₃)-O-2') modified nucleosides are arranged throughout the wings of a gapmer motif.

### Compositions and Methods for Formulating Pharmaceutical Compositions

Antisense oligonucleotides may be admixed with pharmaceutically acceptable active or inert substances for the preparation of pharmaceutical compositions or formulations. Compositions and methods for the formulation of pharmaceutical compositions are dependent upon a number of criteria, including, but not limited to, route of administration, extent of disease, or dose to be administered.

An antisense compound targeted to a C90RF72 nucleic acid can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound targeted to a C90RF72 nucleic acid and a pharmaceutically acceptable diluent. In certain embodiments, the pharmaceutically acceptable diluent is PBS. In certain embodiments, the antisense compound is an antisense oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an antisense compound which are cleaved by endogenous nucleases within the body, to form the active antisense compound.

### Conjugated Antisense Compounds

Antisense compounds may be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of C90RF72 nucleic acids can be tested *in vitro* in a variety of cell types. Cell types used for such analyses are available from commerical vendors (*e.g*. American Type Culture Collection, Manassus, VA; Zen-Bio, Inc., Research Triangle Park, NC; Clonetics Corporation, Walkersville, MD) and are cultured according to the vendor's instructions using commercially available reagents (e.g. Invitrogen Life Technologies, Carlsbad, CA). Illustrative cell types include, but are not limited to, HepG2 cells, Hep3B cells, and primary hepatocytes.

### In vitro testing of antisense oligonucleotides

Described herein are methods for treatment of cells with antisense oligonucleotides, which can be modified appropriately for treatment with other antisense compounds.

In general, cells are treated with antisense oligonucleotides when the cells reach approximately 60-80% confluency in culture.

One reagent commonly used to introduce antisense oligonucleotides into cultured cells includes the cationic lipid transfection reagent LIPOFECTIN (Invitrogen, Carlsbad, CA). Antisense oligonucleotides are mixed with LIPOFECTIN in OPTI-MEM 1 (Invitrogen, Carlsbad, CA) to achieve the desired final concentration of antisense oligonucleotide and a LIPOFECTIN concentration that typically ranges 2 to 12 ug/mL per 100 nM antisense oligonucleotide.

Another reagent used to introduce antisense oligonucleotides into cultured cells includes LIPOFECTAMINE (Invitrogen, Carlsbad, CA). Antisense oligonucleotide is mixed with LIPOFECTAMINE in OPTI-MEM 1 reduced serum medium (Invitrogen, Carlsbad, CA) to achieve the desired concentration of antisense oligonucleotide and a LIPOFECTAMINE concentration that typically ranges 2 to 12 ug/mL per 100 nM antisense oligonucleotide.

Another technique used to introduce antisense oligonucleotides into cultured cells includes electroporation.

Cells are treated with antisense oligonucleotides by routine methods. Cells are typically harvested 16-24 hours after antisense oligonucleotide treatment, at which time RNA or protein levels of target nucleic acids are measured by methods known in the art and described herein. In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art. Antisense oligonucleotides are typically used at concentrations ranging from 1 nM to 300 nM when transfected with LIPOFECTAMINE. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art. RNA is prepared using methods well known in the art, for example, using the TRIZOL Reagent (Invitrogen, Carlsbad, CA) according to the manufacturer's recommended protocols.

### Analysis of inhibition of target levels or expression

Inhibition of levels or expression of a C90RF72 nucleic acid can be assayed in a variety of ways known in the art. For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels may be accomplished by quantitative real-time PCR using the ABI PRISM 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents are obtained from Invitrogen (Carlsbad, CA). RT real-time-PCR reactions are carried out by methods well known to those skilled in the art.

Gene (or RNA) target quantities obtained by real time PCR are normalized using either the expression level of a gene whose expression is constant, such as cyclophilin A, or by quantifying total RNA using RIBOGREEN (Invitrogen, Inc. Carlsbad, CA). Cyclophilin A expression is quantified by real time PCR, by being run simultaneously with the target, multiplexing, or separately. Total RNA is quantified using RIBOGREEN RNA quantification reagent (Invetrogen, Inc. Eugene, OR). Methods of RNA quantification by RIBOGREEN are taught in Jones, L.J., et al, (Analytical Biochemistry, 1998, 265, 368-374). A CYTOFLUOR 4000 instrument (PE Applied Biosystems) is used to measure RIBOGREEN fluorescence.

Probes and primers are designed to hybridize to a C90RF72 nucleic acid. Methods for designing real-time PCR probes and primers are well known in the art, and may include the use of software such as PRIMER EXPRESS Software (Applied Biosystems, Foster City, CA).

### Strand Specific Semi-Quantitative PCR Analysis of Target RNA Levels

Analysis of specific, low abundance target RNA strand levels may be accomplished by reverse transcription, PCR, and gel densitometry analysis using the Gel Logic 200 Imaging System and Kodak MI software (Kodak Scientific Imaging Systems, Rochester, NY, USA) according to manufacturer's instructions.

RT-PCR reactions are carried out as taught in Ladd, P.D., et al, (Human Molecular Genetics, 2007, 16, 3174-3187) and in Sopher, B.L., et al, (Neuron, 2011, 70, 1071-1084) and such methods are well known in the art.

The PCR amplification products are loaded onto gels, stained with ethidium bromide, and subjected to densitometry analysis. Mean intensities from regions of interest (ROI) that correspond to the bands of interest in the gel are measured.

Gene (or RNA) target quantities obtained by PCR are normalized using the expression level of a housekeeping gene whose expression is constant, such as GAPDH. Expression of the housekeeping gene (or RNA) is analyzed and measured using the same methods as the target.

Probes and primers are designed to hybridize to a C90RF72 nucleic acid. Methods for designing RT-PCR probes and primers are well known in the art, and may include the use of software such as PRIMER EXPRESS Software (Applied Biosystems, Foster City, CA).

### Analysis of Protein Levels

Antisense inhibition of C90RF72 nucleic acids can be assessed by measuring C90RF72 protein levels. Protein levels of C90RF72 can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art. Antibodies useful for the detection of mouse, rat, monkey, and human C90RF72 are commercially available.

### In vivo testing of antisense compounds

Antisense compounds, for example, antisense oligonucleotides, are tested in animals to assess their ability to inhibit expression of C90RF72 and produce phenotypic changes, such as, improved motor function and respiration. In certain embodiments, motor function is measured by rotarod, grip strength, pole climb, open field performance, balance beam, hindpaw footprint testing in the animal. In certain embodiments, respiration is measured by whole body plethysmograph, invasive resistance, and compliance measurements in the animal. Testing may be performed in normal animals, or in experimental disease models. For administration to animals, antisense oligonucleotides are formulated in a pharmaceutically acceptable diluent, such as phosphate-buffered saline. Administration includes parenteral routes of administration, such as intraperitoneal, intravenous, and subcutaneous. Calculation of antisense oligonucleotide dosage and dosing frequency is within the abilities of those skilled in the art, and depends upon factors such as route of administration and animal body weight. Following a period of treatment with antisense oligonucleotides, RNA is isolated from CNS tissue or CSF and changes in C9ORF72 nucleic acid expression are measured.

### Targeting C9ORF72

Antisense oligonucleotides described herein may hybridize to a C9ORF72 nucleic acid derived from either DNA strand. For example, antisense oligonucleotides described herein may hybridize to a C9ORF72 antisense transcript or a C9ORF72 sense transcript. Antisense oligonucleotides described herein may hybridize to a C9ORF72 nucleic acid in any stage of RNA processing. Described herein are antisense oligonucleotides that are complementary to a pre-mRNA or a mature mRNA. Additionally, antisense oligonucleotides described herein may hybridize to any element of a C9ORF72 nucleic acid. For example, described herein are antisense oligonucleotides that are complementary to an exon, an intron, the 5' UTR, the 3' UTR, a repeat region, a hexanucleotide repeat expansion, a splice junction, an exon:exon splice junction, an exonic splicing silencer (ESS), an exonic splicing enhancer (ESE), exon la, exon lb, exon 1c, exon 1d, exon le, exon 2, exon 3, exon 4, exon 5, exon 6, exon 7, exon 8, exon 9, exon 10, exon11, intron 1, intron 2, intron 3, intron 4, intron 5, intron 6, intron 7, intron 8, intron 9, or intron 10 of a C9ORF72 nucleic acid.

In certain embodiments of the disclosure, antisense oligonucleotides described herein hybridize to all variants of C9ORF72 derived from the sense strand. In certain embodiments of the disclosure, the antisense oligonucleotides described herein selectively hybridize to certain variants of C9ORF72 derived from the sense strand. In certain embodiments of the disclosure, the antisense oligonucleotides described herein selectively hybridize to variants of C9ORF72 derived from the sense strand containing a hexanucleotide repeat expansion. In certain embodiments of the disclosure, the antisense oliognucleotides described herein selectively hybridize to pre-mRNA variants containing a hexanucleotide repeat. In certain embodiments of the disclosure, pre-mRNA variants of C9ORF72 containing a hexanucleotide repeat expansion include SEQ ID NO: 1-3 and 6-10. In certain embodiments of the disclosure, such hexanucleotide repeat expansion comprises at least 24 repeats of any of GGGGCC, GGGGGG, GGGGGC, or GGGGCG.

In certain embodiments of the disclosure, the antisense oligonucleotides described herein inhibit expression of all variants of C9ORF72 derived from the sense strand. In certain embodiments of the disclosure, the antisense oligonucleotides described herein inhibit expression of all variants of C9ORF72 derived from the sense strand equally. In certain embodiments of the disclosure, the antisense oligonucleotides described herein preferentially inhibit expression of one or more variants of C9ORF72 derived from the sense strand. In certain embodiments of the disclosure, the antisense oligonucleotides described herein preferentially inhibit expression of variants of C9ORF72 derived from the sense strand containing a hexanucleotide repeat expansion. In certain embodiments of the disclosure, the antisense oliognucleotides described herein selectively inhibit expression of pre-mRNA variants containing the hexanucleotide repeat. In certain embodiments of the disclosure, the antisense oliognucleotides described herein selectively inhibit expression of C9ORF72 pathogenic associated mRNA variants. In certain embodiments of the disclosure, pre-mRNA variants of C9ORF72 containing a hexanucleotide repeat expansion include SEQ ID NO: 1-3 and 6-10. In certain embodiments of the disclosure, such hexanucleotide repeat expansion comprises at least 24 repeats of any of GGGGCC, GGGGGG, GGGGGC, or GGGGCG. In certain embodiments of the disclosure, the hexanucleotide repeat expansion forms C9ORF72 sense foci. In certain embodiments of the disclosure, antisense oligonucleotides described herein are useful for reducing C9ORF72 sense foci. C9ORF72 sense foci may be reduced in terms of percent of cells with foci as well as number of foci per cell.

### C9OFF72 Features

Antisense oligonucleotides described herein may hybridize to any C9ORF72 nucleic acid at any state of processing within any element of the C9ORF72 gene. In certain embodiments, antisense oligonucleotides described herein may target the antisense transcript, e.g., SEQ ID NO: 11. In certain embodiments, antisense oligonucleotides described herein may hybridize to an exon, an intron, the 5' UTR, the 3' UTR, a repeat region, a hexanucleotide repeat expansion, a splice junction, an exon:exon splice junction, an exonic splicing silencer (ESS), an exonic splicing enhancer (ESE), exon 1a, exon 1b, exon 1c, exon 1d, exon 1e, exon 2, exon 3, exon 4, exon 5, exon 6, exon 7, exon 8, exon 9, exon 10, exon 11, intron 1, intron 2, intron 3, intron 4, intron 5, intron 6, intron 7, intron 8, intron 9, or intron 10. For example, antisense oligonucleotides may target any of the exons characterized below in Tables 1-5 described below. Antisense oligonucleotides described herein may also target nucleic acids not characterized below and such nucleic acid may be characterized in GENBANK. Moreover, antisense oligonucleotides described herein may also target elements other than exons and such elements as characterized in GENBANK.

**Table 1**

| Functional Segments for NM_001256054.1 (SEQ ID NO: 1) | | | | |
|---|---|---|---|---|
| **Exon Number** | **mRNA start site** | **mRNA stop site** | **Start site in reference to SEQ ID NO: 2** | **Stop site in reference to SEQ ID NO: 2** |
| exon 1C | 1 | 158 | 1137 | 1294 |
| exon 2 | 159 | 646 | 7839 | 8326 |
| exon 3 | 647 | 706 | 9413 | 9472 |
| exon 4 | 707 | 802 | 12527 | 12622 |
| exon 5 | 803 | 867 | 13354 | 13418 |
| exon 6 | 868 | 940 | 14704 | 14776 |
| exon 7 | 941 | 1057 | 16396 | 16512 |
| exon 8 | 1058 | 1293 | 18207 | 18442 |
| exon 9 | 1294 | 1351 | 24296 | 24353 |
| exon 10 | 1352 | 1461 | 26337 | 26446 |
| exon 11 | 1462 | 3339 | 26581 | 28458 |

**Table 2**

| Functional Segments for NM_018325.3 (SEQ ID NO: 4) | | | | |
|---|---|---|---|---|
| **Exon Number** | **mRNA start site** | **mRNA stop site** | **Start site in reference to SEQ ID NO: 2** | **Stop site in reference to SEQ ID NO: 2** |
| exon 1B | 1 | 63 | 1510 | 1572 |
| exon 2 | 64 | 551 | 7839 | 8326 |
| exon 3 | 552 | 611 | 9413 | 9472 |
| exon 4 | 612 | 707 | 12527 | 12622 |
| exon 5 | 708 | 772 | 13354 | 13418 |
| exon 6 | 773 | 845 | 14704 | 14776 |
| exon 7 | 846 | 962 | 16396 | 16512 |
| exon 8 | 963 | 1198 | 18207 | 18442 |
| exon 9 | 1199 | 1256 | 24296 | 24353 |
| exon 10 | 1257 | 1366 | 26337 | 26446 |
| exon 11 | 1367 | 3244 | 26581 | 28458 |

**Table 3**

| Functional Segments for NM_145005.5 (SEQ ID NO: 6) | | | | |
|---|---|---|---|---|
| **Exon Number** | **mRNA start site** | **mRNA stop site** | **Start site in reference to SEQ ID NO: 2** | **Stop site in reference to SEQ ID NO: 2** |
| exon 1A | 1 | 80 | 1137 | 1216 |
| exon 2 | 81 | 568 | 7839 | 8326 |
| exon 3 | 569 | 628 | 9413 | 9472 |
| exon 4 | 629 | 724 | 12527 | 12622 |
| exon 5B (exon 5 into intron 5) | 725 | 1871 | 13354 | 14500 |

**Table 4**

| Functional Segments for DB079375.1 (SEQ ID NO: 7) | | | | |
|---|---|---|---|---|
| **Exon Number** | **mRNA start site** | **mRNA stop site** | **Start site in reference to SEQ ID NO: 2** | **Stop site in reference to SEQ ID NO: 2** |
| exon IE | 1 | 35 | 1135 | 1169 |
| exon 2 | 36 | 524 | 7839 | 8326 |
| exon 3 (EST ends before end of full exon) | 525 | 562 | 9413 | 9450 |

**Table 5**

| Functional Segments for BU194591.1 (SEQ ID NO: 8) | | | | |
|---|---|---|---|---|
| **Exon Number** | **mRNA start site** | **mRNA stop site** | **Start site in reference to SEQ ID NO: 2** | **Stop site in reference to SEQ ID NO: 2** |
| exon ID | 1 | 36 | 1241 | 1279 |
| exon 2 | 37 | 524 | 7839 | 8326 |
| exon 3 | 525 | 584 | 9413 | 9472 |
| exon 4 | 585 | 680 | 12527 | 12622 |
| exon 5B (exon 5 into intron 5) | 681 | 798 | 13354 | 13465 |

### Certain Indications

Described herein are methods of treating an individual comprising administering one or more pharmaceutical compositions described herein. In certain embodiments, the individual has a neurodegenerative disease. In certain embodiments, the individual is at risk for developing a neurodegenerative disease, including, but not limited to, ALS or FTD. In certain embodiments, the individual has been identified as having a C9ORF72 associated disease. In certain embodiments, the individual has been identified as having a C9ORF72 hexanucleotide repeat expansion associated disease. Described herein are methods for prophylactically reducing C9ORF72 expression in an individual. Certain embodiments include treating an individual in need thereof by administering to an individual a therapeutically effective amount of an antisense compound targeted to a C9ORF72 nucleic acid.

In one embodiment, administration of a therapeutically effective amount of an antisense compound targeted to a C9ORF72 nucleic acid is accompanied by monitoring of C9ORF72 levels in an individual, to determine an individual's response to administration of the antisense compound. An individual's response to administration of the antisense compound may be used by a physician to determine the amount and duration of therapeutic intervention.

In certain embodiments, administration of an antisense compound targeted to a C9ORF72 nucleic acid results in reduction of C9ORF72 expression by at least 15, 20, 25, 30, 35, 40, 45, 50, 55, 60, 65, 70, 75, 80, 85, 90, 95 or 99%, or a range defined by any two of these values. In certain embodiments, administration of an antisense compound targeted to a C9ORF72 nucleic acid results in improved motor function and respiration in an animal. In certain embodiments, administration of a C9ORF72 antisense compound improves motor function and respiration by at least 15, 20, 25, 30, 35, 40, 45, 50, 55, 60, 65, 70, 75, 80, 85, 90, 95 or 99%, or a range defined by any two of these values.

In certain embodiments, administration of an antisense compound targeted to a C9ORF72 antisense transcript results in reduction of C9ORF72 antisense transcript expression by at least 15, 20, 25, 30, 35, 40, 45, 50, 55, 60, 65, 70, 75, 80, 85, 90, 95 or 99%, or a range defined by any two of these values. In certain embodiments, administration of an antisense compound targeted to a C9ORF72 antisense transcript results in improved motor function and respiration in an animal. In certain embodiments, administration of a C9ORF72 antisense compound improves motor function and respiration by at least 15, 20, 25, 30, 35, 40, 45, 50, 55, 60, 65, 70, 75, 80, 85, 90, 95 or 99%, or a range defined by any two of these values. In certain embodiments, administration of a C9ORF72 antisense compound reduces the number of cells with C9ORF72 antisense foci and/or the number of C9ORF72 antisense foci per cell.

In certain embodiments of the disclosure, administration of an antisense compound targeted to a C9ORF72 sense transcript results in reduction of a C9ORF72 sense transcript expression by at least 15, 20, 25, 30, 35, 40, 45, 50, 55, 60, 65, 70, 75, 80, 85, 90, 95 or 99%, or a range defined by any two of these values. In certain embodiments of the disclosure, administration of an antisense compound targeted to a C9ORF72 sense transcript results in improved motor function and respiration in an animal. In certain embodiments of the disclosure, administration of a C9ORF72 antisense compound improves motor function and respiration by at least 15, 20,25, 30, 35, 40, 45, 50, 55, 60, 65, 70, 75, 80, 85, 90, 95 or 99%, or a range defined by any two of these values. In certain embodiments of the disclosure, administration of a C9ORF72 antisense compound reduces the number of cells with C9ORF72 sense foci and/or the number of C9ORF72 sense foci per cell.

In certain embodiments, pharmaceutical compositions comprising an antisense compound targeted to a C9ORF72 nucleic are used for the preparation of a medicament for treating a patient suffering or susceptible to a neurodegenerative disease including ALS and FTD.

### Certain Combination Therapies

In certain embodiments, one or more pharmaceutical compositions described herein are co-administered with one or more other pharmaceutical agents. In certain embodiments, such one or more other pharmaceutical agents are designed to treat the same disease, disorder, or condition as the one or more pharmaceutical compositions described herein. In certain embodiments, such one or more other pharmaceutical agents are designed to treat a different disease, disorder, or condition as the one or more pharmaceutical compositions described herein. In certain embodiments, such one or more other pharmaceutical agents are designed to treat an undesired side effect of one or more pharmaceutical compositions described herein. In certain embodiments, one or more pharmaceutical compositions described herein are co-administered with another pharmaceutical agent to treat an undesired effect of that other pharmaceutical agent. In certain embodiments, one or more pharmaceutical compositions described herein are co-administered with another pharmaceutical agent to produce a combinational effect. In certain embodiments, one or more pharmaceutical compositions described herein are co-administered with another pharmaceutical agent to produce a synergistic effect.

In certain embodiments, one or more pharmaceutical compositions described herein and one or more other pharmaceutical agents are administered at the same time. In certain embodiments, one or more pharmaceutical compositions described herein and one or more other pharmaceutical agents are administered at different times. In certain embodiments, one or more pharmaceutical compositions described herein and one or more other pharmaceutical agents are prepared together in a single formulation. In certain embodiments, one or more pharmaceutical compositions described herein and one or more other pharmaceutical agents are prepared separately.

In certain embodiments, pharmaceutical agents that may be co-administered with a pharmaceutical composition described herein include Riluzole (Rilutek), Lioresal (Lioresal), and Dexpramipexole.

In certain embodiments, pharmaceutical agents that may be co-administered with a C90RF72 antisense transcript specific inhibitor described herein include, but are not limited to, an additional C90RF72 inhibitor. In certain embodiments, the co-adminstered pharmaceutical agent is administered prior to administration of a pharmaceutical composition described herein. In certain embodiments, the co-administered pharmaceutical agent is administered following administration of a pharmaceutical composition described herein. In certain embodiments the co-administered pharmaceutical agent is administered at the same time as a pharmaceutical composition described herein. In certain embodiments the dose of a co-administered pharmaceutical agent is the same as the dose that would be administered if the co-administered pharmaceutical agent was administered alone. In certain embodiments the dose of a co-administered pharmaceutical agent is lower than the dose that would be administered if the co-administered pharmaceutical agent was administered alone. In certain embodiments the dose of a co-administered pharmaceutical agent is greater than the dose that would be administered if the co-administered pharmaceutical agent was administered alone.

In certain embodiments, the co-administration of a second compound enhances the effect of a first compound, such that co-administration of the compounds results in an effect that is greater than the effect of administering the first compound alone. In other embodiments, the co-administration results in effects that are additive of the effects of the compounds when administered alone. In certain embodiments, the co-administration results in effects that are supra-additive of the effects of the compounds when administered alone. In certain embodiments, the first compound is an antisense compound. In certain embodiments, the second compound is an antisense compound.

### Certain Human Therapeutics

The human C90RF72 antisense transcript specific antisense compounds described herein are being evaluated as possible human therapeutics. Various parameters of potency, efficacy, and/or tolerability are being examined. Such parameters include in vitro inhibition of C9ORF72 antisense transcript; in vitro dose response (IC50); in vivo inhibition of of C9ORF72 antisense transcript in a transgenic animal containing a human C9ORF72 transgene in relevant tissues (e.g., brain and/or spinal cord); and/or tolerability in mouse, rat, dog, and/or primate. Tolerability markers that may be measured include blood and serum chemistry parameters, CSF chemistry parameters, body and organ weights, general observations and/or behavioral tests, and/or biochemical markers such as GFAP and/or AIF1. Acute or long term tolerability may be measured.

### Certain Assays for Measuring C9ORF72 Antisense Transcripts

Certain assays described herein are directed to the reduction of C9ORF72 antisense transcript. Additional assays may be used to measure the reduction of C9ORF72 antisense transcript. Additional controls may be used as a baseline for measuring the reduction of C9ORF72 transcript.

### EXAMPLES

### Non-limiting disclosure

While certain compounds, compositions, and methods described herein have been described with specificity in accordance with certain embodiments, the following examples serve only to illustrate the compounds described herein and are not intended to limit the same.

### Example 1: Antisense inhibition of C9ORF72 antisense transcript

Antisense oligonucleotides targeted to C9ORF72 antisense transcript were tested for their effects on C9ORF72 antisense transcript expression *in vitro.* Cultured HepG2 cells were transfected with 50 nM antisense oligonucleotide or water for untransfected controls.

Total RNA was isolated from the cells 24 hours after transfection using TRIzol (Life Technologies) according to the manufacturer's directions. Two DNase reactions were performed, one on the column during RNA purification, and one after purification using amplification grade DNase. The isolated RNA was reverse transcribed to generate cDNA from the C9ORF72 antisense transcript using a primer of the sequence CGACTGGAGCACGAGGACACTGAGGAAAGAGAGGTGCGTCAAA (SEQ ID NO: 12). GAPDH cDNA was reverse transcribed as a control using a primer of the sequence TTTTTTTTTTTTTTTTTT (SEQ ID NO: 13).

Two PCR amplification steps were completed for the C90RF72 antisense cDNA. The first PCR amplification was completed using outer forward primer AAAGAGAAGCAACCGGGC (SEQ ID NO: 14) and reverse primer CGACTGGAGCACGAGGACACTG (SEQ ID NO: 15). The PCR product of the first PCR amplification was subjected to a nested PCR using nested forward primer CAGGGACGGCTGACACA (SEQ ID NO: 16) and reverse primer SEQ ID 15. One PCR amplification of GAPDH was performed with forward primer GTCAACGGATTTGGTCGTATTG (SEQ ID NO: 17) and reverse primer TGGAAGATGGTGATGGGATTT (SEQ ID NO: 18). The amplified cDNA was then loaded onto 5% acrylamide gels and stained with ethidium bromide. Densitometry analysis was performed using Gel Logic 200 and Kodak MI software (Kodak Scientific Imaging Systems, Rochester, NY, USA). The mean intensities from regions of interest (ROI) that corresponded to the C90RF72 antisense cDNA and GAPDH cDNA bands were measured. The intensity of each C90RF72 antisense cDNA band was normalized to its corresponding GAPDH cDNA band. These normalized values for the C90RF72 antisense transcript expression for cells treated with antisense oligonucleotide were then compared to the normalized values for C90RF72 antisense transcript expression in an untransfected control that was run in the same gel. The final value for band intensities obtained was used to calculate the % inhibition.

The table below lists the antisense oligonucleotides designed and tested for C90RF72 antisense transcript expression *in vitro.* ISIS No. 141923 is a negative control that is mismatched to the target. Although ISIS No. 141923 is a negative control in that it is mismatched to the target, it does not necessarily represent a baseline for comparing C90RF72 ASOs targeting the antisense transcript because it causes reduction of antisense transcript. ISIS No. 576816 is a negative control that is complementary to C90RF72 sense transcript. ISIS No. 576816 causes no activity and represents a baseline for comparing the ASOs targeting the C90RF72 antisense transcript. All of the oligonucleotides in the table are 5-10-5 gapmers, 20 nucleosides in length, wherein the central gap segment comprises of ten 2'-deoxynucleosides and is flanked by wing segments on the 5' direction and the 3' direction comprising five nucleosides each. Each nucleoside in the 5' wing segment and each nucleoside in the 3' wing segment has a 2'-MOE modification. The internucleoside linkages throughout each gapmer are phosphorothioate linkages. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to a putative antisense transcript sequence (designated herein as SEQ ID NO: 11). SEQ ID NO: 11 is a sequence that is complementary to nucleotides 1159 to 1734 of SEQ ID NO: 2 (the complement of GENBANK Accession No. NT_008413.18 truncated from nucleotides 27535000 to 27565000).

**Table 6: Inhibition of C9ORF72 Antisense Transcript by Antisense Oligonucleotides**

| ISIS NO. | Start Site | Stop Site | Sequence 5' to 3' | % Inhibition | SEQ ID NO |
|---|---|---|---|---|---|
| 141923 | n/a | n/a | CCTTCCCTGAAGGTTCCTCC | 27 | 19 |
| 576816 | n/a | n/a | GCCTTACTCTAGGACCAAGA | 0 | 20 |
| 664634 | 281 | 300 | GGAACTCAGGAGTCGCGCGC | 21 | 22 |
| 664633 | 284 | 303 | TC TGGAAC TCAGGAGTCGCG | 14 | 23 |
| 664632 | 293 | 312 | GTAGCAAGCTCTGGAACTCA | 25 | 24 |
| 664631 | 296 | 315 | CCTGTAGCAAGCTCTGGAAC | 40 | 25 |
| 664630 | 299 | 318 | CAGCCTGTAGCAAGCTCTGG | 58 | 26 |
| 664628 | 305 | 324 | CAACCGCAGCCTGTAGCAAG | 51 | 28 |
| 664627 | 308 | 327 | AAACAACCGCAGCCTGTAGC | 24 | 29 |
| 664626 | 311 | 330 | GGGAAACAACCGCAGCCTGT | 35 | 30 |
| 664625 | 314 | 333 | GGAGGGAAACAACCGCAGCC | 22 | 31 |
| 664624 | 317 | 336 | CAAGGAGGGAAACAACCGCA | 14 | 32 |
| 664622 | 341 | 360 | TGATAAAGATTAACCAGAAG | 18 | 34 |
| 664621 | 344 | 363 | ACCTGATAAAGATTAACCAG | 2 | 35 |
| 664620 | 347 | 366 | AAGACCTGATAAAGATTAAC | 14 | 36 |
| 664619 | 353 | 372 | CAAGAAAAGACCTGATAAAG | 34 | 37 |
| 664618 | 356 | 375 | GAACAAGAAAAGACCTGATA | 4 | 38 |
| 664617 | 359 | 378 | GGTGAACAAGAAAAGACCTG | 16 | 39 |
| 664616 | 365 | 384 | GCTGAGGGTGAACAAGAAAA | 15 | 40 |
| 664615 | 368 | 387 | CTCGCTGAGGGTGAACAAGA | 37 | 41 |
| 664614 | 371 | 390 | GTACTCGCTGAGGGTGAACA | 31 | 42 |
| 664612 | 377 | 396 | CTCACAGTACTCGCTGAGGG | 11 | 44 |
| 664610 | 383 | 402 | CTTGCTCTCACAGTACTCGC | 79 | 46 |
| 664609 | 386 | 405 | CTACTTGCTCTCACAGTACT | 87 | 47 |
| 664608 | 389 | 408 | CCACTACTTGCTCTCACAGT | 73 | 48 |
| 664607 | 392 | 411 | TCCCCACTACTTGCTCTCAC | 66 | 49 |
| 664606 | 395 | 414 | CTCTCCCCACTACTTGCTCT | 91 | 50 |
| 664605 | 398 | 417 | CCTCTCTCCCCACTACTTGC | 50 | 51 |
| 664604 | 413 | 432 | TTTTGTTTTTCCCACCCTCT | 71 | 52 |
| 664603 | 428 | 447 | TTAGGAGGTGTGTGTTTTTG | 66 | 53 |
| 664602 | 431 | 450 | GGTTTAGGAGGTGTGTGTTT | 52 | 54 |
| 664601 | 434 | 453 | GTGGGTTTAGGAGGTGTGTG | 62 | 55 |
| 664598 | 443 | 462 | AGAGCAGGTGTGGGTTTAGG | 34 | 58 |
| 664597 | 446 | 465 | GCAAGAGCAGGTGTGGGTTT | 29 | 59 |
| 664595 | 452 | 471 | GGTCTAGCAAGAGCAGGTGT | 36 | 61 |

### Example 2: In vivo rodent inhibition and tolerability with treatment of C9ORF72 antisense oligonucleotides

In order to assess the tolerability of inhibition of C90RF72 expression *in vivo,* antisense oligonucleotides targeting a murine C90RF72 nucleic acid were designed and assessed in mouse and rat models.

ISIS 571883 was designed as a 5-10-5 MOE gapmer, 20 nucleosides in length, wherein the central gap segment comprises ten 2'-deoxynucleosides and is flanked by wing segments on both the 5' end and on the 3' end comprising five nucleosides each. Each nucleoside in the 5' wing segment and each nucleoside in the 3' wing segment has a MOE modification. The internucleoside linkages are phosphorothioate linkages. All cytosine residues throughout the gapmer are 5-methylcytosines. ISIS 571883 has a target start site of nucleoside 33704 on the murine C90RF72 genomic sequence, designated herein as SEQ ID NO: 93 (the complement of GENBANK Accession No. NT_166289.1 truncated from nucleosides 3587000 to 3625000).

ISIS 603538 was designed as a 5-10-5 MOE gapmer, 20 nucleosides in length, wherein the central gap segment comprises ten 2'-deoxynucleosides and is flanked by wing segments on both the 5' end and on the 3' end comprising five nucleosides each. Each nucleoside in the 5' wing segment and each nucleoside in the 3' wing segment has a MOE modification. The internucleoside linkages are either phosphorothioate linkages or phosphate ester linkages (Gs Ao Co Co Gs Cs Ts Ts Gs As Gs Ts Ts Ts Gs Co Co Ao Cs A; wherein 's' denotes a phosphorothioate internucleoside linkage, 'o' denotes a phosphate ester linkage; and A, G, C, T denote the relevant nucleosides). All cytosine residues throughout the gapmer are 5-methylcytosines. ISIS 603538 has a target start site of nucleoside 2872 on the rat C90RF72 mRNA sequence, designated herein as SEQ ID NO: 94 (GENBANK Accession No. NM_001007702.1).

### Mouse experiment 1

Groups of 4 C57BL/6 mice each were injected with 50 µg, 100 µg, 300 µg, 500 µg, or 700 µg of ISIS 571883 administered via an intracerebroventricular bolus injection. A control group of four C57/BL6 mice were similarly treated with PBS. Animals were anesthetized with 3% isofluorane and placed in a stereotactic frame. After sterilizing the surgical site, each mouse was injected -0.2 mm anterio-posterior from the bregma and 3 mm dorsoventral to the bregma with the above-mentioned doses of ISIS 571883 using a Hamilton syringe. The incision was closed with sutures. The mice were allowed to recover for 14 days, after which animals were euthanized according to a humane protocol approved by the Institutional Animal Care and Use Committee. Brain and spinal cord tissue were harvested and snap frozen in liquid nitrogen. Prior to freezing, brain tissue was cut transversely five sections using a mouse brain matrix.

### RNA analysis

RNA was extracted from a 2-3 mm brain section posterior to the injection site, from brain frontal cortex and from the lumbar section of the spinal cord tissue for analysis of C90RF72 mRNA expression. C90RF72 mRNA expression was measured by RT-PCR. The data is presented in Table 7. The results indicate that treatment with increasing doses of ISIS 571883 resulted in dose-dependent inhibition of C90RF72 mRNA expression.

The induction of the microglial marker AIF-1 as a measure of CNS toxicity was also assessed. The data is presented in Table 8. The results indicate that treatment with increasing doses of ISIS 571883 did not result in significant increases in AIF-1 mRNA expression. Hence, the injection of ISIS 571883 was deemed tolerable in this model.

**Table 7**

| Percentage inhibition of C90RF72 mRNA expression compared to the PBS control | | | |
|---|---|---|---|
| Dose (µg) | Posterior brain | Cortex | Spinal cord |
| 50 | 22 | 8 | 46 |
| 100 | 22 | 12 | 47 |
| 300 | 55 | 47 | 67 |
| 500 | 61 | 56 | 78 |
| 700 | 65 | 65 | 79 |

**Table 8**

| Percentage expression of AIF-1 mRNA expression compared to the PBS control | | |
|---|---|---|
| Dose (µg) | Posterior brain | Spinal cord |
| 50 | 102 | 89 |
| 100 | 105 | 111 |
| 300 | 107 | 98 |
| 500 | 131 | 124 |
| 700 | 122 | 116 |

### Mouse experiment 2

Groups of 4 C57BL/6 mice each were injected with 500 µg of ISIS 571883 administered via an intracerebroventricular bolus injection in a procedure similar to that described above. A control group of four C57/BL6 mice were similarly treated with PBS. The mice were tested at regular time points after ICV administration.

### Behavior analysis

Two standard assays to assess motor behavior were employed; the rotarod assay and grip strength assay. In case of the rotarod assays, the time of latency to fall was measured. The data for the assays is presented in Tables 9 and 10. The results indicate that there were no significant changes in the motor behavior of the mice as a result of antisense inhibition of ISIS 571883 or due to the ICV injection. Hence, antisense inhibition of C90RF72 was deemed tolerable in this model.

**Table 9**

| Latency to fall (sec) in the rotarod assay | | |
|---|---|---|
| Weeks after inj ection | PBS | ISIS 571883 |
| 0 | 66 | 66 |
| 4 | 91 | 70 |
| 8 | 94 | 84 |

**Table 10**

| Mean hindlimb grip strength (g) in the grip strength assay | | |
|---|---|---|
| Weeks after injection | PBS | ISIS 571883 |
| 0 | 57 | 63 |
| 1 | 65 | 51 |
| 2 | 51 | 52 |
| 3 | 51 | 51 |
| 4 | 59 | 72 |
| 5 | 60 | 64 |
| 6 | 61 | 72 |
| 7 | 67 | 68 |
| 8 | 66 | 70 |
| 9 | 63 | 61 |
| 10 | 48 | 46 |

### Rat experiment

Groups of 4 Sprague-Dawley rats each were injected with 700 µg, 1,000 µg, or 3,000 µg of ISIS 603538 administered via an intrathecal bolus injection. A control group of four Sprague-Dawley rats was similarly treated with PBS. Animals were anesthetized with 3% isofluorane and placed in a stereotactic frame. After sterilizing the surgical site, each rat was injected with 30 µL of ASO solution administered via 8 cm intrathecal catheter 2 cm into the spinal canal with a 50 µL flush. The rats were allowed to recover for 4 weeks, after which animals were euthanized according to a humane protocol approved by the Institutional Animal Care and Use Committee.

### RNA analysis

RNA was extracted from a 2-3 mm brain section posterior to the injection stie, from brain frontal cortex, and from the cervical and lumbar sections of the spinal cord tissue for analysis of C90RF72 mRNA expression. C90RF72 mRNA expression was measured by RT-PCR. The data is presented in Table 11. The results indicate that treatment with increasing doses of ISIS 603538 resulted in dose-dependent inhibition of C90RF72 mRNA expression.

The induction of the microglial marker AIF-1 as a measure of CNS toxicity was also assessed. The data is presented in Table 12. The results indicate that treatment with increasing doses of ISIS 603538 did not result in significant increases in AIF-1 mRNA expression. Hence, the injection of ISIS 603538 was deemed tolerable in this model.

**Table 11**

| Percentage inhibition of C90RF72 mRNA expression compared to the PBS control | | | | |
|---|---|---|---|---|
| Dose (µg) | Brain (1 mm section) | Cortex | Spinal cord (lumbar) | Spinal cord (cervical) |
| 700 | 21 | 4 | 86 | 74 |
| 1000 | 53 | 49 | 88 | 82 |
| 3000 | 64 | 62 | 88 | 80 |

**Table 12**

| Percentage expression of AIF-1 mRNA expression compared to the PBS control | | | | |
|---|---|---|---|---|
| Dose (µg) | Brain (1 mm section) | Cortex | Spinal cord (lumbar) | Spinal cord (cervical) |
| 700 | 97 | 119 | 98 | 89 |
| 1000 | 105 | 113 | 122 | 96 |
| 3000 | 109 | 141 | 156 | 115 |

### Body weight analysis

Body weights of the rats were measured at regular time point intervals. The data is presented in Table 13. The results indicate that treatment with increasing doses of ISIS 603538 did not have any significant changes in the body weights of the rats.

**Table 13**

| Body weights of the rats (% initial body weight) | | | | | | |
|---|---|---|---|---|---|---|
| | Dose (µg) | Week 1 | Week 2 | Week 3 | Week 4 | Week 5 |
| PBS | | 100 | 94 | 103 | 105 | 109 |
| ISIS 603538 | 700 | 100 | 94 | 98 | 103 | 107 |
| | 1000 | 100 | 95 | 97 | 101 | 103 |
| | 3000 | 100 | 92 | 98 | 102 | 105 |

### Example 3: Dose response screens of antisense oligonucleotides targeting human C9ORF72 sense transcript in two patient fibroblast lines

Two different fibroblast cell lines from human patients (F09-152 and F09-229) were analyzed with antisense oligonucleotides that target the C90RF72 sense transcript before exon 1B; i.e. antisense oligonucleotides that target the hexanucleotide repeat expansion containing transcript and antisense oligonucleotides that target downstream of exon 1. The target start and stop sites and the target regions with respect to SEQ ID NOs: 1 and 2 for each oligonucleotide are provided in Table 14. ISIS 577061 and ISIS 577065 target C90RF72 upstream of exon 1B and just upstream of the hexanucleotide repeat. The rest of the ISIS oligonucleotides of Table 15 target C90RF72 downstream of exon 1B and the hexanucleotide repeat.

**Table 14**

| Target Start and Stop sites of ISIS oligonucleotides used in a dose response assay in C90RF72 patient fibroblasts | | | |
|---|---|---|---|
| ISIS No | Target Start Site at SEQ ID NO: 1 | Target Start Site at SEQ ID NO: 2 | Target Region |
| 577061 | n/a | 1406 | Upstream of exon 1B |
| 577065 | n/a | 1446 | Upstream of exon 1B |
| 577083 | n/a | 3452 | Downstream of exon 1B |
| 576816 | 232 | 7990 | Exon 2 |
| 576974 | 3132 | 28251 | Exon 11 |

Cells were plated at a density of 20,000 cells per well and transfected using electroporation with 246.9 nM, 740.7 nM, 2,222.2 nM, 6,666.7 nM, and 20,000.0 nM concentrations of antisense oligonucleotide. After a treatment period of approximately 16 hours, RNA was isolated from the cells and C90RF72 mRNA levels were measured by quantitative real-time PCR. Two primer probe sets were used: (1) human C90RF72 primer probe set RTS3750, which measures total mRNA levels, and (2) RTS3905, which targets the hexanucleotide repeat expansion containing transcript, which measures only mRNA transcripts that contain the hexanucleotide repeat expansion. C90RF72 mRNA levels were adjusted according to total RNA content, as measured by RIBOGREEN®. Results are presented as percent inhibition of C90RF72, relative to untreated control cells.

As illustrated in Tables 15-18, below, the two oligonucleotides that target upstream of exon 1B and, therefore, target mRNA transcripts containing the hexanucleotide repeat expansion (ISIS 577061 and ISIS 577065), do not inhibit total mRNA levels of C90RF72 (as measured by RTS3750) as well as ISIS 576974, 576816, and 577083, which target downstream of exon 1B and, therefore, do not target the mRNA transcript containing the hexanucleotide repeat expansion. Expression levels of the C90RF72 mRNA transcript containing the hexanucleotide repeat expansion are low (about 10% of the total C90RF72 expression products), therefore, oligonucleotides targeting the mRNA transcript containing the hexanucleotide repeat expansion do not robustly inhibit total C90RF72 mRNA (as measured by RTS3905), as suggested by Tables 16 and 18 below. Thus, ISIS 577061 and ISIS 577065 preferentially inhibit expression of mRNA transcripts containing the hexanucleotide repeat expansion.

**Table 15**

| Percent inhibition of C90RF72 total mRNA in F09-152 patient fibroblasts in a dose response assay as measured with RTS3750 | | | | | |
|---|---|---|---|---|---|
| ISIS No | 246.9 nM | 740.7 nM | 2222.2 nM | 6666.7 nM | 20000.0 nM |
| 577061 | 6 | 11 | 0 | 18 | 10 |
| 577065 | 10 | 11 | 30 | 29 | 0 |
| 576974 | 61 | 69 | 72 | 83 | 83 |
| 576816 | 35 | 76 | 82 | 91 | 93 |
| 577083 | 28 | 38 | 52 | 75 | 80 |

**Table 16**

| Percent inhibition of C90RF72 mRNA transcripts containing the hexanucleotide repeat expansion in F09-152 patient fibroblasts in a dose response assay as measured with RTS3905 | | | | | |
|---|---|---|---|---|---|
| ISIS No | 246.9 nM | 740.7 nM | 2222.2 nM | 6666.7 nM | 20000.0 nM |
| 577061 | 4 | 28 | 58 | 81 | 87 |
| 577065 | 25 | 54 | 70 | 90 | 94 |
| 576974 | 57 | 77 | 81 | 93 | 92 |
| 576816 | 37 | 77 | 91 | 97 | 98 |
| 577083 | 37 | 53 | 74 | 93 | 94 |

**Table 17**

| Percent inhibition of C90RF72 total mRNA in F09-229 patient fibroblasts in a dose response assay as measured with RTS3750 | | | | | |
|---|---|---|---|---|---|
| ISIS No | 246.9 nM | 740.7 nM | 2222.2 nM | 6666.7 nM | 20000.0 nM |
| 577061 | 0 | 0 | 0 | 17 | 7 |
| 577065 | 8 | 17 | 17 | 16 | 3 |
| 576974 | 43 | 58 | 85 | 85 | 74 |
| 576816 | 45 | 70 | 85 | 81 | 89 |
| 577083 | 22 | 45 | 56 | 76 | 78 |

**Table 18**

| Percent inhibition of C90RF72 mRNA transcripts containing the hexanucleotide repeat expansion in F09-229 patient fibroblasts in a dose response assay as measured with RTS3905 | | | | | |
|---|---|---|---|---|---|
| ISIS No | 246.9 nM | 740.7 nM | 2222.2 nM | 6666.7 nM | 20000.0 nM |
| 577061 | 14 | 36 | 70 | 87 | 89 |
| 577065 | 26 | 48 | 92 | 91 | 98 |
| 576974 | 63 | 87 | 91 | 92 | 91 |
| 576816 | 62 | 81 | 96 | 98 | 100 |
| 577083 | 36 | 64 | 82 | 98 | 96 |

## Claims

1. A compound comprising a modified oligonucleotide consisting of 12 to 50 linked nucleosides wherein the nucleobase sequence of the modified oligonucleotide is at least 90% complementary to an equal length portion of a C9ORF72 antisense transcript, wherein the C9ORF72 antisense transcript is encoded by SEQ ID NO: 11, wherein the modified oligonucleotide comprises at least one modification selected from a modified sugar, a sugar surrogate, and a modified internucleoside linkage, and wherein the modified oligonucleotide is capable of reducing the level of expression of the C9ORF72 antisense transcript.

2. The compound of claim 1, wherein the nucleobase sequence of the modified oligonucleotide is 100% complementary to an equal length portion of a C9ORF72 antisense transcript, wherein the C9ORF72 antisense transcript is encoded by SEQ ID NO: 11.

3. The compound of claim 1 or claim 2, wherein the modified oligonucleotide has a nucleobase sequence comprising at least 8 contiguous nucleobases of a sequence selected from among SEQ ID NOs: 19, 20, 22-26, 28-32, 34-42, 44, 46-55, 58, 59, and 61.

4. A modified oligonucleotide consisting of 12 to 50 linked nucleosides and having a nucleobase sequence comprising at least 8 contiguous nucleobases of a sequence selected from among SEQ ID NOs: 19, 20, 22-26, 28-32, 34-42, 44, 46-55, 58, 59, and 61, wherein the modified oligonucleotide is capable of reducing the level of expression of a C9ORF72 antisense transcript, and wherein the C9ORF72 antisense transcript is encoded by SEQ ID NO: 11.

5. The compound of any one of claims 1-3, or the modified oligonucleotide of claim 4, wherein the modified oligonucleotide comprises at least one modified internucleoside linkage, optionally wherein the at least one modified internucleoside linkage is a phosphorothioate internucleoside linkage.

6. The compound of any one of claims 1-3 and 5, or the modified oligonucleotide of claim 4 or claim 5, wherein at least one nucleobase of the modified oligonucleotide is a modified nucleobase, optionally wherein the modified nucleobase is a 5-methylcytosine.

7. The compound of any one of claims 1-3, 5 and 6, or the modified oligonucleotide of any one of claims 4-6, wherein at least one nucleoside of the modified oligonucleotide comprises a modified sugar, or wherein each nucleoside of the modified oligonucleotide comprises a modified sugar, optionally wherein:
a) the modified sugar is a bicyclic sugar, optionally wherein the bicyclic sugar comprises a chemical bridge between the 4' and 2' position of the sugar, wherein the chemical bridge is selected from: 4'-CH(R)-O-2' and 4'-(CH₂)₂-O-2',wherein R is independently selected from H, C₁-C₆ alkyl, and C₁-C₆ alkoxy, further optionally wherein at least one chemical bridge is 4'-CH(R)-O-2' and wherein R is methyl, H or -CH₂-O-CH₃; or
b) the modified sugar comprises a 2'-O-methoxyethyl group or a 2'-O-methyl group.

8. The compound of any one of claims 1-3 and 5-7, or the modified oligonucleotide of any one of claims 4-7, wherein the at least one nucleoside of the modified oligonucleotide comprises a sugar surrogate, optionally wherein the at least one sugar surrogate is a morpholino or a peptide nucleic acid.

9. The compound of any one of claims 1-3 and 5-8, or the modified oligonucleotide of any one of claims 4-8, wherein
a) the modified oligonucleotide is a gapmer; or
b) the modified oligonucleotide is a single-stranded oligonucleotide.

10. A conjugated compound comprising the compound of any one of claims 1-3 and 5-9.

11. The compound of any of claims 1-3 and 5-10, consisting of the modified oligonucleotide.

12. A double-stranded compound comprising the compound of any one of claims 1-3 and 5-11.

13. A conjugated compound comprising the double-stranded compound of claim 12.

14. A composition comprising a compound of any one of claims 1-3, 5-9 and 11, a double stranded compound of claim 12, a conjugated compound of claim 10 or 13, or a modified oligonucleotide of any one of claims 4-9 and a pharmaceutically acceptable diluent or carrier, optionally wherein:
i) the pharmaceutically acceptable diluent is phosphate-buffered saline (PBS); and/or
ii) the modified oligonucleotide of the compound is a sodium salt.

15. A compound of any one of claims 1-3, 5-9 and 11, a double stranded compound of claim 12, a conjugated compound of claim 10 or 13, a composition of claim 14, or a modified oligonucleotide of any one of claims 4-9 for use in treating a neurodegenerative disease.

## Patentansprüche

1. Verbindung, umfassend ein modifiziertes Oligonukleotid, bestehend aus 12 bis 50 verknüpften Nukleosiden, wobei die Nukleobasensequenz des modifizierten Oligonukleotids zu mindestens 90% zu einem Abschnitt gleicher Länge eines C9ORF72-Antisense-Transkripts komplementär ist, wobei das C9ORF72-Antisense-Trans-kript durch SEQ ID NO: 11 kodiert wird, wobei das modifizierte Oligonukleotid mindestens eine Modifikation umfasst, die aus einem modifizierten Zucker, einem Zuckersurrogat und einer modifizierten Internukleosid-Verknüpfung ausgewählt ist, und wobei das modifizierte Oligonukleotid in der Lage ist, das Expressionsniveau des C9ORF72-Antisense-Transkripts zu reduzieren.

2. Verbindung von Anspruch 1, wobei die Nukleobasensequenz des modifizierten Oligonukleotids 100% komplementär zu einem Abschnitt gleicher Länge eines C9ORF72-Antisense-Transkripts ist, wobei das C9ORF72-Antisense-Transkript durch SEQ ID NO: 11 kodiert wird.

3. Verbindung von Anspruch 1 oder Anspruch 2, wobei das modifizierte Oligonukleotid eine Nukleobasensequenz aufweist, die mindestens 8 aufeinanderfolgende Nukleobasen einer Sequenz umfasst, die aus den SEQ ID NOs: 19, 20, 22-26, 28-32, 34-42, 44, 46-55, 58, 59 und 61 ausgewählt ist.

4. Modifiziertes Oligonukleotid, bestehend aus 12 bis 50 verknüpften Nukleosiden und aufweisend eine Nukleobasensequenz, umfassend mindestens 8 aufeinanderfolgende Nukleobasen einer Sequenz, ausgewählt aus den SEQ ID NOs: 19, 20, 22-26, 28-32, 34-42, 44, 46-55, 58, 59 und 61, wobei das modifizierte Oligonukleotid fähig ist, das Expressionsniveau eines C9ORF72-Antisense-Trans-kripts zu reduzieren, und wobei das C9ORF72-Antisense-Transkript durch SEQ ID NO: 11 kodiert wird.

5. Verbindung von einem der Ansprüche 1-3 oder das modifizierte Oligonukleotid von Anspruch 4, wobei das modifizierte Oligonukleotid mindestens eine modifizierte Internukleosid-Verknüpfung umfasst, wobei die mindestens eine modifizierte Internukleosid-Verknüpfung gegebenenfalls eine Phosphorothioat-Internukleosid-Verknüpfung ist.

6. Verbindung von einem der Ansprüche 1-3 und 5 oder das modifizierte Oligonukleotid von Anspruch 4 oder Anspruch 5, wobei mindestens eine Nukleobase des modifizierten Oligonukleotids eine modifizierte Nukleobase ist, wobei die modifizierte Nukleobase gegebenenfalls 5-Methylcytosin ist.

7. Verbindung von einem der Ansprüche 1-3, 5 und 6 oder das modifizierte Oligonukleotid von einem der Ansprüche 4-6, wobei mindestens ein Nukleosid des modifizierten Oligonukleotids einen modifizierten Zucker umfasst oder wobei jedes Nukleosid des modifizierten Oligonukleotids einen modifizierten Zucker umfasst, wobei gegebenenfalls:
a) der modifizierte Zucker ein bicyclischer Zucker ist, wobei der bicyclische Zucker optional eine chemische Brücke zwischen der 4'- und der 2'-Position des Zuckers umfasst, wobei die chemische Brücke ausgewählt ist aus: 4'-CH(R)-O-2' und 4'-(CH₂)₂-O-2', wobei R unabhängig ausgewählt ist aus H, C₁-C₆-Alkyl und C₁-C₆-Alkoxy, wobei ferner mindestens eine chemische Brücke optional 4'-CH(R)-O-2' ist, und worin R Methyl, H oder -CH₂-O-CH₃ ist; oder
b) der modifizierte Zucker eine 2'-O-Methoxyethylgruppe oder eine 2'-O-Methylgruppe umfasst.

8. Verbindung von einem der Ansprüche 1-3 und 5-7 oder das modifizierte Oligonukleotid von einem der Ansprüche 4-7, wobei das mindestens eine Nukleosid des modifizierten Oligonukleotids ein Zuckersurrogat umfasst, wobei es sich bei dem mindestens einen Zuckersurrogat optional um eine Morpholino- oder eine Peptidnukleinsäure handelt.

9. Verbindung von einem der Ansprüche 1-3 und 5-8 oder das modifizierte Oligonukleotid von einem beliebigen der Ansprüche 4-8, wobei
a) das modifizierte Oligonukleotid ein Gapmer ist; oder
b) das modifizierte Oligonukleotid ein einzelsträngiges Oligonukleotid ist.

10. Konjugierte Verbindung, umfassend die Verbindung von einem der Ansprüche 1-3 und 5-9.

11. Verbindung von einem der Ansprüche 1-3 und 5-10, bestehend aus dem modifizierten Oligonukleotid.

12. Doppelsträngige Verbindung, umfassend die Verbindung von einem der Ansprüche 1-3 und 5-11.

13. Konjugierte Verbindung, umfassend die doppelsträngige Verbindung von Anspruch 12.

14. Zusammensetzung, umfassend eine Verbindung von einem der Ansprüche 1-3, 5-9 und 11, eine doppelsträngige Verbindung von Anspruch 12, eine konjugierte Verbindung von Anspruch 10 oder 13 oder ein modifiziertes Oligonukleotid von einem der Ansprüche 4-9 und einen pharmazeutisch annehmbaren Verdünner oder Träger, wobei gegebenenfalls:
i) der pharmazeutisch annehmbare Verdünner phosphatgepufferte Salzlösung (PBS) ist; und/oder
ii) das modifizierte Oligonukleotid der Verbindung ein Natriumsalz ist.

15. Verbindung von einem der Ansprüche 1-3, 5-9 und 11, eine doppelsträngige Verbindung von Anspruch 12, eine konjugierte Verbindung von Anspruch 10 oder 13, eine Zusammensetzung von Anspruch 14 oder ein modifiziertes Oligonukleotid von einem beliebigen der Ansprüche 4-9 zur Verwendung bei der Behandlung einer neurodegenerativen Erkrankung.

## Revendications

1. Composé comprenant un oligonucléotide modifié constitué de 12 à 50 nucléosides liés dans lequel la séquence de bases azotées de l'oligonucléotide modifié est au moins 90 % complémentaire d'une partie de longueur égale d'un produit de transcription antisens C9ORF72, le produit de transcription antisens C9ORF72 étant codé par SEQ ID NO: 11, dans lequel l'oligonucléotide modifié comprend au moins une modification choisie parmi un sucre modifié, un substitut de sucre, et une liaison internucléosidique modifiée, et dans lequel l'oligonucléotide modifié permet de réduire le taux d'expression du produit de transcription antisens C9ORF72.

2. Composé selon la revendication 1, dans lequel la séquence de bases azotées de l'oligonucléotide modifié est 100 % complémentaire d'une partie de longueur égale d'un produit de transcription antisens C9ORF72, le produit de transcription antisens C9ORF72 étant codé par SEQ ID NO: 11.

3. Composé selon la revendication 1 ou la revendication 2, dans lequel l'oligonucléotide modifié a une séquence de bases azotées comprenant au moins 8 bases azotées contiguës d'une séquence choisie parmi SEQ ID NO: 19, 20, 22-26, 28-32, 34-42, 44, 46-55, 58, 59 et 61.

4. Oligonucléotide modifié constitué de 12 à 50 nucléosides liés et ayant une séquence de bases azotées comprenant au moins 8 bases azotées contiguës d'une séquence choisie parmi SEQ ID NO: 19, 20, 22-26, 28-32, 34-42, 44, 46-55, 58, 59, et 61, l'oligonucléotide modifié permettant de réduire le taux d'expression d'un produit de transcription antisens ant C9ORF72, et le produit de transcription antisens C9ORF72 étant codé par SEQ ID NO: 11.

5. Composé selon l'une quelconque des revendications 1 à 3, ou oligonucléotide modifié selon la revendication 4, l'oligonucléotide modifié comprenant au moins une liaison internucléosidique modifiée, facultativement dans lequel l'au moins une liaison internucléosidique modifiée est une liaison internucléosidique phosphorothioate.

6. Composé selon l'une quelconque des revendications 1 à 3 et 5, ou oligonucléotide modifié selon la revendication 4 ou la revendication 5, dans lequel au moins une base azotée de l'oligonucléotide modifié est une base azotée modifiée, facultativement dans lequel la base azotée modifiée est une 5-méthyl-cytosine.

7. Composé selon l'une quelconque des revendications 1 à 3, 5 et 6, ou oligonucléotide modifié selon l'une quelconque des revendications 4 à 6, dans lequel au moins un nucléoside de l'oligonucléotide modifié comprend un sucre modifié, ou dans lequel chaque nucléoside de l'oligonucléotide modifié comprend un sucre modifié, facultativement dans lequel :
a) le sucre modifié est un sucre bicyclique, facultativement dans lequel le sucre bicyclique comprend un pont chimique entre les position 4' et 2' du sucre, dans lequel le pont chimique est choisi parmi : 4'-CH(R)-O-2' et 4'-(CH₂)₂-O-2', où R est indépendamment choisi parmi H, alkyle en C₁-C₆, et alcoxy en C₁-C₆, facultativement dans lequel, en outre au moins un pont chimique est 4'-CH(R)-O-2' et dans lequel R est méthyle, H ou -CH₂-O-CH₃ ; ou
b) le sucre modifié comprend un groupe 2'-O-méthoxy-éthyle ou un groupe 2'-O-méthyle.

8. Composé selon l'une quelconque des revendications 1 à 3 et 5 à 7, ou oligonucléotide modifié selon l'une quelconque des revendications 4 à 7, dans lequel l'au moins un nucléoside de l'oligonucléotide modifié comprend un substitut de sucre, facultativement dans lequel l'au moins un substitut de sucre est un acide nucléique morpholino ou peptidique.

9. Composé selon l'une quelconque des revendications 1 à 3 et 5 à 8, ou oligonucléotide modifié selon l'une quelconque des revendications 4 à 8, dans lequel
a) l'oligonucléotide modifié est un gapmère ; ou
b) l'oligonucléotide modifié est un oligonucléotide simple brin.

10. Composé conjugué comprenant le composé selon l'une quelconque des revendications 1 à 3 et 5 à 9.

11. Composé selon l'une quelconque des revendications 1 à 3 et 5 à 10, constitué de l'oligonucléotide modifié.

12. Composé double brin comprenant le composé selon l'une quelconque des revendications 1 à 3 et 5 à 11.

13. Composé conjugué comprenant le composé double brin selon la revendication 12.

14. Composition comprenant un composé selon l'une quelconque des revendications 1 à 3, 5 à 9 et 11, un composé double brin selon la revendication 12, un composé conjugué selon la revendication 10 ou 13, ou un oligonucléotide modifié selon l'une quelconque des revendications 4 à 9 et un diluant ou véhicule pharmaceutiquement acceptable, facultativement dans laquelle :
i) le diluant pharmaceutiquement acceptable est un tampon phosphate salin (PBS) ; et/ou
ii) l'oligonucléotide modifié du composé est un sel de sodium.

15. Composé selon l'une quelconque des revendications 1 à 3, 5 à 9 et 11, composé double brin selon la revendication 12, composé conjugué selon la revendication 10 ou 13, composition selon la revendication 14, ou oligonucléotide modifié selon l'une quelconque des revendications 4 à 9 pour utilisation dans le traitement d'une maladie neurodégénérative.
